# EUROPEAN PATENT APPLICATION

(11) **EP 4 570 913 A1**
(43) Date of publication of application: **18.06.2025**
(21) Application number: 23851970.6
(22) Date of filing: 11.08.2023
(51) Int. Cl.: C12N 15/63, C07K 16/28, A61K 39/395, A61P 25/06

(54) **ANTI-CGRP ANTIBODY AND USE**

(30) Priority: 11.08.2022 CN 202210962095
(71) Applicant: Shanghai Junshi Biosciences Co., Ltd., Pilot Free Trade Zone Shanghai 201210 (CN)
(72) Inventor: DONG, Chao, Suzhou, Jiangsu 215002 (CN); YAO, Jian, Suzhou, Jiangsu 215002 (CN); ZHOU, Yuehua, Suzhou, Jiangsu 215002 (CN); LIU, Hui, Suzhou, Jiangsu 215002 (CN); LIU, Dandan, Suzhou, Jiangsu 215002 (CN); ZHANG, Jing, Suzhou, Jiangsu 215002 (CN); LIU, Hongchuan, Suzhou, Jiangsu 215002 (CN); FENG, Hui, Suzhou, Jiangsu 215002 (CN); YAO, Sheng, Suzhou, Jiangsu 215002 (CN)
(74) Representative: Zanoli, Enrico
(86) International application number: PCT/CN2023/112454
(87) International publication number: WO 2024/032750

(57) **Abstract**

Provided are an antibody specifically binding to CGRP or an antigen-binding fragment thereof, and a composition comprising same. Also provided are a nucleic acid molecule encoding the antibody or the antigen-binding fragment thereof, a vector and a host cell for expressing the antibody or the antigen-binding fragment thereof, and therapeutic and diagnostic methods and use of the antibody or the antigen-binding fragment thereof.

## Description

### CROSS REFERENCE TO RELATED APPLICATIONS

The present application claims priority to the Chinese Patent Application No. 202210962095.X, filed on August 11, 2022, which is incorporated herein by reference in its entirety.

### TECHNICAL FIELD

The present disclosure provides an antibody specifically binding to human calcitonin gene-related peptide (hereinafter referred to as "CGRP") or an antigen-binding fragment thereof, and a composition comprising same. Also provided are a nucleic acid molecule encoding the antibody or the antigen-binding fragment thereof disclosed herein, a vector and a host cell used for expressing the antibody or the antigen-binding fragment thereof disclosed herein, and therapeutic and diagnostic methods and use of the antibody or the antigen-binding fragment thereof disclosed herein.

### BACKGROUND

CGRP is a neuropeptide consisting of 37 amino acid residues. It is widely distributed in peripheral and central nervous systems, and plays an important role in functional regulation of cardiovascular system, pain, and nervous system, etc. CGRP is a member of the calcitonin gene-related peptide family, which includes two CGRP forms, adrenomedullin, islet amyloid polypeptide, calcitonin, and calcitonin receptor-stimulating peptide. The two forms of CGRP, i.e., CGRP-α and CGRP-β forms, are present in humans and have similar activities. The two forms of CGRP differ by 3 amino acids and exhibit different distributions.

CGRP is involved in the regulation of many physiological functions of the body.

CGRP has a powerful vasodilative effect and is one of the most potent known peripheral microvasodilators. CGRP is involved in the regulation of various physiological activities by activating CGRP receptors on the cell membrane, and particularly, plays an important role in vasodilation and migraine. Many studies have shown that CGRP is involved in the central nervous system learning and memory processes. Also, it has been reported that CGRP is involved in the regulation of related metabolism during the development of diabetes mellitus. Recent studies have shown that CGRP has a protective effect against several diseases in the cardiovascular system.

Migraine is a common trigeminal vascular headache, which seriously affects the daily life of patients. Triptan is used as a specific analgesic during migraine attack but is limited to use in patients with cardiovascular diseases due to its vasoconstrictive effect. Also, some patients demonstrate resistance to triptan, leading to an urgent need for new effective drugs. Clinical studies have shown that CGRP plays an important role in acute migraine attack, and theoretically, the CGRP activity can be reduced by preventing the synthesis and release of CGRP, reducing the concentration of CGRP after release, and preventing CGRP from activating its receptors.

In spite of currently available CGRP antibodies for treating migraine and for treating inflammatory pain, and advantages of monoclonal CGRP antibodies such as large molecular weights, long half-lives, difficulties in passing through the blood-brain barrier, the absence of toxic metabolites, strong selectivity, and good tolerance, there is still a need for more novel anti-CGRP antibodies.

### SUMMARY

The present application is intended to provide an antibody capable of specifically binding to CGRP (i.e., an anti-CGRP antibody) or an antigen-binding fragment thereof, a polynucleotide molecule encoding the antibody or the antigen-binding fragment thereof disclosed herein, an expression vector and a host cell for expressing the antibody or the antigen-binding fragment thereof disclosed herein, and use of the antibody or the antigen-binding fragment thereof disclosed herein.

In one aspect, the present disclosure provides an anti-CGRP antibody or an antigen-binding fragment thereof, comprising an HCDR1, an HCDR2, an HCDR3, an LCDR1, an LCDR2, and an LCDR3 defined as follows:
HCDR1: DX¹NMH (SEQ ID NO: 103) or GYTMN (SEQ ID NO: 43), wherein X¹ is F or Y;
HCDR2: X²X³X⁴PYNX⁵X⁶X⁷X⁸YNX⁹X¹⁰FX¹¹X¹² (SEQ ID NO: 104) or LINPYIGNTHYNQKFKD (SEQ ID NO: 44), wherein X² is Y or N, X³ is I or V, X⁴ is Y, S, or F, X⁵ is G or A, X⁶ is D, G, V, or H, X⁷ is V, T, S, or A, X⁸ is V or A, X⁹ is Q or R, X¹⁰ is K or N, X¹¹ is T, K, N, or R, and X¹² is N, F, or S;
HCDR3: EGLGGY (SEQ ID NO: 39) or ELDSGFDY (SEQ ID NO: 45);
LCDR1: X¹³X¹⁴SQNIZYX¹⁵X¹⁶ (SEQ ID NO: 105), wherein X¹³ is K, Q, or R, X¹⁴ is A or S, Z is DYDGYA (SEQ ID NO: 114), DFDGYA (SEQ ID NO: 115), DGNT (SEQ ID NO: 116), VHSNGDT (SEQ ID NO: 117), VHSNADT (SEQ ID NO: 118), DYDGYT (SEQ ID NO: 119), or DYGGYS (SEQ ID NO: 120), X¹⁵ is L or M, and X¹⁶ is N or E;
LCDR2: SX¹⁷FNLES (SEQ ID NO: 106) or KVSNRFS (SEQ ID NO: 74), wherein X¹⁷ is V or I; and
LCDR3: X¹⁸QSX¹⁹X²⁰X²¹PYT (SEQ ID NO: 107) or YQGSHVPWT (SEQ ID NO: 75), wherein X¹⁸ is Q, X¹⁹ is N or H, X²⁰ is E or D, X²¹ is F, V, Y, or A.

In some embodiments, the antibody or the antigen-binding fragment thereof comprises: an HCDR1 having the amino acid sequence set forth in SEQ ID NOs: 2, 11, 19, 26, 31, 37, or 43; an HCDR2 having the amino acid sequence set forth in SEQ ID NOs: 3, 110, 12, 111, 20, 112, 27, 32, 38, or 44; an HCDR3 having the amino acid sequence set forth in SEQ ID NOs: 4, 13, 21, 28, 33, 39, or 45; an LCDR1 having the amino acid sequence set forth in SEQ ID NOs: 51, 59, 67, 73, 113, 81, or 86; an LCDR2 having the amino acid sequence set forth in SEQ ID NOs: 52, 60, 68, 74, 82, or 87; and an LCDR3 having the amino acid sequence set forth in SEQ ID NOs: 53, 61, 69, 75, 83, or 88.

In one aspect, the present disclosure provides an anti-CGRP antibody or an antigen-binding fragment thereof, comprising a heavy chain variable region and/or a light chain variable region, wherein
the heavy chain variable region comprises:
   (I) an HCDR1, an HCDR2, and an HCDR3 having the amino acid sequences set forth in SEQ ID NO: 2, SEQ ID NO: 3, and SEQ ID NO: 4, respectively; or an HCDR1, an HCDR2, and an HCDR3 differing from the amino acid sequences set forth in SEQ ID NO: 2, SEQ ID NO: 3, and SEQ ID NO: 4, respectively, by 1, 2, or 3 amino acids; or
   (II) an HCDR1, an HCDR2, and an HCDR3 having the amino acid sequences set forth in SEQ ID NO: 2, SEQ ID NO: 110, and SEQ ID NO: 4, respectively; or an HCDR1, an HCDR2, and an HCDR3 differing from the amino acid sequences set forth in SEQ ID NO: 2, SEQ ID NO: 110, and SEQ ID NO: 4, respectively, by 1, 2, or 3 amino acids; or
   (III) an HCDR1, an HCDR2, and an HCDR3 having the amino acid sequences set forth in SEQ ID NO: 11, SEQ ID NO: 12, and SEQ ID NO: 13, respectively; or an HCDR1, an HCDR2, and an HCDR3 differing from the amino acid sequences set forth in SEQ ID NO: 11, SEQ ID NO: 12, and SEQ ID NO: 13, respectively, by 1, 2, or 3 amino acids; or
   (IV) an HCDR1, an HCDR2, and an HCDR3 having the amino acid sequences set forth in SEQ ID NO: 11, SEQ ID NO: 111, and SEQ ID NO: 13, respectively; or an HCDR1, an HCDR2, and an HCDR3 differing from the amino acid sequences set forth in SEQ ID NO: 11, SEQ ID NO: 111, and SEQ ID NO: 13, respectively, by 1, 2, or 3 amino acids; or
   (V) an HCDR1, an HCDR2, and an HCDR3 having the amino acid sequences set forth in SEQ ID NO: 19, SEQ ID NO: 20, and SEQ ID NO: 21, respectively; or an HCDR1, an HCDR2, and an HCDR3 differing from the amino acid sequences set forth in SEQ ID NO: 19, SEQ ID NO: 20, and SEQ ID NO: 21, respectively, by 1, 2, or 3 amino acids; or
   (VI) an HCDR1, an HCDR2, and an HCDR3 having the amino acid sequences set forth in SEQ ID NO: 19, SEQ ID NO: 112, and SEQ ID NO: 21, respectively; or an HCDR1, an HCDR2, and an HCDR3 differing from the amino acid sequences set forth in SEQ ID NO: 19, SEQ ID NO: 112, and SEQ ID NO: 21, respectively, by 1, 2, or 3 amino acids; or
   (VII) an HCDR1, an HCDR2, and an HCDR3 having the amino acid sequences set forth in SEQ ID NO: 26, SEQ ID NO: 27, and SEQ ID NO: 28, respectively; or an HCDR1, an HCDR2, and an HCDR3 differing from the amino acid sequences set forth in SEQ ID NO: 26, SEQ ID NO: 27, and SEQ ID NO: 28, respectively, by 1, 2, or 3 amino acids; or
   (VIII) an HCDR1, an HCDR2, and an HCDR3 having the amino acid sequences set forth in SEQ ID NO: 31, SEQ ID NO: 32, and SEQ ID NO: 33, respectively; or an HCDR1, an HCDR2, and an HCDR3 differing from the amino acid sequences set forth in SEQ ID NO: 31, SEQ ID NO: 32, and SEQ ID NO: 33, respectively, by 1, 2, or 3 amino acids; or
   (IX) an HCDR1, an HCDR2, and an HCDR3 having the amino acid sequences set forth in SEQ ID NO: 37, SEQ ID NO: 38, and SEQ ID NO: 39, respectively; or an HCDR1, an HCDR2, and an HCDR3 differing from the amino acid sequences set forth in SEQ ID NO: 37, SEQ ID NO: 38, and SEQ ID NO: 39, respectively, by 1, 2, or 3 amino acids; or
   (X) an HCDR1, an HCDR2, and an HCDR3 having the amino acid sequences set forth in SEQ ID NO: 43, SEQ ID NO: 44, and SEQ ID NO: 45, respectively; or an HCDR1, an HCDR2, and an HCDR3 differing from the amino acid sequences set forth in SEQ ID NO: 43, SEQ ID NO: 44, and SEQ ID NO: 45, respectively, by 1, 2, or 3 amino acids;
the light chain variable region comprises:
   (I) an LCDR1, an LCDR2, and an LCDR3 having the amino acid sequences set forth in SEQ ID NO: 51, SEQ ID NO: 52, and SEQ ID NO: 53, respectively; or an LCDR1, an LCDR2, and an LCDR3 differing from the amino acid sequences set forth in SEQ ID NO: 51, SEQ ID NO: 52, and SEQ ID NO: 53, respectively, by 1, 2, or 3 amino acids; or
   (II) an LCDR1, an LCDR2, and an LCDR3 having the amino acid sequences set forth in SEQ ID NO: 59, SEQ ID NO: 60, and SEQ ID NO: 61, respectively; or an LCDR1, an LCDR2, and an LCDR3 differing from the amino acid sequences set forth in SEQ ID NO: 59, SEQ ID NO: 60, and SEQ ID NO: 61, respectively, by 1, 2, or 3 amino acids; or
   (III) an LCDR1, an LCDR2, and an LCDR3 having the amino acid sequences set forth in SEQ ID NO: 67, SEQ ID NO: 68, and SEQ ID NO: 69, respectively; or an LCDR1, an LCDR2, and an LCDR3 differing from the amino acid sequences set forth in SEQ ID NO: 67, SEQ ID NO: 68, and SEQ ID NO: 69, respectively, by 1, 2, or 3 amino acids; or
   (IV) an LCDR1, an LCDR2, and an LCDR3 having the amino acid sequences set forth in SEQ ID NO: 73, SEQ ID NO: 74, and SEQ ID NO: 75, respectively; or an LCDR1, an LCDR2, and an LCDR3 differing from the amino acid sequences set forth in SEQ ID NO: 73, SEQ ID NO: 74, and SEQ ID NO: 75, respectively, by 1, 2, or 3 amino acids; or
   (V) an LCDR1, an LCDR2, and an LCDR3 having the amino acid sequences set forth in SEQ ID NO: 113, SEQ ID NO: 74, and SEQ ID NO: 75, respectively; or an LCDR1, an LCDR2, and an LCDR3 differing from the amino acid sequences set forth in SEQ ID NO: 113, SEQ ID NO: 74, and SEQ ID NO: 75, respectively, by 1, 2, or 3 amino acids; or
   (VI) an LCDR1, an LCDR2, and an LCDR3 having the amino acid sequences set forth in SEQ ID NO: 81, SEQ ID NO: 82, and SEQ ID NO: 83, respectively; or an LCDR1, an LCDR2, and an LCDR3 differing from the amino acid sequences set forth in SEQ ID NO: 81, SEQ ID NO: 82, and SEQ ID NO: 83, respectively, by 1, 2, or 3 amino acids; or
   (VII) an LCDR1, an LCDR2, and an LCDR3 having the amino acid sequences set forth in SEQ ID NO: 86, SEQ ID NO: 87, and SEQ ID NO: 88, respectively; or an LCDR1, an LCDR2, and an LCDR3 differing from the amino acid sequences set forth in SEQ ID NO: 86, SEQ ID NO: 87, and SEQ ID NO: 88, respectively, by 1, 2, or 3 amino acids.

In some embodiments, the antibody or the antigen-binding fragment thereof disclosed herein comprises:
(I) a heavy chain variable region, comprising an HCDR1, an HCDR2, and an HCDR3 having the amino acid sequences set forth in SEQ ID NO: 31, SEQ ID NO: 32, and SEQ ID NO: 33, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(II) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(III) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(IV) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(V) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 81, SEQ ID NO: 82, and SEQ ID NO: 83, respectively; or
(VI) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 86, SEQ ID NO: 87, and SEQ ID NO: 88, respectively; or
(VII) a heavy chain variable region, comprising an HCDR1, an HCDR2, and an HCDR3 having the amino acid sequences set forth in SEQ ID NO: 26, SEQ ID NO: 27, and SEQ ID NO: 28, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(VIII) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(IX) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(X) a heavy chain variable region, comprising an HCDR1, an HCDR2, and an HCDR3 having the amino acid sequences set forth in SEQ ID NO: 43, SEQ ID NO: 44, and SEQ ID NO: 45, respectively; and a light chain variable region, comprising an LCDR1, an LCDR2, and an LCDR3 having the amino acid sequences set forth in SEQ ID NO: 73, SEQ ID NO: 74, and SEQ ID NO: 75, respectively; or
(XI) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XII) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(XIII) a heavy chain variable region, comprising an HCDR1, an HCDR2, and an HCDR3 having the amino acid sequences set forth in SEQ ID NO: 37, SEQ ID NO: 38, and SEQ ID NO: 39, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XIV) a heavy chain variable region, comprising an HCDR1, an HCDR2, and an HCDR3 having the amino acid sequences set forth in SEQ ID NO: 19, SEQ ID NO: 112, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively.

In some embodiments, the antibody disclosed herein is a murine antibody or a chimeric antibody. In some embodiments, the antibody disclosed herein further comprises heavy chain framework regions and/or light chain framework regions derived from a murine antibody.

In some embodiments, the antibody or the antigen-binding fragment thereof disclosed herein comprises a heavy chain variable region and a light chain variable region, wherein
(I) the heavy chain variable region comprises the amino acid sequence set forth in SEQ ID NO: 30, or comprises an amino acid sequence having at least 95%, 96%, 97%, 98%, or 99% sequence identity to the amino acid sequence set forth in SEQ ID NO: 30, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(II) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(III) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(IV) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(V) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 80, or comprises an amino acid sequence having at least 95%, 96%, 97%, 98%, or 99% sequence identity to the amino acid sequence set forth in SEQ ID NO: 80; or
(VI) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 85, or comprises an amino acid sequence having at least 95%, 96%, 97%, 98%, or 99% sequence identity to the amino acid sequence set forth in SEQ ID NO: 85; or
(VII) the heavy chain variable region comprises the amino acid sequence set forth in SEQ ID NO: 25, or comprises an amino acid sequence having at least 95%, 96%, 97%, 98%, or 99% sequence identity to the amino acid sequence set forth in SEQ ID NO: 25, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(VIII) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(IX) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(X) the heavy chain variable region comprises the amino acid sequence set forth in SEQ ID NO: 42, or comprises an amino acid sequence having at least 95%, 96%, 97%, 98%, or 99% sequence identity to the amino acid sequence set forth in SEQ ID NO: 42, and the light chain variable region comprises the amino acid sequence set forth in SEQ ID NO: 72, or comprises an amino acid sequence having at least 95%, 96%, 97%, 98%, or 99% sequence identity to the amino acid sequence set forth in SEQ ID NO: 72; or
(XI) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(XII) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(XIII) the heavy chain variable region comprises the amino acid sequence set forth in SEQ ID NO: 36, or comprises an amino acid sequence having at least 95%, 96%, 97%, 98%, or 99% sequence identity to the amino acid sequence set forth in SEQ ID NO: 36, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50.

In some embodiments, the antibody disclosed herein is a humanized antibody. In some embodiments, the antibody disclosed herein comprises heavy chain framework regions (e.g., heavy chain framework regions contained in an amino acid sequence encoded by a human heavy chain germline gene) and/or light chain framework regions (e.g., light chain framework regions contained in an amino acid sequence encoded by a human light chain germline gene) derived from a human immunoglobulin. The heavy chain framework regions and/or the light chain framework regions optionally comprise one or more (e.g., 1, 2, 3, 4, 5, 6, 7, 8, 9, or 10) back mutations from a human residue to a murine residue.

In some embodiments, the antibody or the antigen-binding fragment thereof disclosed herein comprises:
(I) a heavy chain variable region, comprising the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and
   a light chain variable region, comprising the amino acid sequence set forth in any one of SEQ ID NOs: 54, 55, 56, 57, 62, 63, 64, 65, 70, 71, 76, 77, 78, 79, 84, 89, or 90; or
(II) a heavy chain variable region, comprising an amino acid sequence having at least 95%, 96%, 97%, 98%, or 99% sequence identity to the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and
   a light chain variable region, comprising an amino acid sequence having at least 95%, 96%, 97%, 98%, or 99% sequence identity to the amino acid sequence set forth in any one of SEQ ID NOs: 54, 55, 56, 57, 62, 63, 64, 65, 70, 71, 76, 77, 78, 79, 84, 89, or 90; or
(III) a heavy chain variable region having the amino acid sequence set forth in SEQ ID NO: 46 and a light chain variable region having the amino acid sequence set forth in SEQ ID NO: 78; or

a heavy chain variable region having the amino acid sequence set forth in SEQ ID NO: 47 and a light chain variable region having the amino acid sequence set forth in SEQ ID NO: 78; or
a heavy chain variable region having the amino acid sequence set forth in SEQ ID NO: 48 and a light chain variable region having the amino acid sequence set forth in SEQ ID NO: 77; or
a heavy chain variable region having the amino acid sequence set forth in SEQ ID NO: 8 and a light chain variable region having the amino acid sequence set forth in SEQ ID NO: 63; or
a heavy chain variable region having the amino acid sequence set forth in SEQ ID NO: 24 and a light chain variable region having the amino acid sequence set forth in SEQ ID NO: 54; or
a heavy chain variable region having the amino acid sequence set forth in SEQ ID NO: 8 and a light chain variable region having the amino acid sequence set forth in SEQ ID NO: 54.

In some embodiments, the antibody or the antigen-binding fragment thereof disclosed herein comprises:
(I) a heavy chain, comprising the amino acid sequence set forth in any one of SEQ ID NOs: 92, 94, or 96, and
   a light chain, comprising the amino acid sequence set forth in any one of SEQ ID NOs: 91, 93, or 95; or
(II) a heavy chain, comprising an amino acid sequence having at least 95%, 96%, 97%, 98%, or 99% sequence identity to the amino acid sequence set forth in any one of SEQ ID NOs: 92, 94, or 96, and
   a light chain, comprising an amino acid sequence having at least 95%, 96%, 97%, 98%, or 99% sequence identity to the amino acid sequence set forth in any one of SEQ ID NOs: 91, 93, or 95; or
(III) a heavy chain having the amino acid sequence set forth in SEQ ID NO: 92 and a light chain having the amino acid sequence set forth in SEQ ID NO: 91, or

a heavy chain having the amino acid sequence set forth in SEQ ID NO: 94 and a light chain having the amino acid sequence set forth in SEQ ID NO: 93, or
a heavy chain having the amino acid sequence set forth in SEQ ID NO: 96 and a light chain having the amino acid sequence set forth in SEQ ID NO: 95.

In some embodiments, the antibody or the antigen-binding fragment thereof disclosed herein is a murine antibody, a chimeric antibody, a humanized antibody, or a fully human antibody, or an antigen-binding fragment thereof.

In some embodiments, in the antibody or the antigen-binding fragment thereof disclosed herein, the antigen-binding fragment is Fab, Fab', F(ab')₂, Fv, scFv, or sdAb.

In some embodiments, in the antibody or the antigen-binding fragment thereof disclosed herein, the antibody or the antigen-binding fragment thereof is of any IgG subtype, such as IgG1, IgG2, IgG3, or IgG4, preferably IgG1 subtype or IgG4 subtype.

In another aspect, the present disclosure provides an isolated anti-CGRP antibody or an antigen-binding fragment thereof, having one or more of the following properties:
(1) binding to an epitope on human CGRP protein that is identical to or completely or partially overlaps with that of the anti-CGRP antibody or the antigen-binding fragment thereof disclosed herein; and
(2) competing for binding to an epitope on human CGRP protein with the anti-CGRP antibody or the antigen-binding fragment thereof disclosed herein.

In some embodiments, the epitope comprises amino acid positions 8-19 in human CGRP protein. In some embodiments, the CGRP includes α-CGRP and/or β-CGRP. In some embodiments, the CGRP has the amino acid sequence set forth in SEQ ID NO: 97.

In some embodiments, the antibody disclosed herein is a monoclonal antibody.

The present disclosure further provides a multispecific antibody, comprising the light chain variable region and the heavy chain variable region of the antibody or the antigen-binding fragment thereof disclosed herein.

The present disclosure further provides a single-chain antibody, comprising the light chain variable region and the heavy chain variable region of the antibody or the antigen-binding fragment thereof disclosed herein.

In yet another aspect, the present disclosure provides a polynucleotide molecule, encoding the anti-CGRP antibody or the antigen-binding fragment thereof disclosed herein.

In yet another aspect, the present disclosure provides an expression vector, comprising the polynucleotide molecule disclosed herein, wherein preferably, the vector is a eukaryotic expression vector.

In yet another aspect, the present disclosure provides a host cell, comprising the polynucleotide molecule disclosed herein or the expression vector disclosed herein, wherein preferably, the host cell is a eukaryotic cell, more preferably, a mammalian cell.

In yet another aspect, the present disclosure provides a method for preparing the anti-CGRP antibody or the antigen-binding fragment thereof disclosed herein, comprising expressing the antibody or the antigen-binding fragment thereof in the host cell disclosed herein in a condition suitable for the expression of the antibody or the antigen-binding fragment thereof, and isolating the expressed antibody or antigen-binding fragment thereof from the host cell.

In yet another aspect, the present disclosure provides a pharmaceutical composition, comprising the anti-CGRP antibody or the antigen-binding fragment thereof disclosed herein, the polynucleotide molecule disclosed herein, the expression vector disclosed herein, the host cell disclosed herein, the multispecific antibody disclosed herein, or the single-chain antibody disclosed herein, and a pharmaceutically acceptable carrier or excipient.

In yet another aspect, the present disclosure provides a pharmaceutical combination, comprising the antibody or the antigen-binding fragment thereof disclosed herein, the polynucleotide molecule disclosed herein, the expression vector disclosed herein, the host cell disclosed herein, the multispecific antibody disclosed herein, the single-chain antibody disclosed herein, or the pharmaceutical composition disclosed herein, and one or more additional therapeutic agents.

In yet another aspect, the present disclosure provides use of the antibody or the antigen-binding fragment thereof disclosed herein, the polynucleotide molecule disclosed herein, the expression vector disclosed herein, the host cell disclosed herein, the multispecific antibody disclosed herein, the single-chain antibody disclosed herein, the pharmaceutical composition disclosed herein, or the pharmaceutical combination disclosed herein in preparing a medicament for treating and/or preventing a CGRP-mediated disease or disorder. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In yet another aspect, the present disclosure provides the antibody or the antigen-binding fragment thereof disclosed herein, the polynucleotide molecule disclosed herein, the expression vector disclosed herein, the host cell disclosed herein, the multispecific antibody disclosed herein, the single-chain antibody disclosed herein, the pharmaceutical composition disclosed herein, or the pharmaceutical combination disclosed herein for use in treating and/or preventing a CGRP-mediated disease or disorder. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In yet another aspect, the present disclosure provides a method for treating and/or preventing a CGRP-mediated disease or disorder, comprising: administering to a subject in need a therapeutically or prophylactically effective amount of the antibody or the antigen-binding fragment thereof disclosed herein, the polynucleotide molecule disclosed herein, the expression vector disclosed herein, the host cell disclosed herein, the multispecific antibody disclosed herein, the single-chain antibody disclosed herein, the pharmaceutical composition disclosed herein, or the pharmaceutical combination disclosed herein. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In yet another aspect, the present disclosure provides a kit, comprising the antibody or the antigen-binding fragment thereof disclosed herein, the polynucleotide molecule disclosed herein, the expression vector disclosed herein, the host cell disclosed herein, the multispecific antibody disclosed herein, the single-chain antibody disclosed herein, the pharmaceutical composition disclosed herein, or the pharmaceutical combination disclosed herein, and preferably, further comprising an administration device.

In yet another aspect, the present disclosure provides a method for detecting the presence of CGRP in a sample by using the antibody or the antigen-binding fragment thereof disclosed herein or a detection composition comprising the antibody or the antigen-binding fragment thereof. In some embodiments, the method comprises: contacting the antibody or the antigen-binding fragment thereof disclosed herein or the detection composition comprising the antibody or the antigen-binding fragment thereof with the sample.

### BRIEF DESCRIPTION OF THE DRAWINGS

FIGs. 1 to 3 illustrate the blocking activity of chimeric antibodies against the binding of CGRP to CALCRL-RAMP1 by luciferase assay.
FIGs. 4 to 6 illustrate the blocking activity of humanized antibodies against the binding of CGRP to CALCRL-RAMP1 by luciferase assay.
FIGs. 7-1 to 7-4 illustrate the inhibitory effects of anti-CGRP chimeric antibodies on capsaicin-induced vasodilation in rats.
FIGs. 8-1 to 8-4 illustrate the inhibitory effects of anti-CGRP humanized antibodies on capsaicin-induced vasodilation in rats.
FIG. 9 illustrates the assay for the effects of antibodies on mean arterial pressure.
FIG. 10 illustrates the assay for the effects of antibodies on middle meningeal artery (MMA) vascular diameter dilation.
FIG. 11 illustrates the detection results of kinetic characterization parameters for the binding of C245 Fab to human CGRP polypeptide fragments.
FIG. 12 illustrates the detection results of kinetic characterization parameters for the binding of hu224 Fab to human CGRP polypeptide fragments.
FIG. 13 illustrates the association-dissociation curves of antibodies and the antigen human α-CGRP at different pH values.

### DETAILED DESCRIPTION

### Definitions

Unless otherwise stated, the present disclosure will be implemented with conventional techniques of molecular biology (including recombinant techniques), microbiology, cytobiology, biochemistry, and immunology, which are all within the skill of the art.

In order to facilitate the understanding of the present disclosure, some technical and scientific terms are specifically defined as follows. Unless otherwise explicitly defined elsewhere herein, all technical and scientific terms used herein have the same meaning as commonly understood by those of ordinary skill in the art to which the present disclosure belongs. For definitions and terminology in the art, those skilled can refer specifically to *Current Protocols in Molecular Biology* (Ausubel). The abbreviations for amino acid residues are standard 3-letter and/or 1-letter codes used in the art to denote one of the 20 commonly used L-amino acids. The singular forms used herein (including claims) include their corresponding plural forms, unless otherwise explicitly specified herein.

The term "about" used in combination with a numerical value is intended to encompass the numerical values in a range from a lower limit less than the specified numerical value by 5% to an upper limit greater than the specified numerical value by 5%, including but not limited to ±5%, ±2%, ±1%, and ±0.1%, as these variations are suitable for implementing the disclosed methods.

The term "and/or" should be interpreted as any one of the options or a combination of any two or more of the options.

As used herein, the term "or" should be interpreted as having the same meaning as "and/or" defined above. For example, when items in a list are separated, "or" or "and/or" should be interpreted as being inclusive, i.e., including at least one of the list of numbers or elements, but also including more than one, and, optionally, additional unlisted items. Only in cases where contrary terms such as "only one", "exactly one" or "consisting of ..." as used in the claims are explicitly indicated, will it refer to the one number solely listed or one element of the list.

Unless the contrary is explicitly indicated, as used herein, the terms "a" and "one" should be interpreted as "at least one".

The terms "calcitonin gene-related peptide" and "CGRP" as used herein refer to any form of calcitonin gene-related peptide and variants thereof that maintain at least a portion of the activity of CGRP. For example, CGRP may be α-CGRP or β-CGRP. CGRP as used herein includes CGRPs of native sequences from all mammalian species, e.g., human, canine, feline, equine, and bovine.

The term "percent (%) amino acid sequence identity", or simply "identity", is defined as the percentage of amino acid residues in a candidate amino acid sequence that are identical to those in a reference amino acid sequence after aligning the amino acid sequences (with gaps introduced if necessary) to achieve maximum percent sequence identity without considering any conservative substitution as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring the alignment, including any algorithm required to give the maximum alignment for the full length of the aligned sequences.

The terms "activity" and "bioactivity", or the terms "biological property" and "biological characteristic" are used interchangeably herein and include, but are not limited to, epitope/antigen affinity and specificity, the ability to neutralize or antagonize CGRP activity *in vivo* or *in vitro,* the IC₅₀, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain a high expression level of the protein in mammalian cells. The aforementioned properties or characteristics are observed, determined, or assessed using techniques well known in the art, including but not limited to ELISA, FACS, or BIACORE plasma resonance analysis, unlimited *in vitro* or *in vivo* neutralization assays, receptor binding, cytokine or growth factor production and/or secretion, signal transduction, and immunohistochemistry of tissue sections of different sources (including human, primate or any other source).

The term "antibody" refers to any form of an antibody with the desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "isolated antibody" refers to the purified state of a binding compound, and, in this case, means that the molecule is substantially free of other biomolecules, such as nucleic acids, proteins, lipids, sugars, or other substances such as cell debris and growth medium. The term "isolate(d)" does not mean the complete absence of such substances or the absence of water, buffers, or salts, unless they are present in amounts that will significantly interfere with the experimental or therapeutic use of the binding compounds described herein.

The term "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically comprise a large number of antibodies targeting (or specific for) different epitopes. The modifier "monoclonal" indicates the characteristic of an antibody obtained from a substantially homogeneous population of antibodies, and should not be construed as producing the antibody by any particular method.

The term "full-length antibody" refers to an immunoglobulin molecule comprising at least four peptide chains when present naturally, including two heavy (H) chains and two light (L) chains linked to each other by disulfide bonds. Each of the heavy chains consists of a heavy chain variable region (abbreviated herein as VH) and a heavy chain constant region (abbreviated herein as CH). The heavy chain constant region consists of 3 domains, i.e., CH1, CH2, and CH3. Each of the light chains consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, and FR4. The variable regions of the heavy and light chains contain binding domains that interact with antigens. The constant regions of an antibody can mediate the binding of immunoglobulins to host tissues or factors, including various cells of the immune system (e.g., effector cells) and the first component (C1q) of classical complement system.

The term "antigen-binding fragment" of an antibody ("parent antibody") includes a fragment or a derivative of the antibody, generally comprising at least one fragment of an antigen-binding region or variable region (e.g., one or more CDRs) of a parent antibody which retains at least some of the binding specificity of the parent antibody. Examples of the binding fragment of an antibody include, but are not limited to, Fab, Fab', F(ab')₂, and Fv fragments; a diabody; a linear antibody; a single-chain antibody molecule, e.g., sc-Fv; and a nanobody and a multispecific antibody formed by fragments of the antibody. The binding fragment or the derivative generally retains at least 10% of the antigen-binding activity of the parent antibody when the antigen-binding activity is expressed on a molar concentration basis. Preferably, the binding fragment or the derivative retains at least 20%, 50%, 70%, 80%, 90%, 95%, or 100% or more of the antigen binding affinity of the parent antibody. It is also contemplated that the antigen-binding fragment of the antibody may comprise conservative or non-conservative amino acid substitutions that do not significantly alter its bioactivity (referred to as "conservative variants" or "function-conservative variants" of the antibody). The term "binding compound" refers to both the antibody and the binding fragment thereof.

The term "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

The term "domain antibody" is an immunofunctional immunoglobulin fragment that contains only the heavy chain variable region or the light chain variable region. In certain cases, two or more VH regions are covalently linked to a peptide linker to form a bivalent domain antibody. The 2 VH regions of the bivalent domain antibody may target the same or different antigens.

The term "bivalent antibody" comprises 2 antigen-binding sites. In certain cases, the 2 binding sites have the same antigen specificity. However, the bivalent antibody may be bispecific.

The term "diabody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of another chain to form two antigen-binding sites.

The term "murine antibody" or "hybridoma antibody" in the present disclosure refers to an anti-human CGRP monoclonal antibody prepared according to the knowledge and skills in the art. The preparation is performed by injecting the CGRP antigen to a test subject and then isolating hybridomas expressing antibodies with the desired sequences or functional properties.

The term "chimeric antibody" is an antibody having variable domains of a first antibody and constant domains of a second antibody, wherein the first and second antibodies are from different species. Typically, the variable domains are obtained from an antibody of an animal such as a rodent ("parent antibody"), and the constant domain sequences are obtained from a human antibody, such that the resulting chimeric antibody is less likely to induce an adverse immune response in a human subject as compared to the parent rodent antibody.

The term "humanized antibody" refers to an antibody form comprising sequences from both human and non-human (such as mouse and rat) antibodies. In general, the humanized antibody comprises substantially all of at least one, and typically two, variable domains, in which all or substantially all of the hypervariable loops are equivalent to those of a non-human immunoglobulin and all or substantially all of the framework regions (FRs) are those of a human immunoglobulin sequence. The humanized antibody may optionally comprise at least a portion of a human immunoglobulin constant region (Fc).

The term "fully human antibody" refers to an antibody that comprises only human immunoglobulin sequences. A fully human antibody may contain mouse glycochains if produced in mice, mouse cells or hybridomas derived from mouse cells. Likewise, the "mouse antibody" refers to an antibody that comprises only mouse immunoglobulin sequences. Alternatively, a fully human antibody may contain rat glycochains if produced in rats, rat cells or hybridomas derived from rat cells. Likewise, the "rat antibody" refers to an antibody that comprises only rat immunoglobulin sequences.

The "isotype" of an antibody refers to the type of antibody (e.g., IgM, IgE, and IgG (such as IgG1, IgG2, or IgG4)) provided by the heavy chain constant region gene. The isotype also includes modified forms of one of these types in which modifications have been made to alter the Fc function, e.g., to enhance or attenuate the effector function or the binding to Fc receptors.

The term "Fc region" is used herein to define the C-terminal region of an immunoglobulin heavy chain comprising at least a part of constant regions. The term includes native sequence Fc regions and variant Fc regions. In some embodiments, the Fc region of a human IgG heavy chain extends from Cys226 or Pro230 to the carboxyl terminus of the heavy chain. However, the C-terminal lysine (Lys447) of the Fc region may or may not be present (numbering in this paragraph is according to the EU numbering system, also known as the EU index, e.g., Rabat et al., Sequences of Proteins of Immunological Interest, 5th Ed. Public Health Service, National Institutes of Health, Bethesda, MD, 1991).

The term "epitope" refers to a protein determinant capable of specific binding to an antibody. The epitopes are usually composed of a variety of chemically active surface molecules such as amino acids or sugar side chains and usually have specific three-dimensional structural characteristics as well as specific charge characteristics. Conformational epitopes and non-conformational epitopes differ in that the binding to the former rather than the latter shall fail in the presence of denaturing solvents.

The term "cross-reaction" as described herein refers to the binding to antigenic fragments of the same target molecule of human, monkey and/or murine (mouse or rat) origin. Thus, the "cross-reaction" should be interpreted as an interspecies reaction with the same molecule X expressed in different species. The cross-reaction specificity of monoclonal antibodies recognizing human CGRP, and monkey and/or murine (mouse or rat) CGRP can be determined by FACS analysis.

The "affinity" or "binding affinity" refers to the inherent binding affinity that reflects the interaction between members of a binding pair. The affinity of molecule X for its partner Y can be generally represented by the equilibrium dissociation constant (K_{D}), which is a ratio of the dissociation rate constant (k_{dis}) to the association rate constant (kₒₙ). The affinity can be measured using common methods known in the art. One specific method for measuring affinity is the ForteBio kinetic binding assay as described herein.

The term "not bind to" a protein or cell means the absence of binding to the protein or cell, or the absence of binding with high affinity, i.e., binding to the protein or cell with a K_{D} of 1.0 × 10⁻⁶ M or higher, more preferably 1.0 × 10⁻⁵ M or higher, more preferably 1.0 × 10⁻⁴ M or higher, 1.0 × 10⁻³ M or higher, and more preferably 1.0 × 10⁻² M or higher.

The term "high affinity" for IgG antibodies refers to a K_{D} for the antigen of 1.0 × 10⁻⁶ M or less, preferably 5.0 × 10⁻⁸ M or less, more preferably 1.0 × 10⁻⁸ M or less, 5.0 × 10⁻⁹ M or less, and more preferably 1.0 × 10⁻⁹ M or less. For other antibody subtypes, the "high-affinity" binding may vary. For example, the "high-affinity" binding of the IgM subtype refers to a K_{D} of 10⁻⁶ M or less, preferably 10⁻⁷ M or less, and more preferably 10⁻⁸ M or less.

The terms "nucleic acid", "polynucleotide", "nucleic acid molecule", and "polynucleotide molecule" refer to deoxyribonucleic acid (DNA) or ribonucleic acid (RNA) and polymers thereof in either single- or double-stranded form. Unless explicitly limited, the term includes nucleic acids containing known analogs of natural nucleotides that have binding properties similar to that of the reference nucleic acid and are metabolized in a manner similar to naturally occurring nucleotides (see U.S. Pat. No. 8,278,036 to Kariko et al., which discloses an mRNA molecule with uridine replaced by pseudouridine, a method for synthesizing the mRNA molecule, and a method for delivering a therapeutic protein *in vivo*)*.* Unless otherwise specified, a particular nucleic acid sequence also implicitly includes conservatively modified variants thereof (e.g., degenerate codon substitutions), alleles, orthologs, SNPs, and complementary sequences as well as the sequence explicitly indicated. Specifically, the degenerate codon substitutions can be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The "construct" refers to any recombinant polynucleotide molecule (such as plasmid, cosmid, virus, autonomously replicating polynucleotide molecule, phage, or linear or circular single- or double-stranded DNA or RNA polynucleotide molecule) derived from any origin, capable of genomic integration or autonomous replication, and comprising a polynucleotide molecule where one or more polynucleotide molecules have been linked in a functionally operative manner (i.e., operably linked). The recombinant construct typically comprises the polynucleotide disclosed herein operably linked to transcription initiation regulatory sequences that will direct transcription of the polynucleotide in a host cell. Both heterologous and non-heterologous (i.e., endogenous) promoters can be used to direct the expression of the nucleic acid of the present disclosure.

The "vector" refers to any recombinant polynucleotide construct that can be used for transformation purpose (i.e., the introduction of heterologous DNA into a host cell). One such vector is the "plasmid", which refers to a circular double-stranded DNA loop into which additional DNA segments can be ligated. Another such vector is the viral vector, in which additional DNA segments can be ligated into the viral genome. Some certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and are thereby replicated along with the host genome. In addition, some certain vectors are capable of directing the expression of operably linked genes. Such vectors are referred to herein as "expression vectors".

The term "expression vector" as used herein refers to a nucleic acid molecule capable of replicating and expressing a target gene when transformed, transfected, or transduced into a host cell. The expression vector comprises one or more phenotypic selectable markers and an origin of replication to ensure the maintenance of the vector and to provide amplification in the host if needed.

Unless otherwise or explicitly specified in the context, the "activation", "stimulation", and "treatment" for a cell or a receptor may have the same meaning, for example, the cell or the receptor is activated, stimulated, or treated with a ligand. The "ligand" includes natural and synthetic ligands, such as cytokines, cytokine variants, analogs, mutant proteins, and binding compounds derived from antibodies. The "ligand" also includes small molecules, such as peptidomimetics of cytokines and peptidomimetics of antibodies. The "activation" may refer to the activation of a cell regulated by internal mechanisms and external or environmental factors. The "response/reaction", e.g., a response of a cell, a tissue, an organ, or an organism, includes changes in biochemical or physiological behaviors (e.g., concentration, density, adhesion or migration, gene expression rate, or differentiation state within a biological compartment), where the changes are associated with the activation, stimulation, or treatment, or are associated with an internal mechanism such as genetic programming.

As used herein, the term "treat", "treating", or "treatment" for any disease or disorder refers in one embodiment to ameliorating the disease or disorder (i.e., slowing or arresting or reducing the progression of the disease or at least one of its clinical symptoms). In another embodiment, the "treat", "treating", or "treatment" refers to alleviating or ameliorating at least one physical parameter, including those physical parameters that may not be discernible by the patient. In another embodiment, the "treat", "treating", or "treatment" refers to modulating the disease or disorder, physically (e.g., stabilization of discernible symptoms), physiologically (e.g., stabilization of physical parameters), or both. Unless explicitly described herein, methods for assessing the treatment and/or prevention of a disease are generally known in the art.

The "subject" includes any human or non-human animals. The term "non-human animal" includes all vertebrates, e.g., mammals and non-mammals, such as non-human primates, sheep, dogs, cats, horses, cattle, chickens, amphibians, and reptiles. As used herein, the term "cyno" or "cynomolgus" refers to cynomolgus monkeys.

The administration "in combination with" one or more other therapeutic agents includes simultaneous (concurrent) administration and sequential administration in any order.

The "therapeutically effective amount", "therapeutically effective dose", and "effective amount" refer to an amount of the CGRP antibody or the antigen-binding fragment thereof disclosed herein that is effective in preventing or ameliorating one or more symptoms of a disease or condition or the progression of the disease or condition when administered alone or in combination with an additional therapeutic agent to a cell, a tissue, or a subject. The therapeutically effective dose also refers to an amount of the antibody or the antigen-binding fragment thereof sufficient to cause the amelioration of symptoms, e.g., an amount for treating, curing, preventing, or ameliorating a related medical condition or promoting the treatment, cure, prevention, or amelioration of such a condition. When an active ingredient is administered to an individual alone, the therapeutically effective dose refers to the amount of the ingredient only. For combined use, the therapeutically effective dose refers to the combined amount of active ingredients that produce the therapeutic effect, regardless of whether such active ingredients are administered in combination, sequentially, or simultaneously. The effective amount of a therapeutic agent will increase a diagnostic index or parameter by at least 10%, generally at least 20%, preferably at least about 30%, more preferably at least 40%, and most preferably at least 50%.

The "pharmaceutically acceptable carrier" refers to a component in a pharmaceutical formulation or composition other than the active component that is non-toxic to the subject. The pharmaceutically acceptable carrier includes, but is not limited to, a buffer, an excipient, a stabilizer, or a preservative.

The term "cancer" is used herein to refer to a group of cells that exhibit abnormally high levels of proliferation and growth. The cancer may be benign (also referred to as benign tumor), premalignant, or malignant. The cancer cell may be a solid cancer cell or a leukemia cancer cell. The term "tumor" as used herein refers to one or more cells comprising cancer. The term "tumor growth" is used herein to refer to the proliferation or growth of one or more cells comprising cancer, which results in a corresponding increase in the size or extent of the cancer.

### Anti-CGRP Antibodies

In one aspect, the present disclosure provides an antibody or an antigen-binding fragment thereof specifically binding to CGRP. The term "anti-CGRP antibody", "anti-CGRP", "CGRP antibody", or "CGRP-binding antibody" refers to an antibody that is capable of binding to a CGRP protein or a fragment thereof with sufficient affinity such that the antibody can be used as a diagnostic and/or therapeutic agent targeting CGRP.

In some embodiments, the present disclosure provides an antibody that binds to CGRP protein (e.g., α-CGRP and/or β-CGRP).

In some embodiments, the antibody disclosed herein binds to human CGRP protein. In some embodiments, the antibody disclosed herein binds to an epitope on human CGRP protein comprising amino acid positions 8-19.

In some embodiments, the anti-CGRP antibody disclosed herein comprises:
(I) a heavy chain variable region, comprising an HCDR1, an HCDR2, and an HCDR3 having the amino acid sequences set forth in SEQ ID NO: 31, SEQ ID NO: 32, and SEQ ID NO: 33, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(II) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(III) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(IV) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(V) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 81, SEQ ID NO: 82, and SEQ ID NO: 83, respectively; or
(VI) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 86, SEQ ID NO: 87, and SEQ ID NO: 88, respectively; or
(VII) a heavy chain variable region, comprising an HCDR1, an HCDR2, and an HCDR3 having the amino acid sequences set forth in SEQ ID NO: 26, SEQ ID NO: 27, and SEQ ID NO: 28, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(VIII) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(IX) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(X) a heavy chain variable region, comprising an HCDR1, an HCDR2, and an HCDR3 having the amino acid sequences set forth in SEQ ID NO: 43, SEQ ID NO: 44, and SEQ ID NO: 45, respectively; and a light chain variable region, comprising an LCDR1, an LCDR2, and an LCDR3 having the amino acid sequences set forth in SEQ ID NO: 73, SEQ ID NO: 74, and SEQ ID NO: 75, respectively; or
(XI) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XII) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(XIII) a heavy chain variable region, comprising an HCDR1, an HCDR2, and an HCDR3 having the amino acid sequences set forth in SEQ ID NO: 37, SEQ ID NO: 38, and SEQ ID NO: 39, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XIV) a heavy chain variable region, comprising an HCDR1, an HCDR2, and an HCDR3 having the amino acid sequences set forth in SEQ ID NO: 19, SEQ ID NO: 112, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively.

In some embodiments, the antibody or the antigen-binding fragment thereof disclosed herein comprises a heavy chain variable region and/or a light chain variable region, wherein
the heavy chain variable region comprises:
   (I) an HCDR1, an HCDR2, and an HCDR3 having the amino acid sequences set forth in SEQ ID NO: 2, SEQ ID NO: 3, and SEQ ID NO: 4, respectively; or
   (II) an HCDR1, an HCDR2, and an HCDR3 having the amino acid sequences set forth in SEQ ID NO: 2, SEQ ID NO: 110, and SEQ ID NO: 4, respectively; or
   (III) an HCDR1, an HCDR2, and an HCDR3 having the amino acid sequences set forth in SEQ ID NO: 11, SEQ ID NO: 12, and SEQ ID NO: 13, respectively; or
   (IV) an HCDR1, an HCDR2, and an HCDR3 having the amino acid sequences set forth in SEQ ID NO: 11, SEQ ID NO: 111, and SEQ ID NO: 13, respectively; or
   (V) an HCDR1, an HCDR2, and an HCDR3 having the amino acid sequences set forth in SEQ ID NO: 19, SEQ ID NO: 20, and SEQ ID NO: 21, respectively; or
   (VI) an HCDR1, an HCDR2, and an HCDR3 having the amino acid sequences set forth in SEQ ID NO: 19, SEQ ID NO: 112, and SEQ ID NO: 21, respectively; or
   (VII) an HCDR1, an HCDR2, and an HCDR3 having the amino acid sequences set forth in SEQ ID NO: 26, SEQ ID NO: 27, and SEQ ID NO: 28, respectively; or
   (VIII) an HCDR1, an HCDR2, and an HCDR3 having the amino acid sequences set forth in SEQ ID NO: 31, SEQ ID NO: 32, and SEQ ID NO: 33, respectively; or
   (IX) an HCDR1, an HCDR2, and an HCDR3 having the amino acid sequences set forth in SEQ ID NO: 37, SEQ ID NO: 38, and SEQ ID NO: 39, respectively; or
   (X) an HCDR1, an HCDR2, and an HCDR3 having the amino acid sequences set forth in SEQ ID NO: 43, SEQ ID NO: 44, and SEQ ID NO: 45, respectively;
the light chain variable region comprises:
   (I) an LCDR1, an LCDR2, and an LCDR3 having the amino acid sequences set forth in SEQ ID NO: 51, SEQ ID NO: 52, and SEQ ID NO: 53, respectively; or
   (II) an LCDR1, an LCDR2, and an LCDR3 having the amino acid sequences set forth in SEQ ID NO: 59, SEQ ID NO: 60, and SEQ ID NO: 61, respectively; or
   (III) an LCDR1, an LCDR2, and an LCDR3 having the amino acid sequences set forth in SEQ ID NO: 67, SEQ ID NO: 68, and SEQ ID NO: 69, respectively; or
   (IV) an LCDR1, an LCDR2, and an LCDR3 having the amino acid sequences set forth in SEQ ID NO: 73, SEQ ID NO: 74, and SEQ ID NO: 75, respectively; or
   (V) an LCDR1, an LCDR2, and an LCDR3 having the amino acid sequences set forth in SEQ ID NO: 113, SEQ ID NO: 74, and SEQ ID NO: 75, respectively; or
   (VI) an LCDR1, an LCDR2, and an LCDR3 having the amino acid sequences set forth in SEQ ID NO: 81, SEQ ID NO: 82, and SEQ ID NO: 83, respectively; or
   (VII) an LCDR1, an LCDR2, and an LCDR3 having the amino acid sequences set forth in SEQ ID NO: 86, SEQ ID NO: 87, and SEQ ID NO: 88, respectively.

In some embodiments, the antibody or the antigen-binding fragment thereof disclosed herein comprises:
(1) the HCDR1, the HCDR2, and the HCDR3 of the heavy chain variable region set forth in SEQ ID NO: 46, and the LCDR1, the LCDR2, and the LCDR3 of the light chain variable region set forth in SEQ ID NO: 78; or
(2) the HCDR1, the HCDR2, and the HCDR3 of the heavy chain variable region set forth in SEQ ID NO: 47, and the LCDR1, the LCDR2, and the LCDR3 of the light chain variable region set forth in SEQ ID NO: 78; or
(3) the HCDR1, the HCDR2, and the HCDR3 of the heavy chain variable region set forth in SEQ ID NO: 48, and the LCDR1, the LCDR2, and the LCDR3 of the light chain variable region set forth in SEQ ID NO: 77; or
(4) the HCDR1, the HCDR2, and the HCDR3 of the heavy chain variable region set forth in SEQ ID NO: 8, and the LCDR1, the LCDR2, and the LCDR3 of the light chain variable region set forth in SEQ ID NO: 63; or
(5) the HCDR1, the HCDR2, and the HCDR3 of the heavy chain variable region set forth in SEQ ID NO: 24, and the LCDR1, the LCDR2, and the LCDR3 of the light chain variable region set forth in SEQ ID NO: 54; or
(6) the HCDR1, the HCDR2, and the HCDR3 of the heavy chain variable region set forth in SEQ ID NO: 8, and the LCDR1, the LCDR2, and the LCDR3 of the light chain variable region set forth in SEQ ID NO: 54.

In some embodiments, the antibody or the antigen-binding fragment thereof disclosed herein comprises a heavy chain variable region and a light chain variable region, wherein
(I) the heavy chain variable region comprises the amino acid sequence set forth in SEQ ID NO: 30, or comprises an amino acid sequence having at least 95%, 96%, 97%, 98%, or 99% sequence identity to the amino acid sequence set forth in SEQ ID NO: 30, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(II) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(III) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(IV) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(V) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 80, or comprises an amino acid sequence having at least 95%, 96%, 97%, 98%, or 99% sequence identity to the amino acid sequence set forth in SEQ ID NO: 80; or
(VI) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 85, or comprises an amino acid sequence having at least 95%, 96%, 97%, 98%, or 99% sequence identity to the amino acid sequence set forth in SEQ ID NO: 85; or
(VII) the heavy chain variable region comprises the amino acid sequence set forth in SEQ ID NO: 25, or comprises an amino acid sequence having at least 95%, 96%, 97%, 98%, or 99% seqtauence identity to the amino acid sequence set forth in SEQ ID NO: 25, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(VIII) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(IX) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(X) the heavy chain variable region comprises the amino acid sequence set forth in SEQ ID NO: 42, or comprises an amino acid sequence having at least 95%, 96%, 97%, 98%, or 99% sequence identity to the amino acid sequence set forth in SEQ ID NO: 42, and the light chain variable region comprises the amino acid sequence set forth in SEQ ID NO: 72, or comprises an amino acid sequence having at least 95%, 96%, 97%, 98%, or 99% sequence identity to the amino acid sequence set forth in SEQ ID NO: 72; or
(XI) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(XII) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(XIII) the heavy chain variable region comprises the amino acid sequence set forth in SEQ ID NO: 36, or comprises an amino acid sequence having at least 95%, 96%, 97%, 98%, or 99% sequence identity to the amino acid sequence set forth in SEQ ID NO: 36, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50.

In some embodiments, the antibody or the antigen-binding fragment thereof disclosed herein comprises an HCDR1, an HCDR2, an HCDR3, an LCDR1, an LCDR2, and an LCDR3 as follows:
HCDR1: DX¹NMH (SEQ ID NO: 103) or GYTMN (SEQ ID NO: 43), wherein X¹ is F or Y;
HCDR2: X²X³X⁴PYNX⁵X⁶X⁷X⁸YNX⁹X¹⁰FX¹¹X¹² (SEQ ID NO: 104) or LINPYIGNTHYNQKFKD (SEQ ID NO: 44), wherein X² is Y or N, X³ is I or V, X⁴ is Y, S, or F, X⁵ is G or A, X⁶ is D, G, V, or H, X⁷ is V, T, S, or A, X⁸ is V or A, X⁹ is Q or R, X¹⁰ is K, N, or D, X¹¹ is T, K, N, or R, and X¹² is N, F, or S;
HCDR3: EGLGGY (SEQ ID NO: 39) or ELDSGFDY (SEQ ID NO: 45);
LCDR1: X¹³X¹⁴SQNIZYX¹⁵X¹⁶ (SEQ ID NO: 105), wherein X¹³ is K, Q, or R, X¹⁴ is A or S, Z is DYDGYA (SEQ ID NO: 114), DFDGYA (SEQ ID NO: 115), DGNT (SEQ ID NO: 116), VHSNGDT (SEQ ID NO: 117), VHSNADT (SEQ ID NO: 118), DYDGYT (SEQ ID NO: 119), or DYGGYS (SEQ ID NO: 120), X¹⁵ is L or M, and X¹⁶ is N or E;
LCDR2: SX¹⁷FNLES (SEQ ID NO: 106) or KVSNRFS (SEQ ID NO: 74), wherein X¹⁷ is V or I; and
LCDR3: X¹⁸QSX¹⁹X²⁰X²¹PYT (SEQ ID NO: 107) or YQGSHVPWT (SEQ ID NO: 75), wherein X¹⁸ is Q, X¹⁹ is N or H, X²⁰ is E or D, X²¹ is F, V, Y, or A.

In some embodiments, the antibody or the antigen-binding fragment thereof comprises: an HCDR1 having the amino acid sequence set forth in SEQ ID NOs: 2, 11, 19, 26, 31, 37, or 43; an HCDR2 having the amino acid sequence set forth in SEQ ID NOs: 3, 110, 12, 111, 20, 112, 27, 32, 38, or 44; an HCDR3 having the amino acid sequence set forth in SEQ ID NOs: 4, 13, 21, 28, 33, 39, or 45; an LCDR1 having the amino acid sequence set forth in SEQ ID NOs: 51, 59, 67, 73, 113, 81, or 86; an LCDR2 having the amino acid sequence set forth in SEQ ID NOs: 52, 60, 68, 74, 82, or 87; and an LCDR3 having the amino acid sequence set forth in SEQ ID NOs: 53, 61, 69, 75, 83, or 88.

In some embodiments, the antibody or the antigen-binding fragment thereof disclosed herein comprises:
(I) a heavy chain variable region, comprising the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and
   a light chain variable region, comprising the amino acid sequence set forth in any one of SEQ ID NOs: 54, 55, 56, 57, 62, 63, 64, 65, 70, 71, 76, 77, 78, 79, 84, 89, or 90; or
(II) a heavy chain variable region, comprising an amino acid sequence having at least 95%, 96%, 97%, 98%, or 99% sequence identity to the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and
a light chain variable region, comprising an amino acid sequence having at least 95%, 96%, 97%, 98%, or 99% sequence identity to the amino acid sequence set forth in any one of SEQ ID NOs: 54, 55, 56, 57, 62, 63, 64, 65, 70, 71, 76, 77, 78, 79, 84, 89, or 90.

In some embodiments, the antibody or the antigen-binding fragment thereof disclosed herein comprises an HCDR1, an HCDR2, an HCDR3, an LCDR1, an LCDR2, and an LCDR3 as follows:
HCDR1: DX₁NMH or GYTMN, wherein X₁ is F or Y;
HCDR2: X₂X₃X₄PYNX₅X₆X₇X₈YNX₉X₁₀FX₁₁X₁₂ or LINPYIGNTHYNQKFKD, wherein X₂ is Y or N, X₃ is I, X₄ is Y, X₅ is G or A, X₆ is D or G, X₇ is V or T, X₈ is V, X₉ is Q, X₁₀ is K or N, X₁₁ is T or N, and X₁₂ is N;
HCDR3: EGLGGY or ELDSGFDY;
LCDR1: X₁₃X₁₄SQNIZYX₁₅X₁₆, wherein X₁₃ is K or R, X₁₄ is A or S, Z is DYDGYA, DFDGYA, or VHSNGDT, X₁₅ is L or M, and X₁₆ is N or E;
LCDR2: SX₁₇FNLES or KVSNRFS, wherein X₁₇ is V; and
LCDR3: X₁₈QSX₁₉X₂₀X₂₁PYT or YQGSHVPWT, wherein X₁₈ is Q, X₁₉ is N or H, X₂₀ is E, and X₂₁ is F or V.

In some embodiments, the antibody or the antigen-binding fragment thereof disclosed herein comprises a heavy chain variable region and a light chain variable region, wherein the heavy chain variable region comprises the amino acid sequence set forth in any one of SEQ ID NOs: 46, 47, 48, 8, and 24, or comprises an amino acid sequence having at least 95%, 96%, 97%, 98%, or 99% sequence identity to the amino acid sequence set forth in any one of SEQ ID NOs: 46, 47, 48, 8, and 24; and/or, the light chain variable region comprises the amino acid sequence set forth in any one of SEQ ID NOs: 77, 78, 54, and 63, or comprises an amino acid sequence having at least 95%, 96%, 97%, 98%, or 99% sequence identity to the amino acid sequence set forth in any one of SEQ ID NOs: 77, 78, 54, and 63.

In some embodiments, the antibody or the antigen-binding fragment thereof disclosed herein comprises an HCDR1, an HCDR2, and an HCDR3 having the amino acid sequences set forth in SEQ ID NO: 43, SEQ ID NO: 44, and SEQ ID NO: 45, respectively, and an LCDR1, an LCDR2, and an LCDR3 having the amino acid sequences set forth in SEQ ID NO: 73, SEQ ID NO: 74, and SEQ ID NO: 75, respectively.

In some embodiments, the antibody or the antigen-binding fragment thereof disclosed herein comprises a heavy chain variable region and a light chain variable region, wherein the heavy chain variable region comprises the amino acid sequence set forth in any one of SEQ ID NOs: 42, 46, 47, 48, and 49 (preferably any one of SEQ ID NOs: 46, 47, and 48), or comprises an amino acid sequence having at least 95%, 96%, 97%, 98%, or 99% sequence identity to any one of the foregoing sequences; and/or, the light chain variable region comprises the amino acid sequence set forth in any one of SEQ ID NOs: 72, 76, 77, 78, and 79 (preferably any one of SEQ ID NOs: 77 and 78), or comprises an amino acid sequence having at least 95%, 96%, 97%, 98%, or 99% sequence identity to any one of the foregoing sequences.

In some embodiments, the antibody or the antigen-binding fragment thereof disclosed herein comprises an HCDR1, an HCDR2, an HCDR3, an LCDR1, an LCDR2, and an LCDR3 as follows:
HCDR1: DX₁NMH, wherein X₁ is F or Y;
HCDR2: X₂X₃X₄PYNX₅X₆X₇X₈YNX₉X₁₀FX₁₁X₁₂, wherein X₂ is Y or N, X₃ is I, X₄ is Y, X₅ is G or A, X₆ is D or G, X₇ is V or T, X₈ is V, X₉ is Q, X₁₀ is K or N, X₁₁ is T or N, and X₁₂ is N;
HCDR3: EGLGGY;
LCDR1: X₁₃X₁₄SQNIZYX₁₅X₁₆, wherein X₁₃ is K or R, X₁₄ is A or S, Z is DYDGYA or DFDGYA, X₁₅ is L or M, and X₁₆ is N or E;
LCDR2: SX₁₇FNLES, wherein X₁₇ is V; and
LCDR3: X₁₈QSX₁₉X₂₀X₂₁PYT, wherein X₁₈ is Q, X₁₉ is N or H, X₂₀ is E, and X₂₁ is F or V.

In some embodiments, the antibody or the antigen-binding fragment thereof disclosed herein comprises a heavy chain variable region and a light chain variable region, wherein the heavy chain variable region comprises the amino acid sequence set forth in any one of SEQ ID NOs: 8 and 24, or comprises an amino acid sequence having at least 95%, 96%, 97%, 98%, or 99% sequence identity to the amino acid sequence set forth in any one of SEQ ID NOs: 8 and 24; and/or, the light chain variable region comprises the amino acid sequence set forth in any one of SEQ ID NOs: 54 and 63, or comprises an amino acid sequence having at least 95%, 96%, 97%, 98%, or 99% sequence identity to the amino acid sequence set forth in any one of SEQ ID NOs: 54 and 63.

In some embodiments, the antibody or the antigen-binding fragment thereof disclosed herein comprises a heavy chain variable region and a light chain variable region, wherein
(I) the heavy chain variable region comprises the amino acid sequence set forth in SEQ ID NO: 46, or comprises an amino acid sequence having at least 95%, 96%, 97%, 98%, or 99% sequence identity to the amino acid sequence set forth in SEQ ID NO: 46, and the light chain variable region comprises the amino acid sequence set forth in SEQ ID NO: 78, or comprises an amino acid sequence having at least 95%, 96%, 97%, 98%, or 99% sequence identity to the amino acid sequence set forth in SEQ ID NO: 78; or
(II) the heavy chain variable region comprises the amino acid sequence set forth in SEQ ID NO: 47, or comprises an amino acid sequence having at least 95%, 96%, 97%, 98%, or 99% sequence identity to the amino acid sequence set forth in SEQ ID NO: 47, and the light chain variable region comprises the amino acid sequence set forth in SEQ ID NO: 78, or comprises an amino acid sequence having at least 95%, 96%, 97%, 98%, or 99% sequence identity to the amino acid sequence set forth in SEQ ID NO: 78; or
(III) the heavy chain variable region comprises the amino acid sequence set forth in SEQ ID NO: 48, or comprises an amino acid sequence having at least 95%, 96%, 97%, 98%, or 99% sequence identity to the amino acid sequence set forth in SEQ ID NO: 48, and the light chain variable region comprises the amino acid sequence set forth in SEQ ID NO: 77, or comprises an amino acid sequence having at least 95%, 96%, 97%, 98%, or 99% sequence identity to the amino acid sequence set forth in SEQ ID NO: 77; or
(IV) the heavy chain variable region comprises the amino acid sequence set forth in SEQ ID NO: 8, or comprises an amino acid sequence having at least 95%, 96%, 97%, 98%, or 99% sequence identity to the amino acid sequence set forth in SEQ ID NO: 8, and the light chain variable region comprises the amino acid sequence set forth in SEQ ID NO: 63, or comprises an amino acid sequence having at least 95%, 96%, 97%, 98%, or 99% sequence identity to the amino acid sequence set forth in SEQ ID NO: 63; or
(V) the heavy chain variable region comprises the amino acid sequence set forth in SEQ ID NO: 24, or comprises an amino acid sequence having at least 95%, 96%, 97%, 98%, or 99% sequence identity to the amino acid sequence set forth in SEQ ID NO: 24, and the light chain variable region comprises the amino acid sequence set forth in SEQ ID NO: 54, or comprises an amino acid sequence having at least 95%, 96%, 97%, 98%, or 99% sequence identity to the amino acid sequence set forth in SEQ ID NO: 54; or
(VI) the heavy chain variable region comprises the amino acid sequence set forth in SEQ ID NO: 8, or comprises an amino acid sequence having at least 95%, 96%, 97%, 98%, or 99% sequence identity to the amino acid sequence set forth in SEQ ID NO: 8, and the light chain variable region comprises the amino acid sequence set forth in SEQ ID NO: 54, or comprises an amino acid sequence having at least 95%, 96%, 97%, 98%, or 99% sequence identity to the amino acid sequence set forth in SEQ ID NO: 54.

In some embodiments, the antibody or the antigen-binding fragment thereof disclosed herein comprises: a heavy chain variable region having the amino acid sequence set forth in SEQ ID NO: 46 and a light chain variable region having the amino acid sequence set forth in SEQ ID NO: 78; or
a heavy chain variable region having the amino acid sequence set forth in SEQ ID NO: 47 and a light chain variable region having the amino acid sequence set forth in SEQ ID NO: 78; or
a heavy chain variable region having the amino acid sequence set forth in SEQ ID NO: 48 and a light chain variable region having the amino acid sequence set forth in SEQ ID NO: 77; or
a heavy chain variable region having the amino acid sequence set forth in SEQ ID NO: 8 and a light chain variable region having the amino acid sequence set forth in SEQ ID NO: 63; or
a heavy chain variable region having the amino acid sequence set forth in SEQ ID NO: 24 and a light chain variable region having the amino acid sequence set forth in SEQ ID NO: 54; or
a heavy chain variable region having the amino acid sequence set forth in SEQ ID NO: 8 and a light chain varitaable region having the amino acid sequence set forth in SEQ ID NO: 54.

In some embodiments, the antibody or the antigen-binding fragment thereof disclosed herein comprises a constant region derived from a human immunoglobulin. In some embodiments, the antibody or the antigen-binding fragment thereof disclosed herein comprises a heavy chain constant region set forth in SEQ ID NO: 108 and/or a light chain constant region set forth in SEQ ID NO: 109.

In some embodiments, the antibody or the antigen-binding fragment thereof disclosed herein comprises:
(I) a heavy chain, comprising the amino acid sequence set forth in any one of SEQ ID NOs: 92, 94, or 96, and
   a light chain, comprising the amino acid sequence set forth in any one of SEQ ID NOs: 91, 93, or 95; or
(II) a heavy chain, comprising an amino acid sequence having at least 95%, 96%, 97%, 98%, or 99% sequence identity to the amino acid sequence set forth in any one of SEQ ID NOs: 92, 94, or 96, and
   a light chain, comprising an amino acid sequence having at least 95%, 96%, 97%, 98%, or 99% sequence identity to the amino acid sequence set forth in any one of SEQ ID NOs: 91, 93, or 95; or
(III) a heavy chain having the amino acid sequence set forth in SEQ ID NO: 92 and a light chain having the amino acid sequence set forth in SEQ ID NO: 91, or

a heavy chain having the amino acid sequence set forth in SEQ ID NO: 94 and a light chain having the amino acid sequence set forth in SEQ ID NO: 93, or
a heavy chain having the amino acid sequence set forth in SEQ ID NO: 96 and a light chain having the amino acid sequence set forth in SEQ ID NO: 95.

In some embodiments, the antibody or the antigen-binding fragment thereof disclosed herein is a murine antibody, a chimeric antibody, a humanized antibody, or a fully human antibody, or an antigen-binding fragment thereof.

Any suitable method for producing antibodies may be employed to produce the antibody disclosed herein. CGRP in any suitable form may be used as the immunogen (antigen) for antibody production. By way of example rather than limitation, any CGRP variant or fragment thereof may be used as the immunogen. In some embodiments, hybridoma cells that produce murine monoclonal anti-human CGRP antibodies can be produced by methods well known in the art.

Antibodies derived from rodents (e.g., mice) may induce undesired immunogenicity of the antibodies when used as therapeutic agents *in vivo.* Repeated use of these antibodies induces immune responses in the human body to therapeutic antibodies. Such immune responses result in at least a loss of therapeutic efficacy and, in severe cases, a potentially lethal allergic reaction. One method for reducing the immunogenicity of rodent antibodies comprises producing chimeric antibodies, in which the mouse variable region is fused to the human constant region (Liu et al., (1987) Proc. Natl. Acad. Sci. USA 84:3439-43). However, the preservation of intact rodent variable regions in a chimeric antibody can still induce deleterious immunogenicity in patients. The grafting of complementarity-determining region (CDR) loops of the rodent variable domain onto a human framework (i.e., humanization) has been used to further minimize rodent sequences (Jones et al., (1986) Nature 321:522; Verhoeyen et al., (1988) Science 239:1534*).*

In some embodiments, the chimeric or humanized antibody of the present disclosure can be prepared on the basis of the sequence of the prepared murine monoclonal hybridoma antibody. DNA encoding an immunoglobulin having heavy and light chains can be obtained from a murine hybridoma of interest and engineered to comprise non-murine (e.g., human) immunoglobulin sequences using standard molecular biology techniques.

In some embodiments, to prepare the chimeric CGRP antibody of the present disclosure, the chimeric heavy chain and the chimeric light chain can be obtained by operably linking the immunoglobulin heavy chain and light chain variable regions of hybridoma origin to the human IgG constant regions, respectively, using methods known in the art (see, e.g., U.S. Pat. No. 4,816,567 to Cabilly et al.). In some embodiments, the chimeric antibody of the present disclosure comprises constant regions which can be selected from subtypes of any human IgG, e.g., IgG1, IgG2, IgG3, and IgG4, preferably IgG4.

In some embodiments, the chimeric CGRP antibody of the present disclosure can be obtained by "mixing and matching" a chimeric light chain expression plasmid with a chimeric heavy chain expression plasmid to transfect expression cells. The CGRP binding of such "mixed and matched" antibodies can be assayed using the above binding assay and other conventional binding assays (e.g., ELISA).

The precise amino acid sequence boundaries of the variable region CDRs of the antibody disclosed herein can be determined using any of many well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures.

Unless otherwise stated, the boundaries of the CDRs of the antibody disclosed herein can be determined by those skilled in the art according to any scheme (e.g., different assignment systems or combinations) in the art. In some embodiments, the CDRs of the antibody disclosed herein are defined by Kabat, Chothia, AbM, Contact, North, or IMGT. In some embodiments, the CDRs of the antibody disclosed herein are defined by Kabat.

It should be noted that the boundaries of the CDRs of the variable regions of the same antibody obtained based on different assignment systems may differ. That is, the CDR sequences of the variable regions of the same antibody defined by different assignment systems are different. Thus, when it comes to defining an antibody with particular CDR sequences defined herein, the scope of the antibody also encompasses antibodies where the variable region sequences comprise the particular CDR sequences but the claimed CDR boundaries differ from the particular CDR boundaries defined in the present invention due to the application of different schemes (e.g., different assignment systems or combinations).

Antibodies with different specificities (i.e., different binding sites for different antigens) have different CDRs. However, although CDRs vary from antibody to antibody, only a limited number of amino acid positions within a CDR are directly involved in antigen binding. The smallest overlapping region can be determined using at least two of the Kabat, Chothia, AbM, Contact and North methods, thereby providing the "smallest binding unit" for antigen binding. The smallest binding unit may be a sub-portion of the CDR. As will be apparent to those skilled in the art, the residues in the remainder of the CDR sequence can be determined by the antibody structure and protein folding. Thus, variants of any of the CDRs presented herein are also contemplated by the present disclosure. For example, in a variant of one CDR, the amino acid residue of the smallest binding unit may remain unchanged, while the remaining CDR residues defined according to Kabat or Chothia may be replaced by conservative amino acid residues.

For the humanized antibodies described herein, murine CDR regions can be inserted into human germline framework regions using methods known in the art. See U.S. Pat. No. 5,225,539 to Winter et al., and U.S. Pat. Nos. 5,530,101; 5,585,089; 5,693,762 and 6,180,370 to Queen et al.

In some embodiments, the amino acid difference includes an amino acid deletion, insertion, or substitution. In some embodiments, the anti-CGRP antibody or the antigen-binding fragment thereof disclosed herein includes those antibodies having an amino acid sequence that has been mutated by an amino acid deletion, insertion, or substitution but still has at least about 90%, 91%, 92%, 93%, 94%, 95%, 96%, 97%, 98%, 99% or 100% identity to the amino acid sequences of the antibodies described above (particularly in the CDR regions set forth in the above sequences). In some embodiments, as compared to the CDR regions set forth in a particular sequence, the antibody disclosed herein has no more than 1, 2, 3, 4, or 5 amino acid mutations (deletions, insertions, or substitutions) in the CDR regions. In some embodiments, as compared to the frame regions in a particular sequence, the antibody disclosed herein has no more than 1, 2, 3, 4, or 5 amino acid mutations (deletions, insertions or substitutions) in the frame regions.

In some embodiments, polynucleotide molecules encoding the antibody disclosed herein include those that have been mutated by nucleotide deletion, insertion, or substitution but still have at least about 60%, 70%, 80%, 90%, 95%, or 100% identity to the corresponding CDR coding regions set forth in the above sequences.

In some embodiments, the CGRP antibody is an IgG antibody, e.g., IgG1, IgG2, IgG3, or IgG4 antibody or a modified form thereof, as described below.

In some embodiments, one or more amino acid modifications may be introduced into an Fc region of the antibody provided herein, thus producing an Fc region variant. The Fc region variant may comprise a human Fc region sequence (e.g., human IgG1, IgG2, IgG3, or IgG4 Fc region) which comprises an amino acid modification (e.g., substitution) at one or more amino acid positions.

In some embodiments, an antibody modified by cysteine engineering may need to be produced, e.g., "sulfo-MAb", wherein one or more residues of the antibody are substituted by a cysteine residue.

In some embodiments, the antibody disclosed herein can be further modified to contain other non-protein moieties known in the art and readily available. Suitable moieties for antibody derivatization include, but are not limited to, water-soluble polymers. Non-limiting examples of the water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (e.g., glycerol), polyvinyl alcohol, and mixtures thereof.

In some embodiments, the antibody disclosed herein can be further used to prepare a multispecific antibody. The multispecific antibody comprises a second functional module (e.g., a second antibody) having a binding specificity different from the antibody disclosed herein, thereby being capable of binding to at least two different binding sites and/or target molecules. Thus, the present disclosure further provides a multispecific antibody comprising the light chain variable region and the heavy chain variable region of the antibody or the antigen-binding fragment thereof disclosed herein.

In some embodiments, the antibody disclosed herein can be further used to prepare a single-chain antibody. Thus, the present disclosure further provides a single-chain antibody comprising the light chain variable region and the heavy chain variable region of the antibody or the antigen-binding fragment thereof disclosed herein.

### Antibody Expression

In yet another aspect, the present disclosure provides a polynucleotide molecule encoding the anti-CGRP antibody or the antigen-binding fragment thereof disclosed herein. The polynucleotide molecule may comprise a polynucleotide molecule encoding the amino acid sequence of the light chain variable region and/or the heavy chain variable region of the antibody, or comprise a polynucleotide molecule encoding the amino acid sequence of the light chain and/or the heavy chain of the antibody.

In yet another aspect, the present disclosure provides an expression vector, comprising the polynucleotide molecule disclosed herein, wherein preferably, the vector is a eukaryotic expression vector. In some embodiments, the polynucleotide molecule disclosed herein is contained in one or more expression vectors.

In yet another aspect, the present disclosure provides a host cell, comprising the polynucleotide molecule disclosed herein or the expression vector disclosed herein, wherein preferably, the host cell is a eukaryotic cell, more preferably, a mammalian cell.

In yet another aspect, the present disclosure provides a method for preparing the anti-CGRP antibody or the antigen-binding fragment thereof disclosed herein, comprising expressing the antibody or the antigen-binding fragment thereof in the host cell disclosed herein in a condition suitable for the expression of the antibody or the antigen-binding fragment thereof, and isolating the expressed antibody or antigen-binding fragment thereof from the host cell.

The present disclosure provides a mammalian host cell for expressing the recombinant antibody of the present disclosure, including a number of immortalized cell lines available from the American Type Culture Collection (ATCC). Those cell lines include, in particular, Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells, and many other cell lines. The mammalian host cell includes human, mouse, rat, dog, monkey, pig, goat, cattle, horse, and hamster cells. Particularly preferred cell lines are selected by determining which cell line has a high expression level.

In one embodiment, the present disclosure provides a method for preparing an anti-CGRP antibody, comprising introducing an expression vector into a mammalian host cell, and culturing the host cell for a period of time sufficient to allow the expression of the antibody in the host cell, or more preferably to allow the secretion of the antibody into a medium in which the host cell grows, thereby producing the antibody. The antibody can be isolated from the medium using standard protein purification methods.

It is likely that antibodies expressed by different cell lines or in transgenic animals have different glycosylations from each other. However, all antibodies encoded by the nucleic acid molecules provided herein or comprising the amino acid sequences provided herein are integral parts of the present disclosure, regardless of the glycosylation of the antibody. Likewise, in some certain embodiments, nonfucosylated antibodies are advantageous because they generally have more potent efficacy *in vitro* and *in vivo* than their fucosylated counterparts, and are unlikely to be immunogenic because their glycan structures are normal components of natural human serum IgG.

### Pharmaceutical Composition and Pharmaceutical Formulation

In yet another aspect, the present disclosure provides a pharmaceutical composition, comprising the anti-CGRP antibody or the antigen-binding fragment thereof disclosed herein, the polynucleotide molecule disclosed herein, the expression vector disclosed herein, the host cell disclosed herein, or the multispecific antibody or the single-chain antibody disclosed herein, and a pharmaceutically acceptable carrier or excipient. It will be appreciated that the anti-CGRP antibody or the pharmaceutical composition thereof disclosed herein can be integrated with a suitable carrier, excipient, and additional reagents in a formulation for administration in combination, thus providing improved transfer, delivery, tolerance, etc.

The term "pharmaceutical composition" refers to a formulation that allows the bioactivity of active ingredients contained therein to be present in an effective form and does not comprise additional ingredients having toxicity unacceptable to a subject to which the formulation is administered.

The pharmaceutical formulation comprising the anti-CGRP antibody disclosed herein, preferably in the form of an aqueous solution or a lyophilized formulation, may be prepared by mixing the anti-CGRP antibody disclosed herein having the desired purity with one or more optional pharmaceutical adjuvants (Remington's Pharmaceutical Sciences, 16th edition, Osol, A. Ed. (1980)).

The pharmaceutical composition or formulation of the present disclosure may further comprise one or more additional active ingredients which are required for a specific indication being treated, preferably active ingredients having complementary activities that do not adversely affect one another. In some embodiments, the additional active ingredients are chemotherapeutic agents, immune checkpoint inhibitors, growth inhibitors, antibiotics, or various known anti-tumor or anti-cancer agents, which are suitably present in combination in amounts that are effective for the purpose intended. In some embodiments, the pharmaceutical composition disclosed herein further comprises a composition of a polynucleotide molecule encoding the anti-CGRP antibody.

In yet another aspect, the present disclosure provides a pharmaceutical combination, comprising the antibody or the antigen-binding fragment thereof disclosed herein, the polynucleotide molecule disclosed herein, the expression vector disclosed herein, the host cell disclosed herein, the multispecific antibody or the single-chain antibody disclosed herein, or the pharmaceutical composition disclosed herein, and one or more additional therapeutic agents.

In yet another aspect, the present disclosure provides a kit, comprising the antibody or the antigen-binding fragment thereof disclosed herein, the polynucleotide molecule disclosed herein, the expression vector disclosed herein, the host cell disclosed herein, the multispecific antibody or the single-chain antibody disclosed herein, or the pharmaceutical composition or the pharmaceutical combination disclosed herein.

### Pharmaceutical Use and Therapeutic Method

Any of the anti-CGRP antibodies disclosed herein can be used in a therapeutic method. It will also be appreciated that when discussing an "antibody", a composition comprising the antibody is also included. The anti-CGRP antibody disclosed herein may be used in a therapeutically or prophylactically effective amount for any therapeutic or prophylactic method described in any one of the embodiments of the present disclosure.

In yet another aspect, the present disclosure provides use of the antibody or the antigen-binding fragment thereof disclosed herein, the polynucleotide molecule disclosed herein, the expression vector disclosed herein, the host cell disclosed herein, the multispecific antibody or the single-chain antibody disclosed herein, or the pharmaceutical composition or the pharmaceutical combination disclosed herein in preparing a medicament for treating and/or preventing a CGRP-mediated disease or disorder. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In yet another aspect, the present disclosure provides the antibody or the antigen-binding fragment thereof disclosed herein, the polynucleotide molecule disclosed herein, the expression vector disclosed herein, the host cell disclosed herein, the multispecific antibody or the single-chain antibody disclosed herein, or the pharmaceutical composition or the pharmaceutical combination disclosed herein for use in treating and/or preventing a CGRP-mediated disease or disorder. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In yet another aspect, the present disclosure provides a method for treating and/or preventing a CGRP-mediated disease or disorder, comprising: administering to a subject in need the antibody or the antigen-binding fragment thereof disclosed herein, the polynucleotide molecule disclosed herein, the expression vector disclosed herein, the host cell disclosed herein, the multispecific antibody or the single-chain antibody disclosed herein, or the pharmaceutical composition or the pharmaceutical combination disclosed herein. Preferably, the disease or disorder is a symptom having CGRP-associated pain; and more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In some embodiments, the pain described herein is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

In some embodiments, the routes of administration of the present disclosure include, but are not limited to, oral administration, intravenous administration, subcutaneous administration, intramuscular administration, intra-arterial administration, intra-articular administration (e.g., in arthritic joints), administration by inhalation or aerosol delivery, intratumoral administration, and the like.

### Methods for Diagnosis and Detection

In yet another aspect, the present disclosure provides a method for detecting the presence of CGRP in a sample using the antibody or the antigen-binding fragment thereof disclosed herein. The term "detection" as used herein includes quantitative or qualitative detection. In some embodiments, the sample is a biological sample. In some certain embodiments, the biological sample is blood, serum, or other liquid samples of biological origin. In some certain embodiments, the biological sample includes cells or tissues. The method comprises contacting the antibody or the antigen-binding fragment thereof disclosed herein or a detection composition comprising the antibody or the antigen-binding fragment thereof with the sample, and detecting the presence or absence of a conjugate or a binding signal produced by the binding of the antibody or the antigen-binding fragment thereof to CGRP. For a detection purpose, the antibody or the antigen-binding fragment thereof disclosed herein may be labeled to indicate whether the conjugate is formed. In some certain embodiments, the methods may be used for diagnostic purposes, or for non-diagnostic purposes.

In yet another aspect, the present disclosure provides use of the antibody or the antigen-binding fragment thereof disclosed herein or a detection composition comprising the antibody or antigen-binding fragment thereof in preparing a detection reagent for detecting the presence of CGRP in a sample.

The present disclosure includes all combinations of particular embodiments. Further embodiments of the present disclosure and the full scope of applicability will become apparent from the detailed description provided below. However, it will be appreciated that the detailed description and the specific examples, while indicating preferred embodiments of the present disclosure, are provided by way of illustration only, as various changes and modifications within the spirit and scope of the present disclosure will become apparent to those skilled in the art from the detailed description. All publications, patents, and patent applications cited herein, including the citations, are hereby incorporated by reference in their entireties for all purposes.

The antibody disclosed herein can be prepared using a variety of methods well known to those skilled in the art, including the specific embodiments listed below, embodiments formed by combinations thereof with other methods, and equivalents well known to those skilled in the art; preferred embodiments include, but are not limited to, the examples of the present disclosure.

The following abbreviations are used in the present disclosure:
KLH for hemocyanin;
PBS for phosphate-buffered saline;
FBS for fetal bovine serum;
HRP for horse radish peroxidase;
BSA for bovine serum albumin;
MMA for middle meningeal artery.

### Examples

The present disclosure is illustrated by the following examples, which, however, are not intended to be limiting in any way. The present disclosure has been described in detail herein, and the specific embodiments are also disclosed. It will be apparent to those skilled in the art that various changes and modifications can be made to the specific embodiments of the present disclosure without departing from the spirit and scope of the present disclosure.

### Example 1. Preparation of recombinant proteins for anti-CGRP antibody preparation and testing

### 1.1 Preparation of human fragment (7-37) α-CGRP polypeptide (for mouse immunization and hybridoma screening)

The human α-CGRP (7-37) polypeptide used to immunize mice was chemically synthesized by Anaspec and conjugated to KLH protein. The molecular weight ratio of the polypeptide to KLH carrier protein was 1:4.

### 1.2 Preparation of human full-length (1-37) α-CGRP polypeptide (for hybridoma screening)

The human α-CGRP (1-37) polypeptide for hybridoma screening was prepared by chemical synthesis in Anaspec and conjugating to biotin protein, and the full-length polypeptide was purchased from Abcam.

### 1.3 Preparation of human full-length (1-37) β-CGRP polypeptide (for hybridoma screening)

### Purchased from Anaspec.

### 1.4 Preparation of rat fragment (7-37) α-CGRP polypeptide (for hybridoma screening)

### Purchased from Tocris.

### Example 2. Preparation of mouse hybridoma cells

### 2.1. Immunization of animals

1.5 Human α-CGRP (7-37)-KLH-conjugated protein dissolved in phosphate-buffered saline (PBS) was used as
the antigen to immunize 5 BALB/c mice (purchased from Simonsen Laboratories of Gilroy, female BALB/c, aged 8 weeks). Following the primary immunization (20 µg polypeptide/mouse), the booster immunization (10 µg polypeptide/mouse) was given once every 1 or 2 weeks for a total of 6 doses.

### 2.2. Cell fusion

On day four after the last booster immunization, the inguinal lymph nodes, the popliteal lymph nodes, and the spleens of the mice were collected and ground in normal saline containing EDTA. A suspension rich in lymphocytes was collected and fused with mouse myeloma cells Sp2/0 using the conventional electrotransfection method. The fusion product was cultured in a DMEM complete medium containing 1:50 HAT (hypoxanthine H, amethopterin A, and thymidine T) for 5 days to screen for target fusion cells (hybridoma cells). Then the medium was changed to a DMEM complete medium containing 1:50 HT (hypoxanthine H and thymidine T), and the culture continued until the end of the screening.

The composition of the DMEM complete medium was as follows: 15% FBS (fetal bovine serum) + 1:50 L-Glutamine + 100 U/mL penicillin-streptomycin + 1:100 OPI (oxaloacetic acid, pyruvic acid, and insulin), and the incubator conditions were 8% CO₂ and 37 °C.

### Example 3. Screening of mouse hybridoma cells and performance tests of obtained anti-CGRP murine antibodies

Among 7680 polyclonal hybridoma cell strains, 273 hybridoma cell strains capable of secreting an antibody binding to human α-CGRP were selected by ELISA. Among the 273 hybridoma cell strains, 29 hybridoma cell strains expressed an antibody capable of binding to rat α-CGRP. Finally, 13 hybridoma cell strains expressing an antibody capable of binding to human β-CGRP were screened from the 29 hybridoma cells by ELISA, and the antibodies secreted by the hybridoma cells were purified and analyzed. Further, the mRNAs of the 13 hybridoma cells were extracted and the coding sequences for antibody variable regions were determined. The 13 hybridoma antibodies thus preliminarily selected were designated 1A2, 1B2, 1F5, 2A2, 2B8, 2C2, 2E4, 3B1, 3C1, 3C5, 3E5, 3F7, and 3H10, respectively.

The procedures and results are shown below.

### 3.1 Binding assay of hybridoma antibodies to human α-CGRP by ELISA

384-well or 96-well MaxiSorp plates (Thermo Fisher Scientific Nunc) were coated with 1 µg/mL human α-CGRP (Abcam) and then blocked with 1% BSA. The hybridoma cell culture supernatant was then transferred to the MaxiSorp plates and incubated for 30 minutes. An HRP-labeled goat anti-mouse secondary antibody was added for the assay.

The binding of the antibodies to human α-CGRP was verified by indirect ELISA. 96-well MaxiSorp plates (Thermo Fisher Scientific Nunc) were coated with 2 µg/mL NeutrAvidin and then blocked with 1% BSA. 1 µg/mL biotinylated human α-CGRP (Anaspec) and the hybridoma cell culture supernatant were then added and co-incubated for 30 minutes in 96-well plates, respectively. An HRP-labeled goat anti-mouse secondary antibody was added for the assay.

### 3.2 Binding assay of hybridoma antibodies to rat α-CGRP by ELISA

96-well MaxiSorp plates (Thermo Fisher Scientific Nunc) were coated with 1 µg/mL rat α-CGRP (Tocris) and then blocked with 1% BSA. The hybridoma cell culture supernatant was then transferred to the MaxiSorp plates and incubated for 30 minutes. An HRP-labeled goat anti-mouse secondary antibody was added for the assay.

### 3.3 Binding assay of hybridoma antibodies to human β-CGRP by ELISA

96-well MaxiSorp plates (Thermo Fisher Scientific Nunc) were coated with 1 µg/mL human β-CGRP (Anaspec) and then blocked with 1% BSA. The hybridoma cell culture supernatant was then transferred to the MaxiSorp plates and incubated for 30 minutes. An HRP-labeled goat anti-mouse secondary antibody was added for the assay.

### Example 4. Sequencing of variable region sequences of anti-CGRP murine antibodies (according to Kabat or IMGT)

The DNA coding sequences corresponding to the variable regions of the anti-CGRP murine antibodies were sequenced by a degenerate primer PCR-based method. Briefly, the 13 hybridoma cell strains were separately subjected to expansion culture and centrifuged at 1000 rpm, and the cells were collected. Total RNA was extracted with Trizol. A first-strand cDNA was synthesized using the total RNA as a template, and then the DNA coding sequences for variable regions were amplified by PCR using the first-strand cDNA as a subsequent template. The PCR primer used was based on an Ig-primer set. PCR products were recovered and purified. The products of the amplification were sequenced to give the amino acid sequences of the heavy chain variable regions and light chain variable regions of the anti-CGRP murine antibodies. The numbers of heavy and light chains of the hybridoma antibodies are shown in Table 1

**Table 1. Numbers of heavy and light chains for the hybridoma antibody**

| | 1A2 | 1B2 | 1F5 | 2A2 | 2B8 | 2C2 | 2E4 | 3B1 | 3C1 | 3C5 | 3E5 | 3F7 | 3H10 |
|---|---|---|---|---|---|---|---|---|---|---|---|---|---|
| Heavy chain | 2 | 1 | 1 | 1 | 2 | 1 | 1 | 1 | 1 | 1 | 1 | 2 | 1 |
| Light chain | 3 | 1 | 3 | 1 | 3 | 1 | 1 | 1 | 1 | 2 | 1 | 3 | 2 |

### Example 5. Construction of anti-CGRP chimeric antibodies

According to the evaluation results of Example 3, anti-CGRP chimeric antibodies were constructed by selecting the light chain and heavy chain variable regions of anti-CGRP murine antibodies 1A2, 1B2, 1F5, 2A2, 2B8, 2C2, 2E4, 3B1, 3C1, 3C5, 3E5, 3F7, and 3H10.

The coding sequences for the heavy chain constant region Fc and the light chain constant region κ were cloned from human B lymphocytes (from Beijing Blood Institute) and introduced into the pCDNA3.1 plasmid to form vectors HXT4S and HXT2, respectively. The heavy and light chain variable region coding sequences of the anti-CGRP murine antibodies described above were synthesized by Tsingke and subcloned into HXT4S and HXT2, respectively, by BSPQI cleavage. Various chimeric heavy and light chain expression plasmids were mixed and paired and used to transfect expression cells (CHOK1 18, Suzhou TopAlliance), and 368 chimeric antibodies were obtained. Their numbers and corresponding variable region amino acid sequences are detailed in Table 2.

**Table 2. Numbers of chimeric antibodies and sources of their heavy chain variable regions and light chain variable regions**

| | **1A2-HC1** | **1A2-HC2** | **1B2-HC** | **1F5-HC** | **2A2-HC** | **2B8-HC1** | **2B8-HC2** | **2C2-HC** | **2E4-HC** | **3B1-HC** | **3C1-HC** | **3E5-HC** | **3C5-HC** | **3F7-HC1** | **3F7-HC2** | **3H10-HC** |
|---|---|---|---|---|---|---|---|---|---|---|---|---|---|---|---|---|
| **1A2-LC1** | C1 | C24 | C47 | C70 | C93 | C116 | C139 | C162 | C185 | C208 | C231 | C254 | C277 | C300 | C323 | C346 |
| **1A2-LC2** | C2 | C25 | C48 | C71 | C94 | C117 | C140 | C163 | C186 | C209 | C232 | C255 | C278 | C301 | C324 | C347 |
| **1A2-LC3** | C3 | C26 | C49 | C72 | C95 | C118 | C141 | C164 | C187 | C210 | C233 | C256 | C279 | C302 | C325 | C348 |
| **1B2-LC** | C4 | C27 | C50 | C73 | C96 | C119 | C142 | C165 | C188 | C211 | C234 | C257 | C280 | C303 | C326 | C349 |
| **1F5-LC1** | C5 | -C28 | C51 | C74 | C97 | C120 | C143 | C166 | C189 | C212 | C235 | C258 | C281 | C304 | C327 | C350 |
| **1F5-LC2** | C6 | C29 | C52 | C75 | C98 | C121 | C144 | C167 | C190 | C213 | C236 | C259 | C282 | C305 | C328 | C351 |
| **1F5-LC3** | C7 | C30 | C53 | C76 | C99 | C122 | C145 | C168 | C191 | C214 | C237 | C260 | C283 | C306 | C329 | C352 |
| **2A2-LC** | C8 | C31 | C54 | C77 | C100 | C123 | C146 | C169 | C192 | C215 | C238 | C261 | C284 | C307 | C330 | C353 |
| **2B8-LC1** | C9 | C32 | C55 | C78 | C101 | C124 | C147 | C170 | C193 | C216 | C239 | C262 | C285 | C308 | C331 | C354 |
| **2B8-LC2** | C10 | C33 | C56 | C79 | C102 | C125 | C148 | C171 | C194 | C217 | C240 | C263 | C286 | C309 | C332 | C355 |
| **2B8-LC3** | C11 | C34 | C57 | C80 | C103 | C126 | C149 | C172 | C195 | C218 | C241 | C264 | C287 | C310 | C333 | C356 |
| **2C2-LC** | C12 | C35 | C58 | C81 | C104 | C127 | C150 | C173 | C196 | C219 | C242 | C265 | C288 | C311 | C334 | C357 |
| **2E4-LC** | C13 | C36 | C59 | C82 | C105 | C128 | C151 | C174 | C197 | C220 | C243 | C266 | C289 | C312 | C335 | C358 |
| **3B1-LC** | C14 | C37 | C60 | C83 | C106 | C129 | C152 | C175 | C198 | C221 | C244 | C267 | C290 | C313 | C336 | C359 |
| **3C1-LC** | C15 | C38 | C61 | C84 | C107 | C130 | C153 | C176 | C199 | C222 | C245 | C268 | C291 | C314 | C337 | C360 |
| **3E5-LC** | C16 | C39 | C62 | C85 | C108 | C131 | C154 | C177 | C200 | C223 | C246 | C269 | C292 | C315 | C338 | C361 |
| **3C5-LC1** | C17 | C40 | C63 | C86 | C109 | C132 | C155 | C178 | C201 | C224 | C247 | C270 | C293 | C316 | C339 | C362 |
| **3C5-LC2** | C18 | C41 | C64 | C87 | C110 | C133 | C156 | C179 | C202 | C225 | C248 | C271 | C294 | C317 | C340 | C363 |
| **3F7-LC1** | C19 | C42 | C65 | C88 | C111 | C134 | C157 | C180 | C203 | C226 | C249 | C272 | C295 | C318 | C341 | C364 |
| **3F7-LC2** | C20 | C43 | C66 | C89 | C112 | C135 | C158 | C181 | C204 | C227 | C250 | C273 | C296 | C319 | C342 | C365 |
| **3F7-LC3** | C21 | C44 | C67 | C90 | C113 | C136 | C159 | C182 | C205 | C228 | C251 | C274 | C297 | C320 | C343 | C366 |
| **3H10-LC1** | C22 | C45 | C68 | C91 | C114 | C137 | C160 | C183 | C206 | C229 | C252 | C275 | C298 | C321 | C344 | C367 |
| **3H10-LC2** | C23 | C46 | C69 | C92 | C115 | C138 | C161 | C184 | C207 | C230 | C253 | C276 | C299 | C322 | C345 | C368 |

### Example 6. Screening of chimeric antibodies

The best chimeric antibodies were screened according to the ability of the chimeric antibodies in Table 3 to bind to full-length (1-37) CGRP and truncated (1-19) CGRP and the cellular biological activity thereof to block the binding of CGRP to CALCRL-RAMP1. The procedures and results are shown below.

### 6.1 Binding assay of chimeric antibodies to full-length (1-37) CGRP by ELISA (cell culture supernatant)

Streptavidin was diluted to 0.5 µg/mL by using PBS (Hyclone) and added into a plate at 100 µL/well. The plate was incubated in a thermostat incubator at 37 °C for 90 min and then washed. 2% BSA was added to the plate at 200 µL/well, and the plate was incubated in the thermostat incubator at 37 °C for 90 min and washed. Biotin-huCGRPaSA was diluted to 2.0 µg/mL with 2% BSA and added into the plate at 100 µL/well, and the plate was incubated in the thermostat incubator at 37 °C for 60 min. The reference antibody (47-RM) was diluted to 2500 ng/mL with a diluent (2% BSA) and serially 4-fold diluted to 0.15 ng/mL, and the chimeric antibodies were diluted with a cell supernatant stock solution. All the chimeric antibody solutions and the reference antibody solution were added into plates at 80 µL/well, and the plates were incubated in a thermostat incubator at 37 °C for 60 min and washed. An HRP-conjugated mouse anti-human antibody IgG4 (manufacturer: Southern Biotech, Cat. No. 9200-05; Fc specificity) was diluted 5000-fold with 2% BSA and added into the plates at 100 µL/well, and the plates were incubated in the thermostat incubator at 37 °C for 60 min and washed. 0.1 mg/mL chromogenic reagent TMB was carefully added at 100 µL/well to avoid bubbles, and the system was incubated at 37 °C for 10 min away from light for chromogenic reaction. Finally, a 2 M hydrochloric acid solution was added at 100 µL/well to stop the reaction while avoiding bubbles, and the plates were read on a microplate reader within 10 min (the detection wavelength was 450 nm; the reference wavelength was 620 nm).

As shown in Table 3, 60 antibody molecules exhibited binding of transient-transfection/expression supernatants to full-length CGRP with OD > 1.6 and a protein concentration greater than 10 µg/mL.

**Table 3. Binding of transient-transfection/expression supernatants to full-length CGRP**

| **Antibody molecules** | **OD value** | **Antibody molecules** | **OD value** | **Antibody molecules** | **OD value** |
|---|---|---|---|---|---|
| C27 | 3.01 | C93 | 3.07 | C263 | 3.02 |
| C28 | 3.07 | C96 | 3.09 | C267 | 2.56 |
| C43 | 1.96 | C97 | 3.03 | C269 | 2.91 |
| C46 | 3.06 | C100 | 3.05 | C271 | 2.68 |
| C48 | 3.12 | C117 | 3.06 | C276 | 3.01 |
| C50 | 3.04 | C119 | 3.08 | C278 | 2.93 |
| C51 | 3.04 | C120 | 3.09 | C280 | 2.92 |
| C54 | 3.06 | C123 | 3.04 | C281 | 2.85 |
| C66 | 2.82 | C125 | 3.08 | C284 | 2.85 |
| C69 | 3.07 | C131 | 3.02 | C292 | 2.96 |
| C71 | 3.06 | C138 | 3.06 | C305 | 2.27 |
| C73 | 3.04 | C140 | 1.84 | C316 | 3.01 |
| C74 | 3.06 | C142 | 1.69 | C319 | 3.06 |
| *C77* | 3.05 | C232 | 3.28 | C347 | 3.01 |
| C79 | 3.03 | C245 | 3.31 | C349 | 2.99 |
| C85 | 3.11 | C253 | 2.46 | C350 | 3.00 |
| C86 | 2.05 | C255 | 3.11 | C352 | 3.11 |
| C88 | 1.96 | C257 | 3.08 | C354 | 2.99 |
| C89 | 2.30 | C258 | 3.12 | C362 | 3.01 |
| C92 | 3.09 | C261 | 3.00 | C366 | 2.96 |

### 6.2 Binding assay of chimeric antibodies to fragment CGRP (1-19) by ELISA (cell-fluid supernatant)

Streptavidin was diluted to 0.5 µg/mL by using PBS (Hyclone) and added into a plate at 100 µL/well. The plate was incubated in a thermostat incubator at 37 °C for 90 min and then washed. 2% BSA was added to the plate at 200 µL/well, and the plate was incubated in the thermostat incubator at 37 °C for 90 min and washed. Biotin-1-19 polypeptide was diluted to 2.0 µg/mL with 2% BSA and added into the plate at 100 µL/well, and the plate was incubated in the thermostat incubator at 37 °C for 60 min. The reference antibody (Lily) was diluted to 2500 ng/mL with a diluent (2% BSA) and then serially 4-fold diluted to 0.15 ng/mL. For chimeric antibodies, the stock solution was diluted. All the chimeric antibody solutions and the reference antibody solution were added into plates at 80 µL/well, and the plates were incubated in a thermostat incubator at 37 °C for 60 min and washed. An HRP-conjugated mouse anti-human antibody IgG4 (manufacturer: Southern Biotech, Cat. No. 9200-05; Fc specificity) was diluted 5000-fold with 2% BSA and added into the plates at 100 µL/well, and the plates were incubated in the thermostat incubator at 37 °C for 60 min and washed. 0.1 mg/mL chromogenic reagent TMB was carefully added at 100 µL/well to avoid bubbles, and the system was incubated at 37 °C for 10 min away from light for chromogenic reaction. Finally, a 2 M hydrochloric acid solution was added at 100 µL/well to stop the reaction while avoiding bubbles, and the plates were read on a microplate reader within 10 min (the detection wavelength was 450 nm; the reference wavelength was 620 nm).

As shown in Table 4, 14 antibody molecules exhibited binding of transient-transfection/expression supernatants to fragment CGRP (1-19) with OD > 0.65 and a protein concentration greater than 10 µg/mL in which the 5 molecules in bold exhibited binding capacity to full-length CGRP (1-37).

**Table 4. Binding of transient-transfection/expression supernatants to fragment CGRP**

| **Antibody molecules** | **OD value** | **Antibody molecules** | **OD value** |
|---|---|---|---|
| **C140** | 0.76 | C238 | 2.92 |
| **C142** | 0.66 | C239 | 2.33 |
| C144 | 0.75 | **C245** | 2.51 |
| C155 | 0.65 | C246 | 0.91 |
| **C232** | 3.15 | **C253** | 2.94 |
| C234 | 2.90 | C295 | 2.40 |
| C235 | 2.84 | C296 | 0.84 |

### 6.3 Binding assay of purified chimeric antibodies to full-length (1-37) CGRP by ELISA (purified antibody)

Streptavidin was diluted to 0.5 µg/mL by using PBS (Hyclone) and added into a plate at 100 µL/well. The plate was incubated in a thermostat incubator at 37 °C for 90 min and then washed. 2% BSA was added to the plate at 200 µL/well, and the plate was incubated in the thermostat incubator at 37 °C for 90 min and washed. Biotin-huCGRPaSA was diluted to 2.0 µg/mL with 2% BSA and added into the plate at 100 µL/well, and the plate was incubated in the thermostat incubator at 37 °C for 60 min. The chimeric antibodies in Table 3 and reference antibody (47-RM-20190815) were diluted to 1000 ng/mL with a diluent (2% BSA) and serially 10-fold diluted to 1 ng/mL. All the chimeric antibody solutions and the reference antibody solution were added into plates at 100 µL/well, and the plates were incubated in a thermostat incubator at 37 °C for 60 min and washed. An HRP-conjugated mouse anti-human antibody IgG4 (manufacturer: Southern Biotech, Cat. No. 9200-05; Fc specificity) was diluted 5000-fold with 2% BSA and added into the plates at 100 µL/well, and the plates were incubated in the thermostat incubator at 37 °C for 60 min and washed. 0.1 mg/mL chromogenic reagent TMB was carefully added at 100 µL/well to avoid bubbles, and the system was incubated at 37 °C for 5 min away from light for chromogenic reaction. Finally, a 2 M hydrochloric acid solution was added at 100 µL/well to stop the reaction while avoiding bubbles, and the plates were read on a microplate reader within 10 min (the detection wavelength was 450 nm; the reference wavelength was 620 nm). EC₅₀ was fit by using a four-parameter logistic regression (4PL) model.

The antibody molecules selected in Table 3 (6.1) and Table 4 (6.2) were expressed by transient transfection and purified, and the binding activity of these antibody molecules to the full-length CGRP was detected by Elisa. As shown in Table 5, 45 antibody molecules exhibited binding activity and relative binding activity as compared to the reference antibody (47-RM-20190815).

**Table 5. Binding of antibody molecules purified from transient-transfection to full-length CGRP**

| Antibody molecules | Relative activity (%) | Antibody molecules | Relative activity (%) | Antibody molecules | Relative activity (%) |
|---|---|---|---|---|---|
| C281 | 286.0 | C347 | 91.4 | C71 | 47.3 |
| C245 | 240.2 | C316 | 100.0 | C46 | 46.2 |
| C69 | 221.4 | C319 | 100.0 | C28 | 43.1 |
| C278 | 215.3 | C276 | 84.1 | C97 | 41.9 |
| C292 | 208.7 | C54 | 72.7 | C50 | 41.7 |
| C257 | 154.4 | C27 | 70.4 | C73 | 41.6 |
| C349 | 143.8 | C77 | 69.9 | C74 | 39.5 |
| C138 | 136.3 | C92 | 68.5 | C119 | 35.8 |
| C131 | 116.1 | C125 | 62.9 | C100 | 35.6 |
| C261 | 113.8 | C48 | 60.4 | C117 | 31.7 |
| C263 | 111.1 | C79 | 59.8 | C120 | 30.6 |
| C350 | 108.5 | C85 | 56.7 | C280 | 15.1 |
| C255 | 108.1 | C96 | 56.4 | C284 | 8.8 |
| C269 | 101.4 | C51 | 56.0 | C232 | 3.4 |
| C258 | 99.8 | C123 | 47.9 | C271 | 1.8 |

### 6.4 Luciferase assay for cellular biological activity of chimeric antibodies to block binding of CGRP to CALCRL-RAMP1

SK-N-MC CRE 2E9 cells (SK-N-MC cells from ATCC, further engineered in-house for luciferase assay) were cultured in a MEM complete medium (Gibco, 11095-080) containing 10% FBS and passaged once every 3 days. On day one of the study, the cells were seeded at a density of 5 × 10⁵ cells per well in a 96-well opaque low white plate (Costar, 3917) and cultured for 16-18 h in an incubator at 37.0 °C, 5.0% CO₂. On day two, the cell supernatant in the 96-well plate was removed by pipetting, and serially diluted CGRP detection products and reference product (Top dose at 10 µg/mL, 3-fold dilution) were added at 50 µL/well. The cells and the antibodies were incubated in an incubator at 37.0 °C, 5.0% CO₂ for 30 min, and the CGRP antigen (working concentration: 4 ng/mL) was added at 50 µL/well. The cells were continuously cultured for 5-6 h at 37.0 °C, 5.0% CO₂, before luciferase substrate ONE-Lite Luciferase Assay System (Vazyme, DD1203-04) was added at 50 µL/well. The mixture was mixed well on a mixer for 5 min away from light at room temperature, and then luminescence signal values were read on an M1000 pro multifunctional microplate reader (Tecan). The IC₅₀ value of the samples of interest was calculated using the data processing software Graphoad Prism8.0, and the relative activity was calculated according to the following formula: IC₅₀ (Ref-TEVA) / IC₅₀ (sample set) × 100%.

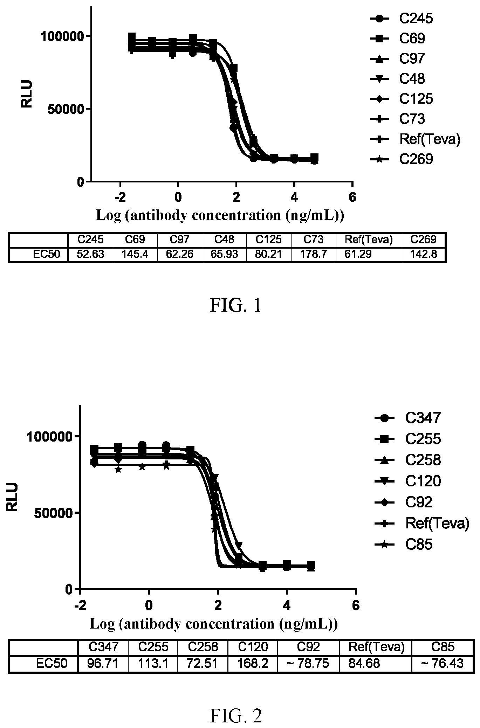

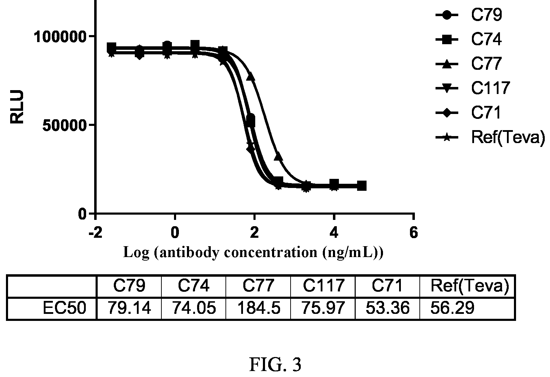

The cellular biological activity of the antibody molecules selected in Table 4 (6.2) and Table 5 (6.3) to block the binding of CGRP to CALCRL-RAMP1 (CGRP receptor dimer) was tested by cell function assay, using fremanezumab from Teva as reference. The detailed results are shown in Tables 6-1 to 6-3 and FIGs. 1 to 3, and a total of 13 chimeric antibodies exhibited relative activity greater than 70%.

**Table 6-1. Cellular biological activity of chimeric antibodies to block binding of CGRP to CALCRL-RAMP1**

| (Plate 1) | | | |
|---|---|---|---|
| | **Sample** | **IC50** | **Relative binding activity (%)** |
| | Ref(Teva) | 61.29 | 100.0 |
| 1 | C245 | 52.63 | 116.5 |
| 2 | C97 | 62.26 | 98.4 |
| 3 | C48 | 65.93 | 93.0 |
| 4 | C125 | 80.21 | 76.4 |
| 5 | C79 | 79.14 | 71.1 |
| 6 | C73 | 178.7 | 34.3 |
| 7 | C69 | 145.4 | 42.2 |

**Table 6-2. Cellular biological activity of chimeric antibodies to block binding of CGRP to CALCRL-RAMP1**

| (Plate 2) | | | |
|---|---|---|---|
| | **Sample** | **IC50** | **Relative binding activity (%)** |
| | Ref(Teva) | 84.68 | 100.0 |
| 1 | C258 | 72.51 | 116.8 |
| 2 | C85 | 76.43 | 110.8 |
| 3 | C92 | 78.75 | 107.5 |
| 4 | C347 | 96.71 | 87.6 |
| 5 | C255 | 113.1 | 74.9 |
| 6 | C120 | 168.2 | 50.3 |

**Table 6-3. Cellular biological activity of chimeric antibodies to block binding of CGRP to CALCRL-RAMP1**

| (Plate 3) | | | |
|---|---|---|---|
| | **Sample** | **IC50** | **Relative binding activity (%)** |
| | Ref(Teva) | 56.29 | 100.0 |
| 1 | C71 | 53.36 | 105.5 |
| 2 | C74 | 74.05 | 76.0 |
| 3 | C117 | 75.97 | 74.1 |
| 4 | C79 | 79.14 | 71.1 |
| 5 | C77 | 184.5 | 30.5 |

The NCBI Ig-Blast (http://www.ncbi.nlm.nih.gov/projects/igblast/) was used to search for consensus sequences in germline and rearranged Ig variable region sequence databases. The amino acid sequences of complementarity-determining regions (CDRs) were identified by sequence annotation and by Internet-based sequence analysis (http://www.imgt.org/IMGT_vquest/vquest and http://www.ncbi.nlm.nih.gov/igblast/), based on Kabat system (Wu, T. T and Kabat, E. A. 1970 J. Exp. Med., 132:211-250) and IMGT system (Lefranc M.-P. et al., 1999 Nucleic Acids Research, 27, 209-212). The light chain and heavy chain variable regions and the CDR amino acid sequences of the selected anti-CGRP murine antibody C245 are shown in Table 7.

**Table 7. Variable regions and CDR amino acid sequences of anti-CGRP murine antibody C245 (KABAT scheme)**

| | |
|---|---|
| Mouse VH (SEQ ID NO: 42) | EVQLQQSGPELVKPGASMKISCKASGYSFTGYTMNWVKQSHGNNLEWIG LINPYIGNTHYNQKFKDKATFTVDRSSSTAYMALLSLTSEDSAVYYCARE LDSGFDYWGQGTTLTVSS |
| Mouse VL (SEQ ID NO: 72) | DVLMTQTPLSLPVSLGDQASLSCRSSQNIVHSNGDTYLEWYLQKPGQSPK LLIYKVSNRFSGVPDRFSGSGSGTDFTLKISRVEAEDLGVYYCYQGSHVPW TFGGGTKLEIK |

### Example 7. Humanization of antibody variable regions

According to the evaluation results of Example 6.4, 13 anti-CGRP chimeric antibodies C258, C245, C85, C92, C71, C97, C48, C347, C125, C74, C255, C177, and C79 were analyzed for their light chain and heavy chain information, and a total of 6 light chains (1A2-LC2, 1F5-LC1, 2B8-LC2, 3C1-LC, 3E5-LC and 3H10-LC2) and 7 heavy chains (1B2-HC, 1F5-HC, 2A2-HC, 2B8-HC1, 3C1-HC, 3E5-HC and 3H10-HC) were selected to construct humanized antibodies.

For humanization of antibody variable regions, human germline IgG genes homologous to the cDNA sequence of the murine antibody variable regions were retrieved in the human immunoglobulin gene database of the NCBI (http://www.ncbi.nlm.nih.gov/igblast/). The amino acid sequences of CDRs of the variable regions and their boundaries were then determined as per the Kabat numbering system. In principle, human IGHV and IGKV with high homology to the variable regions of the murine antibody were selected as templates for humanization and antibody variable regions were humanized by CDR grafting.

Humanization was carried out based on the variable region sequences of the antibodies secreted by the hybridoma cells obtained above. In summary, the humanization process involved the following procedures:
A. The gene sequences of antibodies secreted by the hybridoma cells were compared with the gene sequences of human embryonic antibodies to find sequences with high homology;
B. HLA-DR affinity was analyzed and examined, and human embryonic framework region sequences with low affinity were selected;
C. Computer simulation technology was used to apply molecular docking to analyze the amino acid sequences of the variable regions and their surrounding framework regions, so as to examine their spatial three-dimensional binding mode. The key amino acid individuals that can interact with CGRP and maintain the spatial framework in the gene sequence of the antibodies secreted by the hybridoma cells were analyzed by calculating the electrostatic force, Van der Waals' force, hydrophilicity and hydrophobicity, and entropy value, and grafted to the selected human embryonic gene framework. The amino acid positions of the framework regions which must be retained were labeled;
D. On the basis of the three-dimensional structure of mouse antibodies, embedded residues, residues directly interacting with the CDR region, and residues having an important influence on the conformation of VL and VH were back mutated, and the amino acid residues causing chemical instability in the antibody CDR region were optimized. The optimization results are as follows, with the CDR regions underlined (KABAT scheme):
   **Anti-CGRP-IF5HC (chimeric):** 
EVQLQQSGPELVKPGASVKISCKTSGYTFIDFNMHWVKQSHGKSLEWIG
YIYPYNGDVVYNQNFTNKATLTVDSSSSTAYMELRRVTSEDSAIYYCAR
EGLGGYWGQGTTLTVSS (SEQ ID NO: 1)
      CDR1: DFNMH (SEQ ID NO: 2)
      CDR2-1: YIYPYNGDVVYNQNFTN (SEQ ID NO: 3)
      CDR2-2: YIYPYNADVVYNQDFTN (SEQ ID NO: 110)
      CDR3: EGLGGY (SEQ ID NO: 4)
   **Anti-CGRP-1F5HC-1 (humanized):** 
QVQLVQSGAEVKKPGASVKVSCKASGYTFIDFNMHWVRQAPGQRLEW
MGYIYPYNGDVVYNQNFTNRVTLTVDTSSSTAYMELSSLRSEDTAVYY
CAREGLGGYWGQGTLVTVSS (SEQ ID NO: 5)
   **Anti-CGRP-1F5HC-2 (humanized):** 
QVQLVQSGAEVKKPGASVKVSCKASGYTFTDFNMHWVRQAPGQRLEW
MGYIYPYNGDVVYNQNFTNRVTLTVDTSSSTAYMELSSLRSEDTAVYY
CAREGLGGYWGQGTLVTVSS (SEQ ID NO: 6)
   **Anti-CGRP-1F5HC-3 (humanized):** 
QVQLVQSGAEVKKPGASVKVSCKASGYTFTDFNMHWVRQAPGQGLEW
MGYIYPYNGDVVYNQNFTNRVTLTVDTSSSTAYMELRSLRSDDTAVYY
CAREGLGGYWGQGTLVTVSS (SEQ ID NO: 7)
   **Anti-CGRP-1F5HC-4 (humanized):** 
QVQLVQSGSELKKPGASVKVSCKASGYTFTDFNMHWVRQAPGQGLEW
MGYIYPYNGDVVYNQNFTNRFVLSVDSSSSTAYLQISSLKAEDTAVYYC
AREGLGGYWGQGTLVTVSS (SEQ ID NO: 8)
   **Anti-CGRP-1F5HC-5 (humanized):** 
QVQLVQSGAEVKKPGASVKVSCKASGYTFIDFNMHWVRQAPGQRLEW
MGYIYPYNADVVYNQDFTNRVTLTVDTSSSTAYMELSSLRSEDTAVYY
CAREGLGGYWGQGTLVTVSS (SEQ ID NO: 9)
   **Anti-CGRP-3E5HC (chimeric):** 
EVQLQQSGPELVKPGASVKISCKTSGYTFTDYNMHWVKQSHGKSLEWIG
YISPYNGGTAYNQKFKFKSTLTVDTSSSTAYMELRSLTSEDSAVYYCAR
EGLGGYWGQGTTLTVSS (SEQ ID NO: 10)
      CDR1: DYNMH (SEQ ID NO: 11)
      CDR2-1: YISPYNGGTAYNQKFKF (SEQ ID NO: 12)
      CDR2-2: YISPYNAGTAYNQKFKF (SEQ ID NO: 111)
      CDR3: EGLGGY (SEQ ID NO: 13)
   **Anti-CGRP-3E5HC-1 (humanized):** 
QVQLVQSGAEVKKPGASVKVSCKTSGYTFTDYNMHWVRQAPGQRLEW
MGYISPYNGGTAYNQKFKFRVTLTVDTSSSTAYMELSSLRSEDTAVYY
CAREGLGGYWGQGTLVTVSS (SEQ ID NO: 14)
   **Anti-CGRP-3E5HC-2 (humanized):** 
QVQLVQSGAEVKKPGASVKVSCKASGYTFTDYNMHWVRQAPGQRLEW
MGYISPYNGGTAYNQKFKFRVTLTVDTSSSTAYMELSSLRSEDTAVYY
CAREGLGGYWGQGTLVTVSS (SEQ ID NO: 15)
   **Anti-CGRP-3E5HC-3 (humanized):** QVQLVQSGAEVKKPGASVKVSCKASGYTFTDYNMHWVRQAPGQGLEW 
MGYISPYNGGTAYNQKFKFRVTLTVDTSSSTAYMELSRLRSDDTVVYY
CAREGLGGYWGQGTLVTVSS (SEQ ID NO: 16)
   **Anti-CGRP-3E5HC-4 (humanized):** 
QVQLVQSGAEVKKPGASVKVSCKTSGYTFTDYNMHWVRQAPGQRLEW
MGYISPYNAGTAYNQKFKFRVTLTVDTSSSTAYMELSSLRSEDTAVYYC
AREGLGGYWGQGTLVTVSS (SEQ ID NO: 17)
   **Anti-CGRP-2B8HC1 (chimeric):** 
EVQLQQSGPELVKPGASVKISCKASGYTFTDYNMHWVKQSHGKSLEWI
GNIYPYNGGTVYNQKFNNKATLTVHTSSSTAYMDLRSLTSDDSAVYYC
AREGLGGYWGQGTTLTVSS (SEQ ID NO: 18)
      CDR1: DYNMH (SEQ ID NO: 19)
      CDR2-1: NIYPYNGGTVYNQKFNN (SEQ ID NO: 20)
      CDR2-2: NIYPYNAGTVYNQKFNN (SEQ ID NO: 112)
      CDR3: EGLGGY (SEQ ID NO: 21)
   **Anti-CGRP-2B8HC1-1 (humanized):** 
QVQLVQSGAEVKKPGASVKVSCKASGYTFTDYNMHWVRQAPGQGLEW
MGNIYPYNGGTVYNQKFNNRVTLTVDTSSSTAYMELSRLRSDDTAVYY
CAREGLGGYWGQGTLVTVSS (SEQ ID NO: 22)
   **Anti-CGRP-2B8HC1-2 (humanized):** 
QVQLVQSGAEVKKPGASVKVSCKASGYTFTDYNMHWVRQAPGQGLEW
MGNIYPYNGGTVYNQKFNNRVTLTVDTSSSTAYMELRSLRSDDTAVYY
CAREGLGGYWGQGTLVTVSS (SEQ ID NO: 23)
   **Anti-CGRP-2B8HC1-3 (humanized):** 
QVQLVQSGAEVKKPGASVKVSCKASGYTFTDYNMHWVRQAPGQGLEW
MGNIYPYNAGTVYNQKFNNRVTLTVDTSSSTAYMELSRLRSDDTAVYY
 CAREGLGGYWGQGTLVTVSS (SEQ ID NO: 24)
   **Anti-CGRP-2A2HC (chimeric):** 
EVQLQQSGPELVKPGASVKISCKASGYTFTDYNMHWVKQSHGKSLEWI
GYVVYPYNGGSAYNQKFKSKATLTVDTSSSIAYMDLHSLTSEDSAVYYC
AREGLGGYWGQGTTLAVSS (SEQ ID NO: 25)
      CDR1: DYNMH (SEQ ID NO: 26)
      CDR2: YVYPYNGGSAYNQKFKS (SEQ ID NO: 27)
      CDR3: EGLGGY (SEQ ID NO: 28)
   **Anti-CGRP-2A2HC-1 (humanized):** 
QVQLVQSGAEVKKPGASVKVSCKASGYTFTDYNMHWVRQAPGQRLEW
MGYVYPYNGGSAYNQKFKSRVTLTVDTSSSTAYMELSSLRSEDTAVYY
CAREGLGGYWGQGTLVTVSS (SEQ ID NO: 29)
   **Anti-CGRP-1B2HC (chimeric):** 
EVQLQQSGPEVVKPGASVRISCQASGYTFTDYNMHWVKQSHGKSLEWI
GYIFPYNGVAVYNRKFRSKATLIIDKSSTTAYMELRSLTSEDSAVYYCAR
EGLGGYWGQGTTLTVSS (SEQ ID NO: 30)
      CDR1: DYNMH (SEQ ID NO: 31)
      CDR2: YIFPYNGV A VYNRKFRS (SEQ ID NO: 32)
      CDR3: EGLGGY (SEQ ID NO: 33)
   **Anti-CGRP-1B2HC-1 (humanized):** 
QVQLVQSGAEVKKPGASVKVSCKASGYTFTDYNMHWVRQAPGQGLEW
MGYIFPYNGVAVYNRKFRSRVTLTIDTSSSTAYMELRSLRSDDTAVYYC
AREGLGGYWGQGTLVTVSS (SEQ ID NO: 34)
   **Anti-CGRP-1B2HC-2 (humanized):** 
QVQLVQSGAEVKKPGASVKVSCKASGYTFTDYNMHWVRQAPGQRLEW
MGYIFPYNGVAVYNRKFRSRVTLTVDTSSSTAYMELSSLRSEDTAVYYC
AREGLGGYWGQGTLVTVSS (SEQ ID NO: 35)
   **Anti-CGRP-3H10HC (chimeric):** 
EVQLQQSGPELVQPGASVKISCKASGYTFTDYNMHWVKQSHGESLEWIG
YIYPYNGHTVYNQKFRNKATMTVDSSSSTAYMDLRSLTSEDSAVYYCA
KEGLGGYWGQGTTLTVSS (SEQ ID NO: 36)
      CDR1: DYNMH (SEQ ID NO: 37)
      CDR2: YIYPYNGHTVYNQKFRN (SEQ ID NO: 38)
      CDR3: EGLGGY (SEQ ID NO: 39)
   **Anti-CGRP-3H10HC-1 (humanized):** 
QVQLVQSGAEVKKPGASVKVSCKASGYTFTDYNMHWVRQAPGQGLEW
MGYIYPYNGHTVYNQKFRNRVTMTVDTSSSTAYMELRSLRSDDTAVY
YCAKEGLGGYWGQGTLVTVSS (SEQ ID NO: 40)
   **Anti-CGRP-3H10HC-2 (humanized):** 
QVQLVQSGAEVKKPGASVKVSCKASGYTFTDYNMHWVRQAPGQGLEW
MGYIYPYNGHTVYNQKFRNRVTLTVDTSSSTAYMELRSLRSDDTAVYY
CAREGLGGYWGQGTLVTVSS (SEQ ID NO: 41)
   **Anti-CGRP-3C1HC (chimeric):** 
EVQLQQSGPELVKPGASMKISCKASGYSFTGYTMNWVKQSHGNNLEWI
GLINPYIGNTHYNQKFKDKATFTVDRSSSTAYMALLSLTSEDSAVYYCA
RELDSGFDYWGQGTTLTVSS (SEQ ID NO: 42)
      CDR1: GYTMN (SEQ ID NO: 43)
      CDR2: LINPYIGNTHYNQKFKD (SEQ ID NO: 44)
      CDR3: ELDSGFDY (SEQ ID NO: 45)
   **Anti-CGRP-3C1HC-1 (humanized):** 
QVQLVQSGAEVKKPGASVKVSCKASGYSFTGYTMNWVRQAPGQRLEW
MGLINPYIGNTHYNQKFKDRVTFTVDRSSSTAYMELSSLRSEDTAVYYC
ARELDSGFDYWGQGTLVTVSS (SEQ ID NO: 46)
   **Anti-CGRP-3C1HC-2 (humanized):** 
QVQLVQSGAEVKKPGASVKVSCKASGYTFTGYTMNWVRQAPGQRLEW
MGLINPYIGNTHYNQKFKDRVTFTVDRSSSTAYMELSSLRSEDTAVYYC
ARELDSGFDYWGQGTLVTVSS (SEQ ID NO: 47)
   **Anti-CGRP-3C1HC-3 (humanized):** 
QVQLVQSGSELKKPGASVKVSCKASGYTFTGYTMNWVRQAPGQGLEW
MGLINPYIGNTHYNQKFKDRFVFSVDRSSSTAYLQISSLKAEDTAVYYC
ARELDSGFDYWGQGTLVTVSS (SEQ ID NO: 48)
   **Anti-CGRP-3C1HC-4 (humanized):** 
QVQLVQSGAEVKKPGASVKVSCKASGYTFTGYTMNWVRQAPGQGLEW
MGLINPYIGNTHYNQKFKDRVTFTVDRSSSTAYMELSRLRSDDTAVYY
CARELDSGFDYWGQGTLVTVSS (SEQ ID NO: 49)
   **Anti-CGRP-1A2LC2 (chimeric):** 
DIVLTQSPASLAVSLGQRATISCKASQNIDYDGYAYLNWYQQKPGQPPK
VLIYSVFNLESGIPARFSGSGSGTDFTLNIHPVEEEDAATYYCQQSNEFPY
TFGGGTKLEVK (SEQ ID NO: 50)
      CDR1: KASQNIDYDGYAYLN (SEQ ID NO: 51)
      CDR2: SVFNLES(SEQ ID NO: 52)
      CDR3: QQSNEFPYT (SEQ ID NO: 53)
   **Anti-CGRP-1A2LC2-1 (humanized):** 
DIVLTQSPASLAVSPGQRATITCKASQNIDYDGYAYLNWYQQKPGQPPK
VLIYSVFNLESGVPARFSGSGSGTDFTLTINPVEANDTANYYCQQSNEFP
YTFGQGTKLEIK (SEQ ID NO: 54)
   **Anti-CGRP-1A2LC2-2 (humanized):** 
DIVLTQSPASLAVSPGQRATITCKASQNIDYDGYAYLNWYQQKPGQPPK
VLIYSVFNLESGVPARFSGSGSGTDFTLTINPVEADDTANYYCQQSNEFP
YTFGQGTKLEIK (SEQ ID NO: 55)
   **Anti-CGRP-1A2LC2-3 (humanized):** 
EIVLTQSPATLSLSPGERATLSCKASQNIDYDGYAYLNWYQQKPGQAPR
VLIYSVFNLESGIPARFSGSGSGTDFTLTISSLEPEDFAVYYCQQSNEFPYT
FGQGTKLEIK (SEQ ID NO: 56)
   **Anti-CGRP-1A2LC2-4 (humanized):** 
DIQLTQSPSFLSASVGDRVTITCKASQNIDYDGYAYLNWYQQKPGKAPK
VLIYSVFNLESGVPSRFSGSGSGTEFTLTISSLQPEDFATYYCQQSNEFPY
TFGQGTKLEIK (SEQ ID NO: 57)
   **Anti-CGRP-1F5LC1 (chimeric):** 
DIVLTQSPASLAVSLGQRATISCKASQNIDFDGYAYMNWYQHKPGQPPR
VLIYSVFNLESGIPARFSGSGSGTDFTLNIHPVEEEDAATYYCQQSHEVPY
TFGGGTNLEIK (SEQ ID NO: 58)
      CDR1: KASQNIDFDGYAYMN (SEQ ID NO: 59)
      CDR2: SVFNLES (SEQ ID NO: 60)
      CDR3: QQSHEVPYT (SEQ ID NO: 61)
   **Anti-CGRP-1F5LC1-1 (humanized):** 
EIVLTQSPATLSLSPGERATLSCKASQNIDFDGYAYMNWYQQKPGQAPR
VLIYSVFNLESGIPARFSGSGSGTDFTLTISSLEPEDFAVYYCQQSHEVPY
TFGQGTKLEIK (SEQ ID NO: 62)
   **Anti-CGRP-1F5LC1-2 (humanized):** 
EIVLTQSPATLSLSPGERATLSCKASQNIDFDGYAYMNWYQHKPGQAPR
VLIYSVFNLESGIPARFSGSGSGTDFTLTISSLEPEDFAVYYCQQSHEVPY
TFGQGTKLEIK (SEQ ID NO: 63)
   **Anti-CGRP-1F5LC1-3 (humanized):** 
DIVLTQSPASLAVSPGQRATITCKASQNIDFDGYAYMNWYQQKPGQPPK
VLIYSVFNLESGVPARFSGSGSGTDFTLTINPVEADDTANYYCQQSHEVP
YTFGQGTKLEIK (SEQ ID NO: 64)
   **Anti-CGRP-1F5LC1-4 (humanized):** 
DIVLTQSPDSLAVSLGERATINCKASQNIDFDGYAYMNWYQHKPGQPPK
VLIYSVFNLESGVPDRFSGSGSGTDFTLTISSLQAEDVAVYYCQQSHEVP
YTFGQGTKLEIK (SEQ ID NO: 65)
   **Anti-CGRP-2B8LC2 (chimeric):** 
DVVLTQSPTSLAVSLGQRATISCQASQNIDGNTYMNWYQQKPGQPPKV
LIYSIFNLESGIPARFSGSGSGTHFTLTIHPVEEEDAATYYCQQSNDYPYT
FGGGTKLEIK (SEQ ID NO: 66)
      CDR1: QASQNIDGNTYMN (SEQ ID NO: 67)
      CDR2: SIFNLES (SEQ ID NO: 68)
      CDR3: QQSNDYPYT (SEQ ID NO: 69)
   **Anti-CGRP-2B8LC2-1 (humanized):** 
DIVLTQSPDSLAVSLGERATINCQASQNIDGNTYMNWYQQKPGQPPKVL
IYSIFNLESGVPDRFSGSGSGTDFTLTISSLQAEDVAVYYCQQSNDYPYTF
GQGTKLEIK (SEQ ID NO: 70)
   **Anti-CGRP-2B8LC2-2 (humanized):** DVQLTQSPSFLSASVGDRVTITCQASQNIDGNTYMNWYQQKPGKAPKV 
LIYSIFNLESGVPSRFSGSGSGTEFTLTISSLQPEDFATYYCQQSNDYPYTF
GQGTKLEIK (SEQ ID NO: 71)
   **Anti-CGRP-3C1LC (chimeric):** 
DVLMTQTPLSLPVSLGDQASLSCRSSQNIVHSNGDTYLEWYLQKPGQSP
KLLIYKVSNRFSGVPDRFSGSGSGTDFTLKISRVEAEDLGVYYCYQGSHV
PWTFGGGTKLEIK (SEQ ID NO: 72)
      CDR1-1: RSSQNIVHSNGDTYLE (SEQ ID NO: 73)
      CDR1-2: RSSQNIVHSNADTYLE (SEQ ID NO: 113)
      CDR2: KVSNRFS (SEQ ID NO: 74)
      CDR3: YQGSHVPWT (SEQ ID NO: 75)
   **Anti-CGRP-3C1LC-1 (humanized):** 
DVVMTQSPLSLPVTLGQPASISCRSSQNIVHSNGDTYLEWYQQRPGQSP
RLLIYKVSNRFSGVPDRFSGSGSGTDFTLKISRVEAEDVGVYYCYQGSHV
PWTFGQGTKLEIK (SEQ ID NO: 76)
   **Anti-CGRP-3C1LC-2 (humanized):** 
DVVMTQTPLSLSVTPGQPASISCRSSQNIVHSNGDTYLEWYLQKPGQSP
QLLIYKVSNRFSGVPDRFSGSGSGTDFTLKISRVEAEDVGVYYCYQGSH
VPWTFGQGTKLEIK (SEQ ID NO: 77)
   **Anti-CGRP-3C1LC-3 (humanized):** 
DVVMTQSPDSLAVSLGERATINCRSSQNIVHSNGDTYLEWYQQKPGQPP
KLLIYKVSNRFSGVPDRFSGSGSGTDFTLTISSLQAEDVAVYYCYQGSHV
PWTFGQGTKLEIK (SEQ ID NO: 78)
   **Anti-CGRP-3C1LC-4 (humanized):** 
DVVMTQSPDSLAVSLGERATINCRSSQNIVHSNADTYLEWYQQKPGQPP
KLLIYKVSNRFSGVPDRFSGSGSGTDFTLTISSLQAEDVAVYYCYQGSHV
 PWTFGQGTKLEIK (SEQ ID NO: 79)
   **Anti-CGRP-3ESLC (chimeric):** 
DIVLTQSPASLAVSLGQRATISCKASQNIDYDGYTYLNWYQQKPGQPPK
VLIYSIFNLESGIPARFSGSGSGTDFTLNIHPVEEEDAATYYCQQSNEVPY
TFGGGTKLEIK (SEQ ID NO: 80)
      CDR1: KASQNIDYDGYTYLN (SEQ ID NO: 81)
      CDR2: SIFNLES (SEQ ID NO: 82)
      CDR3: QQSNEVPYT (SEQ ID NO: 83)
   **Anti-CGRP-3E5LC-1 (humanized):** 
DIVLTQSPDSLAVSLGERATINCKASQNIDYDGYTYLNWYQQKPGQPPK
VLIYSIFNLESGVPDRFSGSGSGTDFTLTISSLQAEDVAVYYCQQSNEVPY
TFGQGTKLEIK (SEQ ID NO: 84)
   **Anti-CGRP-3H10LC2 (chimeric):** 
DIVLTQSPDSLAVSLGQRATISCKASQNIDYGGYSYMNWYQQKPGQPPK
VLIYSVFNLESGIPARFSGSGSGTDFTLTIHPVEEEDAATYYCQQSNEAPY
TFGGGTKLEIR (SEQ ID NO: 85)
      CDR1: KASQNIDYGGYSYMN (SEQ ID NO: 86)
      CDR2: SVFNLES (SEQ ID NO: 87)
      CDR3: QQSNEAPYT (SEQ ID NO: 88)
   **Anti-CGRP-3H10LC2-1 (humanized):** 
DIVLTQSPDSLAVSLGERATINCKASQNIDYGGYSYMNWYQQKPGQPPK
VLIYSVFNLESGVPDRFSGSGSGTDFTLTISSLQAEDVAVYYCQQSNEAP
YTFGQGTKLEIK (SEQ ID NO: 89)
   **Anti-CGRP-3H10LC2-2 (humanized):** 
DIQLTQSPSSLSASVGDRVTITCKASQNIDYGGYSYMNWYQQKPGKAPK
VLIYSVFNLESGVPSRFSGSGSGTDFTLTISSLQPEDFATYYCQQSNEAPY
TFGQGTKLEIK (SEQ ID NO: 90)
   **Hu224 LC:** 
DVVMTQSPDSLAVSLGERATINCRSSQNIVHSNGDTYLEWYQQKPGQPP
KLLIYKVSNRFSGVPDRFSGSGSGTDFTLTISSLQAEDVAVYYCYQGSHV
PWTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYA
CEVTHQGLSSPVTKSFNRGEC (SEQ ID NO: 91)
   **Hu224HC:** 
QVQLVQSGAEVKKPGASVKVSCKASGYSFTGYTMNWVRQAPGQRLEW
MGLINPYIGNTHYNQKFKDRVTFTVDRSSSTAYMELSSLRSEDTAVYYC
ARELDSGFDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLG
K (SEQ ID NO: 92)
   **Hu228LC:** 
DVVMTQSPDSLAVSLGERATINCRSSQNIVHSNGDTYLEWYQQKPGQPP
KLLIYKVSNRFSGVPDRFSGSGSGTDFTLTISSLQAEDVAVYYCYQGSHV
PWTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYA
CEVTHQGLSSPVTKSFNRGEC (SEQ ID NO: 93)
   **Hu228HC:** 
QVQLVQSGAEVKKPGASVKVSCKASGYTFTGYTMNWVRQAPGQRLEW
MGLINPYIGNTHYNQKFKDRVTFTVDRSSSTAYMELSSLRSEDTAVYYC
ARELDSGFDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLG
K (SEQ ID NO: 94)
   **Hu231LC:** 
DVVMTQTPLSLSVTPGQPASISCRSSQNIVHSNGDTYLEWYLQKPGQSPQ
LLIYKVSNRFSGVPDRFSGSGSGTDFTLKISRVEAEDVGVYYCYQGSHVP
WTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYA
CEVTHQGLSSPVTKSFNRGEC (SEQ ID NO: 95)
   **Hu231HC:**

QVQLVQSGSELKKPGASVKVSCKASGYTFTGYTMNWVRQAPGQGLEW
MGLINPYIGNTHYNQKFKDRFVFSVDRSSSTAYLQISSLKAEDTAVYYCA
RELDSGFDYWGQGTLVTVSSASTKGPSVFPLAPCSRSTSESTAALGCLVK
DYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNST
YRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVL
DSDGSFFLYSRLTVDKSRWQEGNVFSCSVMHEALHNHYTQKSLSLSLGK

(SEQ ID NO: 96)

The humanized anti-CTLA-4 antibody variable regions designed above were diversely combined, and the four humanized light chains (3C1LC) and four humanized heavy chains (3C1HC) from chimeric antibody C245 were combined to give 237 humanized anti-CGRP antibodies. The numbers and amino acid sequences of the antibodies are detailed in Table 8. The humanized variable regions above were linked to the constant region sequences to prepare full-length antibodies.
Heavy chain constant region: 
ASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKY
GPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGN
VFSCSVMHEALHNHYTQKSLSLSLGK (SEQ ID NO: 108)
Light chain constant region:

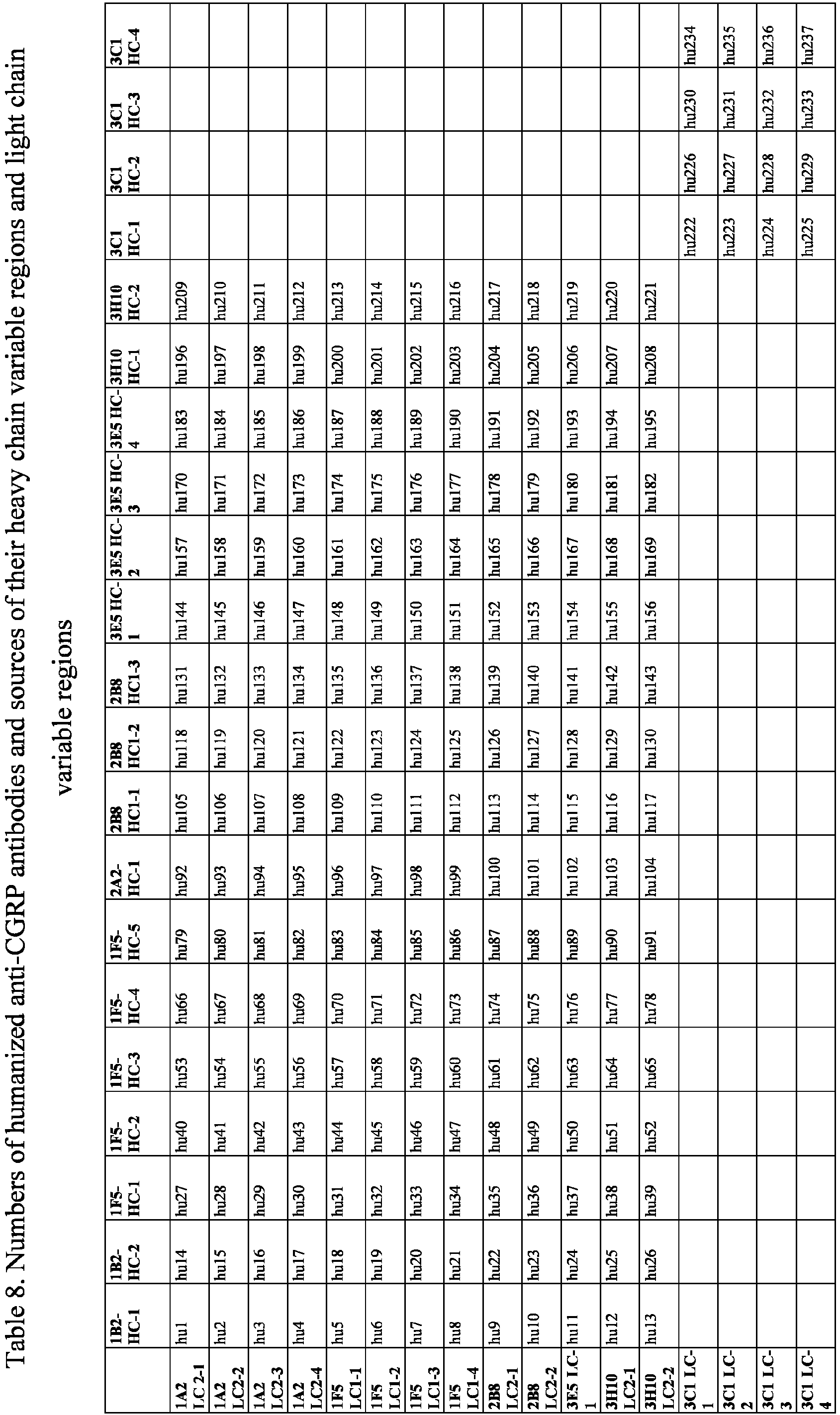



 (SEQ ID NO: 109)

### Example 8. Screening of humanized anti-CGRP antibodies

The optimal humanized antibodies were screened according to the ability of the humanized antibodies in Table 8 (Example 7) to bind to full-length (1-37) CGRP and the cellular biological activity thereof to block the binding of CGRP to CALCRL-RAMP1. The procedures and results are shown below.

### 8.1 Binding assay of humanized antibodies to full-length CGRP (1-37) by ELISA (cell culture supernatant)

Biotin CGRPaSA was diluted with PBS (Hyclone) to 1.0 µg/mL, and the dilution was added to a microplate at 100 µL/well. The plate was let stand in an incubator at 37 °C and coated for 90 min and then washed, and 2% BSA was added to the plate at 200 µL/well. The plate was incubated in an incubator at 37 °C for 90 min and then washed. The humanized antibodies in Table 8 and the reference antibody (chimeric antibody C245) were diluted to 1000 ng/mL with a diluent (2% BSA) with a dilution factor of not more than 10. Then, the chimeric antibodies and the reference antibody were serially 2.5-fold diluted on a sample dilution plate. All the chimeric antibody solutions and the reference antibody solution were added into plates at 100 µL/well, and the plates were incubated in a thermostat incubator at 37 °C for 60 min and washed. An HRP-conjugated goat anti-human antibody IgG (manufacturer: Sigma, Cat. No. A0170; Fc specificity) was diluted 5000-fold with 2% BSA and added into the plates at 100 µL/well, and the plates were incubated in the thermostat incubator at 37 °C for 60 min and washed. Chromogenic reagent TMB was carefully added at 100 µL/well to avoid bubbles, and the system was incubated at 37 °C for 15 min away from light for chromogenic reaction. Finally, a 2 M hydrochloric acid solution was added at 100 µL/well to stop the reaction while avoiding bubbles, and the plates were read on a microplate reader within 10 min (wavelength: 450/620 nm).

The binding activity of the transient-transfection/expression supernatants to full-length CGRP (1-37) is shown in Table 9 using chimeric antibody C245 as the reference.

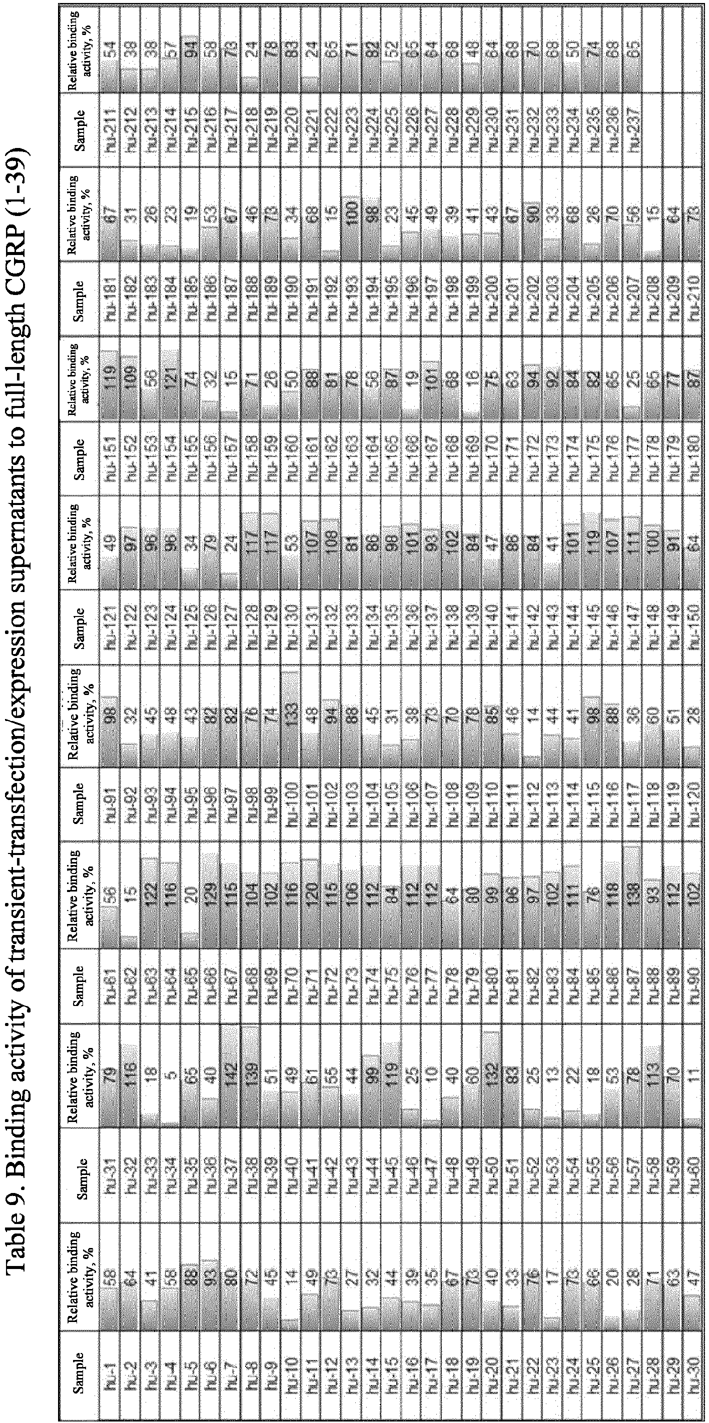

### 8.2 Luciferase assay for cellular biological activity of humanized antibodies to block binding of CGRP to CALCRL-RAMP1

SK-N-MC CRE 2E9 cells were cultured in a MEM complete medium (Gibco, 11095-080) containing 10% FBS and passaged once every 3 days. On day one of the study, the cells were seeded at a density of 5 × 10⁵ cells per well in a 96-well opaque low white plate (Costar, 3917) and cultured for 16-18 h in an incubator at 37.0 °C, 5.0% CO₂. On day two, the cell supernatant in the 96-well plate was removed by pipetting, and serially diluted CGRP detection products and reference product (Top dose at 10 µg/mL, 3-fold dilution) were added at 50 µL/well. The cells and the antibodies were incubated in an incubator at 37.0 °C, 5.0% CO₂ for 30 min, and the CGRP antigen (working concentration: 4 ng/mL) was added at 50 µL/well. The cells were continuously cultured for 5-6 h at 37.0 °C, 5.0% CO₂, before luciferase substrate ONE-Lite Luciferase Assay System (Vazyme, DD1203-04) was added at 50 µL/well. The mixture was mixed well on a mixer for 5 min away from light at room temperature, and then luminescence signal values were read on an M1000 pro multifunctional microplate reader (Tecan). The IC₅₀ value of the samples of interest was calculated using the data processing software Graphoad Prism8.0.

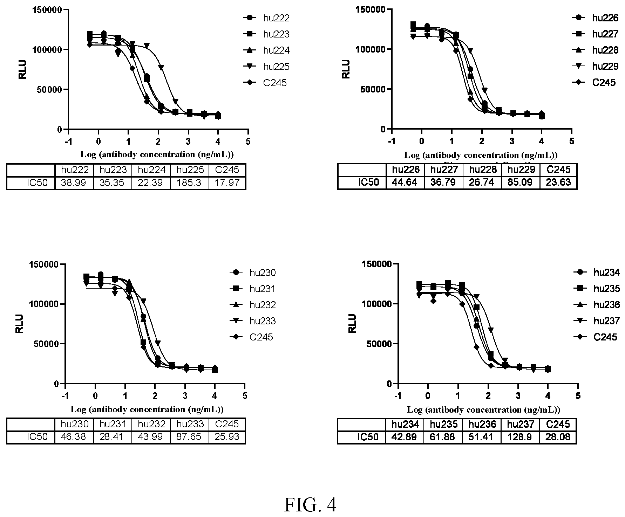

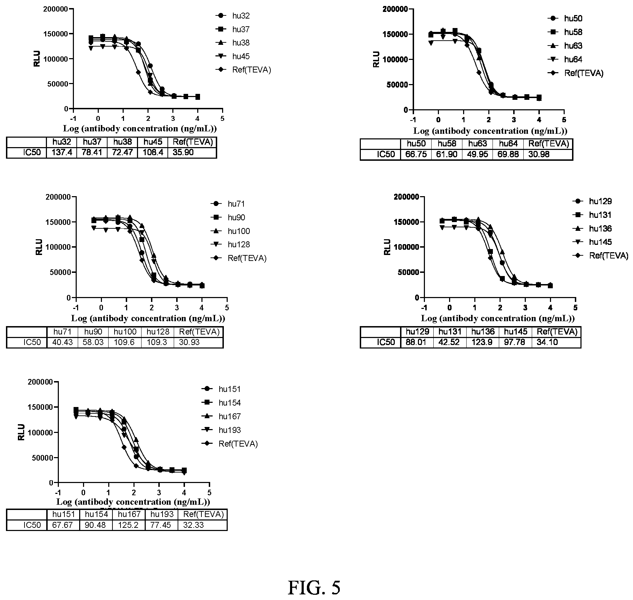

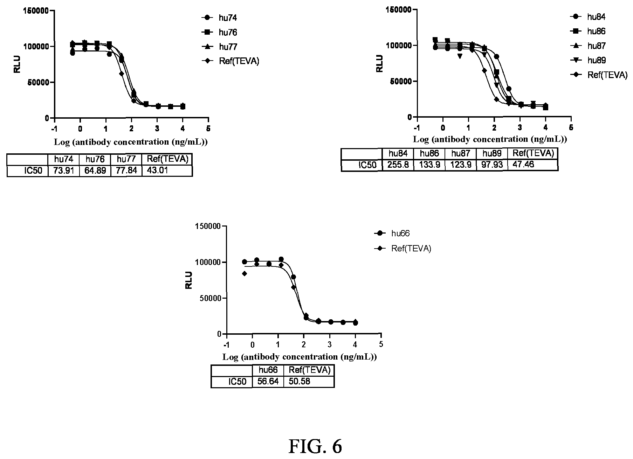

On the premise of ensuring the diversity of light and heavy chains, 44 antibody molecules were selected from Table 9 (8.1), and the cellular biological activity of the antibody molecules to block the binding of CGRP to CALCRL-RAMP1 was tested by cell functionality assay, using fremanezumab from Teva and chimeric antibody C245 as the reference antibodies, so as to find out humanized antibodies with better blocking activity. The detailed results are shown in Table 10 and FIGs. 4 to 6. In summary, humanized antibody molecules hu66, hu71, hu131, hu224, hu228, and hu231 exhibited better inhibitory activity. The variable regions and CDR amino acid sequence numbers of humanized antibody molecules hu66, hu71, hu131, hu224, hu228, and hu231 are shown in Table 11.

**Table 10. Cellular biological activity of chimeric antibodies to block binding of CGRP to CALCRL-RAMP1**

| **Number** | **Chimeric antibody** | **IC50(ng/ml)** | **Reference & relative activity (%)** | |
|---|---|---|---|---|
| 1 | hu222 | 38.99 | C245 IC50=17.97 | 46 |
| 2 | hu223 | 35.35 | | 51 |
| 3 | **hu224** | **22.39** | | **80** |
| 4 | hu225 | 185.3 | | 10 |
| 5 | hu226 | 44.64 | C245 IC50=23.63 | 53 |
| 6 | hu227 | 36.79 | | 64 |
| 7 | **hu228** | **26.74** | | **88** |
| 8 | hu229 | 85.09 | | 28 |
| 9 | hu230 | 46.38 | C245 IC50=25.93 | 56 |
| 10 | **hu231** | **28.41** | | **91** |
| 11 | hu232 | 43.99 | | 59 |
| 12 | hu233 | 87.65 | | 30 |
| 13 | hu234 | 42.89 | C245 IC50=28.08 | 65 |
| 14 | hu235 | 61.88 | | 45 |
| 15 | hu236 | 51.41 | | 55 |
| 16 | hu237 | 128.9 | | 22 |
| 17 | hu32 | 137.40 | Ref(TEVA) IC50=35.90 | 26 |
| 18 | hu37 | 78.41 | | 46 |
| 19 | hu38 | 72.47 | | 50 |
| 20 | hu45 | 108.40 | | 33 |
| 21 | hu50 | 66.75 | Ref(TEVA) IC50=30.98 | 46 |
| 22 | hu58 | 61.90 | | 50 |
| 23 | hu63 | 49.95 | | 62 |
| 24 | hu64 | 69.88 | | 44 |
| 25 | **hu71** | **40.43** | Ref(TEVA) IC50=30.93 | 77 |
| 26 | hu90 | 58.03 | | 53 |
| 27 | hu100 | 109.60 | | 28 |
| 28 | hu128 | 109.30 | | 28 |
| 29 | hu129 | 88.01 | Ref(TEVA) IC50=34.1 | 39 |
| 30 | **hu131** | **42.52** | | **80** |
| 31 | hu136 | 123.90 | | 28 |
| 32 | hu145 | 97.78 | | 35 |
| 33 | hu151 | 67.67 | Ref(TEVA) IC50=32.33 | 48 |
| 34 | hu154 | 90.48 | | 36 |
| 35 | hu167 | 125.20 | | 26 |
| 36 | hu193 | 77.45 | | 42 |
| 37 | hu84 | 255.80 | Ref(TEVA) IC50=47.46 | 19 |
| 38 | hu86 | 133.90 | | 35 |
| 39 | hu87 | 123.90 | | 38 |
| 40 | hu89 | 97.93 | | 48 |
| 41 | hu74 | 73.91 | Ref(TEVA) IC50=43.01 | 58 |
| 42 | hu76 | 64.89 | | 66 |
| 43 | hu77 | 77.84 | | 55 |
| 44 | **hu66** | **56.64** | Ref(TEVA) IC50=50.58 | **89** |

**Table 11. Variable regions and CDR amino acid sequences of humanized anti-human CGRP antibody (KABAT scheme)**

| Humanized antibody | HCDR1 | HCDR2 | HCDR3 | LCDR1 | LCDR2 | LCDR3 | VH | VL |
|---|---|---|---|---|---|---|---|---|
| hu224 | SEQ ID NO: 43 | SEQ ID NO: 44 | SEQ ID NO: 45 | SEQ ID NO: 73 | SEQ ID NO: 74 | SEQ ID NO: 75 | SEQ ID NO: 46 | SEQ ID NO: 78 |
| hu228 | SEQ ID NO: 43 | SEQ ID NO: 44 | SEQ ID NO: 45 | SEQ ID NO: 73 | SEQ ID NO: 74 | SEQ ID NO: 75 | SEQ ID NO: 47 | SEQ ID NO: 78 |
| hu231 | SEQ ID NO: 43 | SEQ ID NO: 44 | SEQ ID NO: 45 | SEQ ID NO: 73 | SEQ ID NO: 74 | SEQ ID NO: 75 | SEQ ID NO: 48 | SEQ ID NO: 77 |
| hu71 | SEQ ID NO: 2 | SEQ ID NO: 3 | SEQ ID NO: 4 | SEQ ID NO: 59 | SEQ ID NO: 60 | SEQ ID NO: 61 | SEQ ID NO: 8 | SEQ ID NO: 63 |
| hu131 | SEQ ID NO: 19 | SEQ ID NO: 20 | SEQ ID NO: 21 | SEQ ID NO: 51 | SEQ ID NO: 52 | SEQ ID NO: 53 | SEQ ID NO: 24 | SEQ ID NO: 54 |
| hu66 | SEQ ID NO: 2 | SEQ ID NO: 3 | SEQ ID NO: 4 | SEQ ID NO: 51 | SEQ ID NO: 52 | SEQ ID NO: 53 | SEQ ID NO: 8 | SEQ ID NO: 54 |

### Example 9. Inhibitory effect of chimeric anti-CGRP antibodies on capsaicin-induced vasodilation in rats

13 chimeric antibody molecules with *in vitro* efficacy of greater than 70% were selected in Example 6.4, and by analyzing the light and heavy chains contained in these antibodies, 7 chimeric antibody molecules C48, C85, C97, C125, C245, C258, and C347 were selected for *in vivo* efficacy assay. The *in vivo* efficacy was evaluated using a rat capsaicin-induced vasodilation model. It was found that C245 had a superior inhibitory effect on capsaicin-induced skin vasodilation to that of the reference Teva, and that the effect was sustained for at least 5 days in the SD rats.

72 male SD rats (SPF (Beijing) Biotechnology Co., Ltd.) were selected. The rat local blood flow increasing model was constructed by percutaneous capsaicin administration, and the rats were randomized into 9 groups by body weight, with 8 animals in each group, i.e., a modeling control group, a Ref (Teva, fremanezumab) group, a C48 group, a C85 group, a C97 group, a C125 group, a C245 group, a C258 group, and a C347 group. The treatments were given subcutaneously at 10 mg/10 mL/kg, while the modeling control group received normal saline at the same volume.

Each group received 1 dose. On days 2 and 4 post-dose, the mice were anesthetized by pentobarbital sodium and marked with O-shaped circles with a diameter of about 8 mm bilaterally along the abdominal midline, while avoiding areas with visible blood vessels. 4 µL of capsaicin (1 mg) solution was uniformly applied in the circle at one side, while the other side was not treated. The skin blood flow data within the O-shaped circles were collected before capsaicin treatment and at 5 min, 10 min, and 20 min after capsaicin treatment, and the comprehensive blood flow change rate and the blood flow increase inhibition rate were calculated according to the blood flow data at each time point.

On day 2 post-dose, the animals in the modeling control group after capsaicin treatment exhibited a significant increase in the blood flow and a comprehensive blood flow change rate of 46.0 ± 5.4% at 20 min after capsaicin treatment. The animals in the C258 group and the C347 group after capsaicin treatment exhibited a certain increase trend in the comprehensive blood flow change rate relative to that of the modeling control group. The animals in the Ref (Teva, fremanezumab) group, the C48 group, the C85 group, the C97 group, and the C125 group exhibited mild decreases in the comprehensive blood flow change rate relative to that of the modeling control group over the 20 min observation period after capsaicin treatment. The animals in the C245 group exhibited a greater decrease in the comprehensive blood flow change rate relative to that of the modeling control group, a comprehensive blood flow change rate of 21.97 ± 3.46% and a blood flow increase inhibition rate of 45.72 ± 8.54% at 5 min after capsaicin treatment, and a further decrease in blood flow over time. At 20 min after capsaicin treatment, the animals in the C245 group exhibited a comprehensive blood flow change rate of 17.81 ± 5.80% and a blood flow increase inhibition rate of 61.25 ± 12.63%.

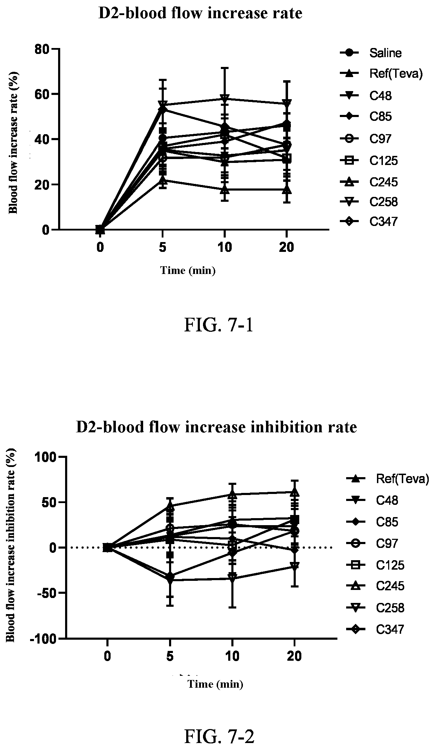

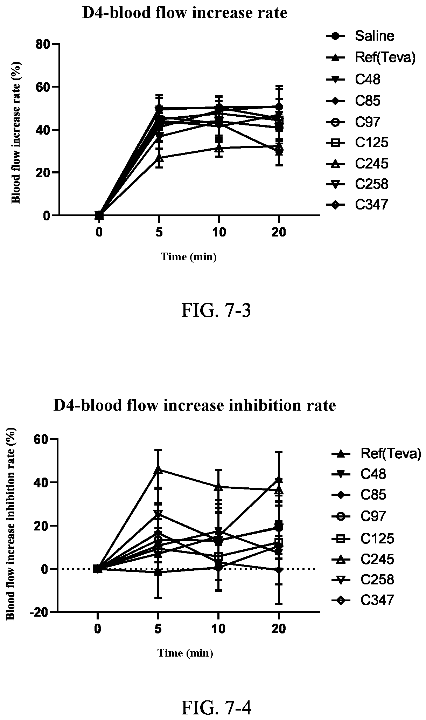

On day 4 post-dose, the animals in the modeling control group after capsaicin treatment exhibited a comprehensive blood flow change rate of about 50%. The animals in the C347 group after capsaicin treatment exhibited a certain increase trend in the blood flow relative to that of the modeling control group. The animals in the C48 group, the C85 group, the C97 group, and the C125 group exhibited mild decrease trends in the blood flow relative to that of the modeling control group, but overall decreases to different extents in the blood flow inhibition rate relative to that on day 2 post-dose. The animals in the C258 group also exhibited a mild decrease in the comprehensive blood flow change rate relative to that of the modeling control group, and exhibited a certain increase in the blood flow increase inhibition rate relative to that on day 2 post-dose. The animals in the C245 group exhibited a greater decrease in the comprehensive blood flow change rate relative to that of the modeling control group, and a comprehensive blood flow change rate of 26.75 ± 4.41% and a blood flow increase inhibition rate that reached 45.88 ± 8.92% at 5 min after capsaicin treatment. The animals in the C245 group exhibited a certain increase trend in the blood flow over time and a blood flow increase inhibition rate of 36.40 ± 5.10% at 20 min after capsaicin treatment. The detailed assay results are shown in FIGs. 7-1 to 7-4. The comprehensive blood flow change rates and the blood flow increase inhibition rates of C245 and the reference antibody Ref (Teva, fremanezumab) are shown in Table 12.

**Table 12. Comprehensive blood flow change rates and blood flow increase inhibition rates of C245 and reference antibody**

| Time | Teva | C245 | Teva | C245 |
|---|---|---|---|---|
| D2 | D2-comprehensive blood flow change rate/% | | D2-blood flow increase inhibition rate/% | |
| 5 min | 35±8 | 21.97±3.46 | 13.5±19.7 | 45.72±8.54 |
| 20 min | 31.1±9.3 | 17.81±5.80 | 32.4±20.2 | 61.2±12.63 |
| D4 | D4-comprehensive blood flow change rate/% | | D4-blood flow increase inhibition rate/% | |
| 5 min | 46±4.9 | 26.75±4.41 | 7±10 | 45.9±8.9 |
| 20 min | 29.6±6.3 | 45.88±8.92 | 41.6±12.4 | 36.40±5.10 |

### Example 10. Inhibitory effect of humanized anti-CGRP antibodies on capsaicin-induced vasodilation in rats

6 humanized antibody molecules with good *in vitro* efficacy were selected in Example 8.2 and further evaluated for their *in vivo* efficacy using a rat capsaicin-induced vasodilation model. It was found that hu224, hu228, and hu231 exhibited inhibitory effects on capsaicin-induced skin vasodilation and that the effects were sustained for at least 4 days in SD rats.

64 male SD rats (Sprague Dawley; SPF (Beijing) Biotechnology Co., Ltd.) were selected. The rat local blood flow increasing model was constructed by percutaneous capsaicin administration, and the rats were randomized into 8 groups by body weight, with 8 animals in each group, i.e., a modeling control group, a Ref (Teva, fremanezumab) group, a hu66 group, a hu71 group, a hu131 group, a hu231 group, a hu224 group, and a hu228 group. The treatments were given subcutaneously at 10 mg/10 mL/kg, while the modeling control group received normal saline at the same volume. Each group received 1 dose. On days 2 and 4 post-dose, the mice were anesthetized by pentobarbital sodium and marked with O-shaped circles with a diameter of about 8 mm bilaterally along the abdominal midline, while avoiding areas with visible blood vessels. 4 µL. of capsaicin (1 mg) solution was uniformly applied in the circle at one side, while the other side was not treated. The skin blood flow data within the O-shaped circles were collected before capsaicin treatment and at 5 min, 10 min, and 20 min after capsaicin treatment, and the comprehensive blood flow change rate and the blood flow increase inhibition rate were calculated according to the blood flow data at each time point.

On day 2 post-dose, the animals in the modeling control group exhibited a comprehensive blood flow change rate of 47.55 ± 8.14% at 5 min after capsaicin treatment, a certain increase trend in the blood flow over time, and a comprehensive blood flow change rate of 57.00 ± 4.71% at 20 min after capsaicin treatment. The animals in the hu71 group and the hu131 group exhibited a certain increase trend in the comprehensive blood flow change rate relative to that of the model control group after capsaicin treatment. The animals in the hu66 group exhibited a mild decrease in the comprehensive blood flow change rate relative to that of the model control group over the 20 min observation period. The animals in the Ref (Teva, fremanezumab) group, the hu231 group, the hu224 group, and the hu228 group exhibited greater decreases in the comprehensive blood flow change rate relative to that of the model control group, comprehensive blood flow change rates of 30.86 ± 8.35%, 25.91 ± 5.97%, 23.1 ± 6.38% and 28.94 ± 7.58% and blood flow increase inhibition rates of 35.09 ± 17.56%, 45.52 ± 12.55%, 51.43 ± 13.43% and 39.14 ± 15.94% at 5 min after capsaicin treatment, respectively, and further decreases in the blood flow over time. At 20 min after capsaicin treatment, the animals in the Ref (Teva, Fremanezumab) group, the hu231 group, the hu224 group, and the hu228 group exhibited the comprehensive blood flow change rates of 24.61 ± 9.68%, 9.94 ± 8.17%, 9.37 ± 8.43%, and 14.84 ± 10.1%, respectively, where the hu231 group, the hu224 group, and the hu228 group exhibited the comprehensive blood flow change rates significantly lower than that of the modeling control group (P ≤ 0.05).

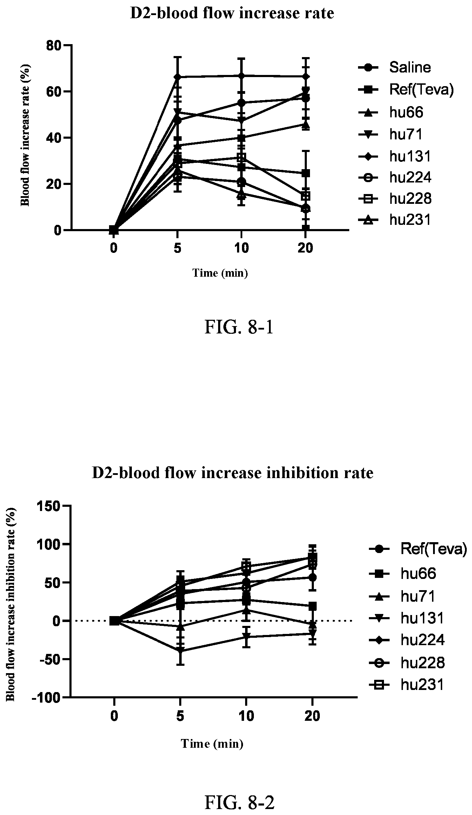

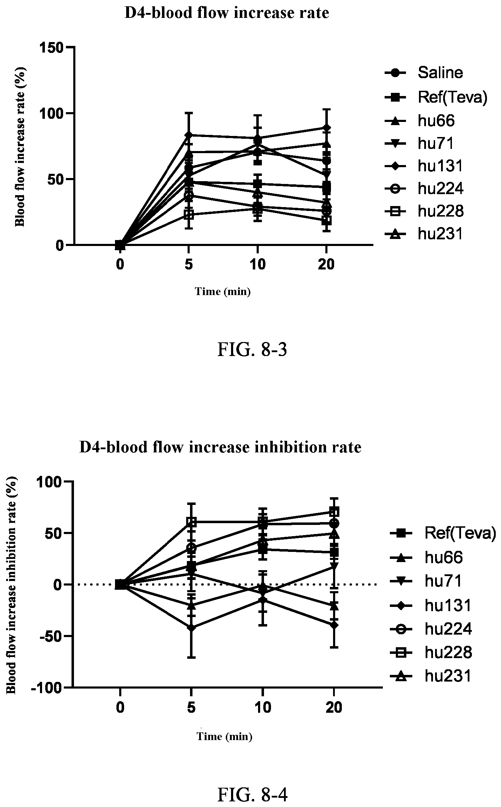

On day 4 post-dose, the animals in the modeling control group after capsaicin treatment exhibited a comprehensive blood flow change rate that was always greater than 50%. The animals in the hu66 group, the hu71 group, and the hu131 group after capsaicin treatment exhibited certain increase trends in the blood flow relative to that of the modeling control group. Similar to those on D2, the animals in the Ref (Teva, fremanezumab) group, the hu231 group, the hu224 group, and the hu228 group exhibited greater decreases in the comprehensive blood flow change rate relative to that of the modeling control group, and the decrease in the comprehensive blood flow change rate exhibited a gradual increase over time. The detailed assay results are shown in FIGs. 8-1 to 8-4. The comprehensive blood flow change rates and the blood flow increase inhibition rates of hu231, hu224 and hu228 and the reference antibody Ref (Teva, fremanezumab) are shown in Table 13.

**Table 13. Comprehensive blood flow change rates and blood flow increase inhibition rates of hu231, hu228 and hu224 and reference antibody**

| Time | Teva | hu231 | hu228 | hu224 | Teva | hu231 | hu228 | hu224 |
|---|---|---|---|---|---|---|---|---|
| D2 | D2-comprehensive blood flow change rate/% | | | | D2-blood flow increase inhibition rate/% | | | |
| 5 min | 30.86±8.35 | 25.91±5.97 | 28.94±7.58 | 23.1±6.38 | 35.09±17.56 | 45.52±12.55 | 39.14±15.94 | 51.43±13.43 |
| 20 min | 24.61±9.68 | 9.94±8.17 | 14.84±10.1 | 9.37±8.43 | 56.82±16.98 | 82.56±14.34 | 73.96±17.72 | 83.57±14.79 |
| D4 | D4-comprehensive blood flow change rate/% | | | | D4-blood flow increase inhibition rate/% | | | |
| 5 min | 47.78±7.31 | 47.94±7.32 | 23±10.43 | 37.74±9.48 | 18.5±12.47 | 18.22±12.48 | 60.76+17.79 | 35.62+16.17 |
| 20 min | 43.95±4.01 | 32.2±6.57 | 18.65±8.11 | 25.94±8.76 | 31.22±6.28 | 49.6±10.28 | 70.82±12.69 | 59.41±13.7 |

### Example 11. Inhibitory effect of humanized anti-CGRP antibodies on closed cranial window electrostimulation-induced middle meningeal artery (MMA) vasodilation in rats

3 humanized antibody molecules hu224, hu228, and hu231 with good *in vitro* efficacy were selected in Example 12, and further evaluated for their *in vivo* efficacy on the MMA diameter and mean arterial pressure in SD rats with closed cranial window electrostimulation-induced migraine. It was found that hu224, hu228, and hu231 exhibited inhibitory effects on closed cranial window electrostimulation-induced MMA vasodilation and that the effects were sustained for at least 120 min in the SD rats.

40 male SD rats (Vital River Laboratory Animal Technology Co., Ltd.) were selected. One day before treatment, the rats were randomized into 5 groups by body weight, with 8 animals in each group, i.e., a vehicle group, a sumatriptan group, a hu224 group, a hu228 group, and a hu231 group. The treatments were given subcutaneously at 10 mg/10 mL/kg, while the vehicle group received normal saline at the same volume. The sumatriptan group was administered 5 min after the first electrostimulation. Each group received 1 dose. At 24 h post-dose, the animals were placed on a thermostat blanket after deep anesthetization by an intraperitoneal compound Zoletil/xylazine injection. The animals were connected to a small animal ventilator by tracheal intubation to ensure stable respiration during the molding and recording process, and the mean arterial pressure was measured by femoral artery intubation. The animals were fixed to a stereotaxic apparatus, the skull was exposed, and the skull above the middle meningeal artery was thinned using a dental drill until the middle meningeal artery was visible, and a closed cranial window (10 × 7 mm²) was established. After 45-60 min of postoperative recovery and the stabilization of MMA diameter, electrostimulation was given to the closed cranial window (about 200 µm to the middle meningeal artery) through a bipolar electrode, and the stimulation conditions of the vehicle group, reference compound group, and treatment group are as follows: the stimulation intensity was 20 V and 10 Hz, the wave width was 0.5 ms, and the stimulation was 30 s. Before electro stimulation, the MMA diameter was measured and the 5-min stable femoral artery pressure was recorded; after the end of electrostimulation, the femoral artery pressure changes and the MMA diameters were recorded at 30 min, 60 min, 90 min, 120 min (4 time points).

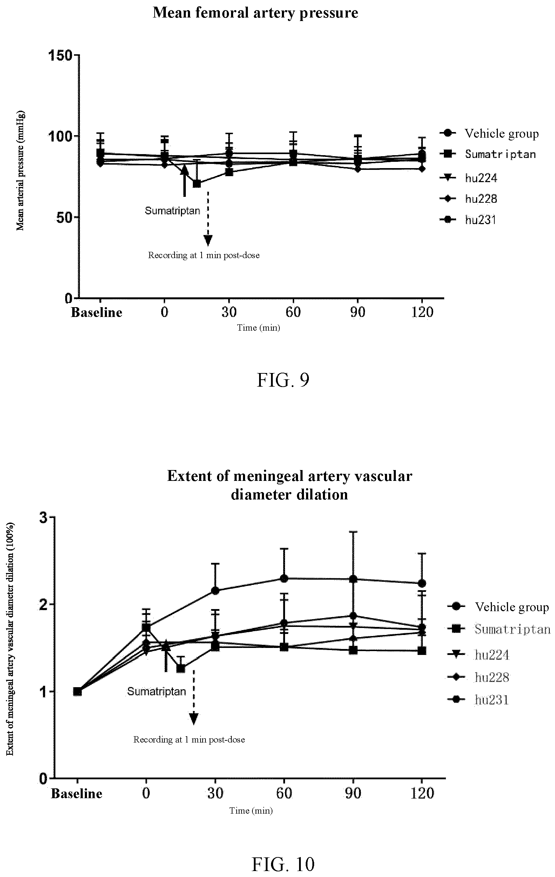

At 30 min, 60 min, 90 min, and 120 min after electrostimulation, the animals in the vehicle group exhibited a significant dilation in the MMA diameter, indicating that the closed cranial window electrostimulation-induced migraine model was successfully established. The animals in the vehicle group, the sumatriptan group, and the test antibody groups did not exhibit significant changes in the mean arterial pressure. Sumatriptan and the test antibodies all exhibited significant inhibitory effects on the electrostimulation-induced MMA vasodilation. The detailed results are shown in FIGs. 9 and 10.

### Example 12. Epitope identification of chimeric antibodies and humanized antibodies binding to CGRP

The kinetic parameters of C245 Fab (Fab monomer fragments derived from antibody C245) binding to six synthetic human CGRP alpha polypeptide fragments (polypeptide 1-13, polypeptide 1-19, polypeptide 1-37, polypeptide 8-19, polypeptide 8-26, and polypeptide 13-24) were determined on an Octet RBD 96e system. Human CGRP synthetic polypeptide fragments were immobilized on the surfaces of SA bioprobes (Sartorius, Cat. No. 18-5019) at the following concentrations: 46.5 nM for hCGRP (1-13), 8.5 nM for CGRP (1-19), 15.5 nM for hCGRP (1-37), 8.5 nM for hCGRP (8-19), 15.5 nM for hCGRP (8-26), and 46.5 nM for hCGRP (13-24). The immobilized synthetic human CGRP polypeptide fragments were bound to 45 nM of C245 Fab and the binding dissociation curves were fitted using analytical software.

The kinetic parameters of hu224 Fab (Fab monomer fragments derived from humanized antibody hu224) binding to four synthetic human CGRP alpha polypeptide fragments (polypeptide 1-13, polypeptide 1-19, polypeptide 1-37, and polypeptide 8-19) were determined on an Octet RBD 96e system. Human CGRP synthetic polypeptide fragments were immobilized on the surfaces of SA bioprobes (Sartorius, Cat. No. 18-5019) at the following concentrations: 8.5 nM for CGRP (1-19), 15.5 nM for hCGRP (1-37), 10 nM for hCGRP (8-19), and 15.5 nM for hCGRP (8-26). The immobilized synthetic human CGRP polypeptide fragments were bound to 45 nM of hu224 Fab and the binding dissociation curves were fitted using analytical software.

The molecular sequences of the synthetic human CGRP alpha polypeptide fragments are as follows:
The molecular sequence of the synthetic hCGRP (1-13) polypeptide fragment was Biotin-Linker(G₅)-ACDTATCVTHRLA, where the N-terminus was biotin-labeled, and CYS residues at positions 7 and 12 theoretically form 1 intrachain disulfide bond.

The molecular sequence of the synthetic hCGRP (1-19) polypeptide fragment was Biotin-Linker(G₅)-ACDTATCVTHRLAGLLSRS, where the N-terminus was biotin-labeled, and CYS residues at positions 7 and 12 theoretically form 1 intrachain disulfide bond.

The molecular sequence of the synthetic hCGRP (1-37) polypeptide fragment was Biotin-Linker(G₅)-ACDTATCVTHRLAGLLSRSGGVVKNNFVPTNVGSKAF-NH₂, where the N-terminus was biotin-labeled, the C-terminus was the tail (F-NH₂ refers to phenylalanine amide, i.e., a CONH₂ structure formed at the tail), and CYS residues at positions 7 and 12 theoretically form 1 intrachain disulfide bond.

The molecular sequence of the synthetic hCGRP (8-19) polypeptide fragment was Blotin-Linker(G₅)-VTHRLAGLLSRS, where the N-terminus was biotin-labeled.

The molecular sequence of the synthetic hCGRP (8-26) polypeptide fragment was Biotin-Linker(G₅)-VTHRLAGLLSRSGGVVKNN, where the N-terminus was biotin-labeled.

The molecular sequence of the synthetic hCGRP (13-24) polypeptide fragment was Biotin-Linker(G₅)-AGLLSRSGGVVK, where the N-terminus was biotin-labeled.

The amino acid sequences of the human CGRP fragments above are summarized in Table 15.

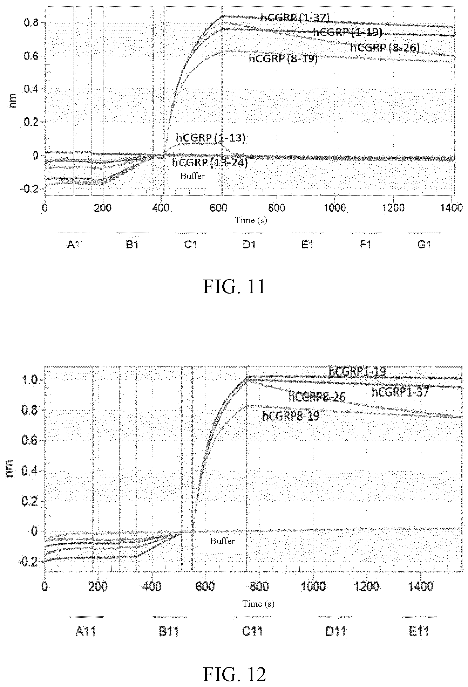

The kinetic parameters of the C245 Fab binding to the six synthetic human CGRP polypeptide fragments are shown in Table 14, and the results of kinetic characterization parameter detection are shown in FIG. 11.

The kinetic parameters of the hu224 Fab binding to the four synthetic human CGRP polypeptide fragments are shown in Table 14, and the results of kinetic characterization parameter detection are shown in FIG. 12.

The C245 Fab exhibited binding activity to all of the four polypeptide fragments hCGRP (1-19), hCGRP (1-37), hCGRP (8-19) and hCGRP (8-26) used, wherein the full-length hCGRP (1-37) polypeptide and the shortest hCGRP (8-19) polypeptide fragment demonstrated similar affinity KD values of 2.487E-10M and 2.523E-10M, respectively, suggesting that amino acids 8-19 in human CGRP are the major binding epitope of the C245 antibody to the human CGRP antigen. hCGRP (1-13) and hCGRP (13-24) polypeptide fragments were synthesized by division at amino acid position 13 at the center of amino acids 8-19 in human CGRP, and then the affinity was detected. The two fragments showed a 100-fold decrease in affinity and no binding activity, respectively, suggesting that the integrity of amino acids 8-19 in human CGRP has an important effect on the binding to the C245 antibody.

The humanized antibody-derived hu224 Fab exhibited binding activity to all of the four polypeptide fragments hCGRP (1-19), hCGRP (1-37), hCGRP (8-19) and hCGRP (8-26) used, wherein the full-length hCGRP (1-37) polypeptide and the shortest hCGRP (8-19) polypeptide fragment demonstrated similar affinity KD values of 2.882E-10M and 4.447E-10M, respectively, suggesting that amino acids 8-19 in human CGRP are the major binding epitope of the antibody to the human CGRP antigen.

**Table 14. Kinetic parameters of C245 Fab binding to six synthetic human CGRP polypeptide fragments**

| Antigen | Antibody | Kon(1/Ms) | Kdis(1/s) | KD(M) |
|---|---|---|---|---|
| hCGRP(1-13) | C245 Fab | 7.045E+05 | 1.718E-02 | 2.438E-8 |
| hCGRP(1-19) | | 3.572E+05 | 2.576E-05 | 7.213E-11 |
| hCGRP(1-37) | | 2.797E+05 | 6.954E-05 | 2.487E-10 |
| hCGRP(8-19) | | 3.603E-05 | 9.315E-05 | 2.523E-10 |
| hCGRP(8-26) | | 3.324E+05 | 3.164E-05 | 9.520E-10 |
| hCGRP(13-24) | | NA | NA | No binding |
| hCGRP(1-19) | hu224 Fab | 3.178E+05 | 2.616E-05 | 8.233E-11 |
| hCGRP(1-37) | | 2.767E+05 | 7.974E-05 | 2.882E-10 |
| hCGRP(8-19) | | 3.407E+05 | 1.515E-04 | 4.447E-10 |
| hCGRP(8-26) | | 3.156E+05 | 3.737E-04 | 1.184E-09 |

| | | | | |
|---|---|---|---|---|
| Note: KD is the affinity constant; Kₒₙ is the antigen-antibody association rate; K_{dis} is the antigen-antibody dissociation rate; KD = K_{dis}/Kₒₙ | | | | |

**Table 15. Amino acid sequences of human α-CGRP fragments and related peptides**

| CGRP | Amino acid sequence | SEQ ID NO |
|---|---|---|
| 1-37 (WT) | ACDTATCVTHRLAGLLSRSGGVVKNNFVPTNVGSKAF | 97 |
| 1-13 | ACDTATCVTHRLA | 98 |
| 1-19 | ACDTATCVTHRLAGLLSRS | 99 |
| 8-19 | VTHRLAGLLSRS | 100 |
| 8-26 | VTHRLAGLLSRSGGVVKNN | 101 |
| 13-24 | AGLLSRSGGVVK | 102 |

### Example 13. pH-dependent antigen binding assay

The association/dissociation profiles for three antibodies Hu224, galcanezumab analogue and fremanezumab analogue binding to the antigen human α-CGRP in solutions at different pH (7.4 and 5.5) were determined on an Octet RBD96e system. The pH 7.4 buffer contained 10 mM HEPES (pH 7.4), 150 mM sodium chloride, 3 mM EDTA, and 0.05% P20. The pH 5.5 buffer contained 10 mM NaAc (pH 5.5), 150 mM sodium chloride, and 0.05% P20. The three antibodies Hu224, galcanezumab analogue, and fremanezumab analogue were immobilized on the surfaces of AHC2 bioprobes (Sartorius, Cat. No. 18-5142) at 10 µg/mL, respectively. 50 nM human α-CGRPs diluted in different pH (7.4 and 5.5) buffers were used to bind to the antibodies during the 0 to 200 s antigen association phase, and different pH (7.4 and 5.5) buffers were used to wash during the 0 to 200 s antigen dissociation phase. The conditions were pH 7.4 association and pH 7.4 dissociation, pH 7.4 association and pH 5.5 dissociation, and pH 5.5 association and pH 5.5 dissociation. The binding signals of the antigen at the time of injection for 200 s were calculated using analytical software and fitted to give the dissociation constant kd. The amino acid sequence of the synthetic human α-CGRP polypeptide fragment is ACDTATCVTHRLAGLLSRSGGVVKNNFVPTNVGSKAF (SEQ ID NO: 97)-NH₂, where the C-terminus was the tail (F-NH₂ refers to phenylalanine amide, i.e., a CONH₂ structure formed at the tail), and CYS residues at positions 7 and 12 theoretically form 1 intrachain disulfide bond.

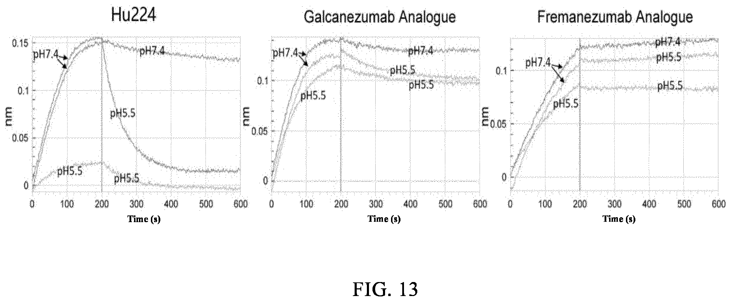

As can be seen from FIG. 13 and the data in Table 16, at pH 5.5, the Hu224 antibody showed significant dissociation relative to galcanezumab and fremanezumab. In the low pH intracellular condition, antibody Hu224 may have low affinity to CGRP, and the high dissociation rate can accelerate the recycling of the antibody. That is, the dissociated antibody is captured by FcRn and recycled to the outside of cells, and the antibody is released in the neutral pH extracellular environment, such that the retention time of the antibody in the blood is increased, and the half-life of the antibody is prolonged. The PK data of cynomolgus monkey also show that the anti-CGRP antibody of the present disclosure has a longer half-life than that of galcanezumab, and it can be predicted that the duration of the *in vivo* efficacy of the anti-CGRP antibody disclosed herein will be longer.

**Table 16. Summary of association/dissociation profiles of antibodies to the antigen human α-CGRP in different pH conditions**

| Antibody | Association condition | Dissociation condition | Binding signal (nm) | Dissociation constant kd (1/s) |
|---|---|---|---|---|
| Hu224 | pH 7.4 | pH 7.4 | 0.15 | 2.65E-04 |
| | pH 7.4 | pH 5.5 | 0.15 | 1.43E-02 |
| | pH 5.5 | pH 5.5 | 0.02 | 1.41E-02 |
| Galcanezumab Analogue | pH 7.4 | pH 7.4 | 0.14 | 8.36E-05 |
| | pH 7.4 | pH 5.5 | 0.12 | 8.71E-03 |
| | pH 5.5 | pH 5.5 | 0.11 | 2.51E-04 |
| Fremanezumab Analogue | pH 7.4 | pH 7.4 | 0.12 | <1.00E-05 |
| | pH 7.4 | pH 5.5 | 0.10 | <1.00E-05 |
| | pH 5.5 | pH 5.5 | 0.08 | 5.10E-05 |

## Claims

1. An anti-CGRP antibody or an antigen-binding fragment thereof, comprising an HCDR1, an HCDR2, an HCDR3, an LCDR1, an LCDR2, and an LCDR3, wherein HCDR1: DX¹NMH or GYTMN, wherein X¹ is F or Y;
HCDR2: X²X³X⁴PYNX⁵X⁶X⁷X⁸YNX⁹X¹⁰FX¹¹X¹² or LINPYIGNTHYNQKFKD, wherein X² is Y or N, X³ is I or V, X⁴ is Y, S, or F, X⁵ is G or A, X⁶ is D, G, V, or H, X⁷ is V, T, S, or A, X⁸ is V or A, X⁹ is Q or R, X¹⁰ is K, N, or D, X¹¹ is T, K, N, or R, and X¹² is N, F, or S;
HCDR3: EGLGGY or ELDSGFDY;
LCDR1: X¹³X¹⁴SQNIZYX¹⁵X¹⁶, wherein X¹³ is K, Q, or R, X¹⁴ is A or S, Z is DYDGYA, DFDGYA, DGNT, VHSNGDT, VHSNADT, DYDGYT, or DYGGYS, X¹⁵ is L or M, and X¹⁶ is N or E;
LCDR2: SX¹⁷FNLES or KVSNRFS, wherein X¹⁷ is V or I; and
LCDR3: X¹⁸QSX¹⁹X²⁰X²¹PYT or YQGSHVPWT, wherein X¹⁸ is Q, X¹⁹ is N or H, X²⁰ is E or D, X²¹ is F, V, Y, or A;
preferably, the antibody or the antigen-binding fragment thereof comprises: an HCDR1 having the amino acid sequence set forth in SEQ ID NOs: 2, 11, 19, 26, 31, 37, or 43; an HCDR2 having the amino acid sequence set forth in SEQ ID NOs: 3, 110, 12, 111, 20, 112, 27, 32, 38, or 44; an HCDR3 having the amino acid sequence set forth in SEQ ID NOs: 4, 13, 21, 28, 33, 39, or 45; an LCDR1 having the amino acid sequence set forth in SEQ ID NOs: 51, 59, 67, 73, 113, 81, or 86; an LCDR2 having the amino acid sequence set forth in SEQ ID NOs: 52, 60, 68, 74, 82, or 87; and an LCDR3 having the amino acid sequence set forth in SEQ ID NOs: 53, 61, 69, 75, 83, or 88.

2. An anti-CGRP antibody or an antigen-binding fragment thereof, comprising a heavy chain variable region and/or a light chain variable region, wherein
the heavy chain variable region comprises:
(I) an HCDR1, an HCDR2, and an HCDR3 having the amino acid sequences set forth in SEQ ID NO: 2, SEQ ID NO: 3, and SEQ ID NO: 4, respectively; or an HCDR1, an HCDR2, and an HCDR3 differing from the amino acid sequences set forth in SEQ ID NO: 2, SEQ ID NO: 3, and SEQ ID NO: 4, respectively, by 1, 2, or 3 amino acids; or
(II) an HCDR1, an HCDR2, and an HCDR3 having the amino acid sequences set forth in SEQ ID NO: 2, SEQ ID NO: 110, and SEQ ID NO: 4, respectively; or an HCDR1, an HCDR2, and an HCDR3 differing from the amino acid sequences set forth in SEQ ID NO: 2, SEQ ID NO: 110, and SEQ ID NO: 4, respectively, by 1, 2, or 3 amino acids; or
(III) an HCDR1, an HCDR2, and an HCDR3 having the amino acid sequences set forth in SEQ ID NO: 11, SEQ ID NO: 12, and SEQ ID NO: 13, respectively; or an HCDR1, an HCDR2, and an HCDR3 differing from the amino acid sequences set forth in SEQ ID NO: 11, SEQ ID NO: 12, and SEQ ID NO: 13, respectively, by 1, 2, or 3 amino acids; or
(IV) an HCDR1, an HCDR2, and an HCDR3 having the amino acid sequences set forth in SEQ ID NO: 11, SEQ ID NO: 111, and SEQ ID NO: 13, respectively; or an HCDR1, an HCDR2, and an HCDR3 differing from the amino acid sequences set forth in SEQ ID NO: 11, SEQ ID NO: 111, and SEQ ID NO: 13, respectively, by 1, 2, or 3 amino acids; or
(V) an HCDR1, an HCDR2, and an HCDR3 having the amino acid sequences set forth in SEQ ID NO: 19, SEQ ID NO: 20, and SEQ ID NO: 21, respectively; or an HCDR1, an HCDR2, and an HCDR3 differing from the amino acid sequences set forth in SEQ ID NO: 19, SEQ ID NO: 20, and SEQ ID NO: 21, respectively, by 1, 2, or 3 amino acids; or
(VI) an HCDR1, an HCDR2, and an HCDR3 having the amino acid sequences set forth in SEQ ID NO: 19, SEQ ID NO: 112, and SEQ ID NO: 21, respectively; or an HCDR1, an HCDR2, and an HCDR3 differing from the amino acid sequences set forth in SEQ ID NO: 19, SEQ ID NO: 112, and SEQ ID NO: 21, respectively, by 1, 2, or 3 amino acids; or
(VII) an HCDR1, an HCDR2, and an HCDR3 having the amino acid sequences set forth in SEQ ID NO: 26, SEQ ID NO: 27, and SEQ ID NO: 28, respectively; or an HCDR1, an HCDR2, and an HCDR3 differing from the amino acid sequences set forth in SEQ ID NO: 26, SEQ ID NO: 27, and SEQ ID NO: 28, respectively, by 1, 2, or 3 amino acids; or
(VIII) an HCDR1, an HCDR2, and an HCDR3 having the amino acid sequences set forth in SEQ ID NO: 31, SEQ ID NO: 32, and SEQ ID NO: 33, respectively; or an HCDR1, an HCDR2, and an HCDR3 differing from the amino acid sequences set forth in SEQ ID NO: 31, SEQ ID NO: 32, and SEQ ID NO: 33, respectively, by 1, 2, or 3 amino acids; or
(IX) an HCDR1, an HCDR2, and an HCDR3 having the amino acid sequences set forth in SEQ ID NO: 37, SEQ ID NO: 38, and SEQ ID NO: 39, respectively; or an HCDR1, an HCDR2, and an HCDR3 differing from the amino acid sequences set forth in SEQ ID NO: 37, SEQ ID NO: 38, and SEQ ID NO: 39, respectively, by 1, 2, or 3 amino acids; or
(X) an HCDR1, an HCDR2, and an HCDR3 having the amino acid sequences set forth in SEQ ID NO: 43, SEQ ID NO: 44, and SEQ ID NO: 45, respectively; or an HCDR1, an HCDR2, and an HCDR3 differing from the amino acid sequences set forth in SEQ ID NO: 43, SEQ ID NO: 44, and SEQ ID NO: 45, respectively, by 1, 2, or 3 amino acids;
the light chain variable region comprises:
(I) an LCDR1, an LCDR2, and an LCDR3 having the amino acid sequences set forth in SEQ ID NO: 51, SEQ ID NO: 52, and SEQ ID NO: 53, respectively; or an LCDR1, an LCDR2, and an LCDR3 differing from the amino acid sequences set forth in SEQ ID NO: 51, SEQ ID NO: 52, and SEQ ID NO: 53, respectively, by 1, 2, or 3 amino acids; or
(II) an LCDR1, an LCDR2, and an LCDR3 having the amino acid sequences set forth in SEQ ID NO: 59, SEQ ID NO: 60, and SEQ ID NO: 61, respectively; or an LCDR1, an LCDR2, and an LCDR3 differing from the amino acid sequences set forth in SEQ ID NO: 59, SEQ ID NO: 60, and SEQ ID NO: 61, respectively, by 1, 2, or 3 amino acids; or
(III) an LCDR1, an LCDR2, and an LCDR3 having the amino acid sequences set forth in SEQ ID NO: 67, SEQ ID NO: 68, and SEQ ID NO: 69, respectively; or an LCDR1, an LCDR2, and an LCDR3 differing from the amino acid sequences set forth in SEQ ID NO: 67, SEQ ID NO: 68, and SEQ ID NO: 69, respectively, by 1, 2, or 3 amino acids; or
(IV) an LCDR1, an LCDR2, and an LCDR3 having the amino acid sequences set forth in SEQ ID NO: 73, SEQ ID NO: 74, and SEQ ID NO: 75, respectively; or an LCDR1, an LCDR2, and an LCDR3 differing from the amino acid sequences set forth in SEQ ID NO: 73, SEQ ID NO: 74, and SEQ ID NO: 75, respectively, by 1, 2, or 3 amino acids; or
(V) an LCDR1, an LCDR2, and an LCDR3 having the amino acid sequences set forth in SEQ ID NO: 113, SEQ ID NO: 74, and SEQ ID NO: 75, respectively; or an LCDR1, an LCDR2, and an LCDR3 differing from the amino acid sequences set forth in SEQ ID NO: 113, SEQ ID NO: 74, and SEQ ID NO: 75, respectively, by 1, 2, or 3 amino acids; or
(VI) an LCDR1, an LCDR2, and an LCDR3 having the amino acid sequences set forth in SEQ ID NO: 81, SEQ ID NO: 82, and SEQ ID NO: 83, respectively; or an LCDR1, an LCDR2, and an LCDR3 differing from the amino acid sequences set forth in SEQ ID NO: 81, SEQ ID NO: 82, and SEQ ID NO: 83, respectively, by 1, 2, or 3 amino acids; or
(VII) an LCDR1, an LCDR2, and an LCDR3 having the amino acid sequences set forth in SEQ ID NO: 86, SEQ ID NO: 87, and SEQ ID NO: 88, respectively; or an LCDR1, an LCDR2, and an LCDR3 differing from the amino acid sequences set forth in SEQ ID NO: 86, SEQ ID NO: 87, and SEQ ID NO: 88, respectively, by 1, 2, or 3 amino acids;
preferably, the antibody or the antigen-binding fragment thereof comprises:
(I) a heavy chain variable region, comprising an HCDR1, an HCDR2, and an HCDR3 having the amino acid sequences set forth in SEQ ID NO: 31, SEQ ID NO: 32, and SEQ ID NO: 33, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(II) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(III) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(IV) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(V) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 81, SEQ ID NO: 82, and SEQ ID NO: 83, respectively; or
(VI) a heavy chain variable region, comprising an HCDR1, an HCDR2, and an HCDR3 having the amino acid sequences set forth in SEQ ID NO: 2, SEQ ID NO: 3, and SEQ ID NO: 4, respectively; and a light chain variable region, comprising an LCDR1, an LCDR2, and an LCDR3 having the amino acid sequences set forth in SEQ ID NO: 86, SEQ ID NO: 87, and SEQ ID NO: 88, respectively; or
(VII) a heavy chain variable region, comprising an HCDR1, an HCDR2, and an HCDR3 having the amino acid sequences set forth in SEQ ID NO: 26, SEQ ID NO: 27, and SEQ ID NO: 28, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(VIII) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(IX) a heavy chain variable region, comprising an HCDR1, an HCDR2, and an HCDR3 having the amino acid sequences set forth in SEQ ID NO: 19, SEQ ID NO: 20, and SEQ ID NO: 21, respectively; and a light chain variable region, comprising an LCDR1, an LCDR2, and an LCDR3 having the amino acid sequences set forth in SEQ ID NO: 67, SEQ ID NO: 68, and SEQ ID NO: 69, respectively; or
(X) a heavy chain variable region, comprising an HCDR1, an HCDR2, and an HCDR3 having the amino acid sequences set forth in SEQ ID NO: 43, SEQ ID NO: 44, and SEQ ID NO: 45, respectively; and a light chain variable region, comprising an LCDR1, an LCDR2, and an LCDR3 having the amino acid sequences set forth in SEQ ID NO: 73, SEQ ID NO: 74, and SEQ ID NO: 75, respectively; or
(XI) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XII) a heavy chain variable region, comprising an HCDR1, an HCDR2, and an HCDR3 having the amino acid sequences set forth in SEQ ID NO: 11, SEQ ID NO: 12, and SEQ ID NO: 13, respectively; and a light chain variable region, comprising an LCDR1, an LCDR2, and an LCDR3 having the amino acid sequences set forth in SEQ ID NO: 59, SEQ ID NO: 60, and SEQ ID NO: 61, respectively; or
(XIII) a heavy chain variable region, comprising an HCDR1, an HCDR2, and an HCDR3 having the amino acid sequences set forth in SEQ ID NO: 37, SEQ ID NO: 38, and SEQ ID NO: 39, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively; or
(XIV) a heavy chain variable region, comprising an HCDR1, an HCDR2, and an HCDR3 having the amino acid sequences set forth in SEQ ID NO: 19, SEQ ID NO: 112, and SEQ ID NO: 21, respectively; and a light chain variable region, comprising an LCDR1, an LCDR2, and an LCDR3 having the amino acid sequences set forth in SEQ ID NO: 51, SEQ ID NO: 52, and SEQ ID NO: 53, respectively;
more preferably, the antibody or the antigen-binding fragment thereof comprises a heavy chain variable region and a light chain variable region, wherein:
(I) the heavy chain variable region comprises the amino acid sequence set forth in SEQ ID NO: 30, or comprises an amino acid sequence having at least 95%, 96%, 97%, 98%, or 99% sequence identity to the amino acid sequence set forth in SEQ ID NO: 30, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(II) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(III) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(IV) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(V) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 80, or comprises an amino acid sequence having at least 95%, 96%, 97%, 98%, or 99% sequence identity to the amino acid sequence set forth in SEQ ID NO: 80; or
(VI) the heavy chain variable region comprises the amino acid sequence set forth in SEQ ID NO: 1, or comprises an amino acid sequence having at least 95%, 96%, 97%, 98%, or 99% sequence identity to the amino acid sequence set forth in SEQ ID NO: 1, and the light chain variable region comprises the amino acid sequence set forth in SEQ ID NO: 85, or comprises an amino acid sequence having at least 95%, 96%, 97%, 98%, or 99% sequence identity to the amino acid sequence set forth in SEQ ID NO: 85; or
(VII) the heavy chain variable region comprises the amino acid sequence set forth in SEQ ID NO: 25, or comprises an amino acid sequence having at least 95%, 96%, 97%, 98%, or 99% sequence identity to the amino acid sequence set forth in SEQ ID NO: 25, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(VIII) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(IX) the heavy chain variable region comprises the amino acid sequence set forth in SEQ ID NO: 18, or comprises an amino acid sequence having at least 95%, 96%, 97%, 98%, or 99% sequence identity to the amino acid sequence set forth in SEQ ID NO: 18, and the light chain variable region comprises the amino acid sequence set forth in SEQ ID NO: 66, or comprises an amino acid sequence having at least 95%, 96%, 97%, 98%, or 99% sequence identity to the amino acid sequence set forth in SEQ ID NO: 66; or
(X) the heavy chain variable region comprises the amino acid sequence set forth in SEQ ID NO: 42, or comprises an amino acid sequence having at least 95%, 96%, 97%, 98%, or 99% sequence identity to the amino acid sequence set forth in SEQ ID NO: 42, and the light chain variable region comprises the amino acid sequence set forth in SEQ ID NO: 72, or comprises an amino acid sequence having at least 95%, 96%, 97%, 98%, or 99% sequence identity to the amino acid sequence set forth in SEQ ID NO: 72; or
(XI) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50; or
(XII) the heavy chain variable region comprises the amino acid sequence set forth in SEQ ID NO: 10, or comprises an amino acid sequence having at least 95%, 96%, 97%, 98%, or 99% sequence identity to the amino acid sequence set forth in SEQ ID NO: 10, and the light chain variable region comprises the amino acid sequence set forth in SEQ ID NO: 58, or comprises an amino acid sequence having at least 95%, 96%, 97%, 98%, or 99% sequence identity to the amino acid sequence set forth in SEQ ID NO: 58; or
(XIII) the heavy chain variable region comprises the amino acid sequence set forth in SEQ ID NO: 36, or comprises an amino acid sequence having at least 95%, 96%, 97%, 98%, or 99% sequence identity to the amino acid sequence set forth in SEQ ID NO: 36, and the light chain variable region comprises the amino acid sequence set forth in SEQ ID NO: 50, or comprises an amino acid sequence having at least 95%, 96%, 97%, 98%, or 99% sequence identity to the amino acid sequence set forth in SEQ ID NO: 50.

3. The antibody or the antigen-binding fragment thereof according to claim 1 or 2, comprising:
(I) a heavy chain variable region, comprising the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and a light chain variable region, comprising the amino acid sequence set forth in any one of SEQ ID NOs: 54, 55, 56, 57, 62, 63, 64, 65, 70, 71, 76, 77, 78, 79, 84, 89, or 90; or
(II) a heavy chain variable region, comprising an amino acid sequence having at least 95%, 96%, 97%, 98%, or 99% sequence identity to the amino acid sequence set forth in any one of SEQ ID NOs: 5, 6, 7, 8, 9, 14, 15, 16, 17, 22, 23, 24, 29, 34, 35, 40, 41, 46, 47, 48, or 49; and a light chain variable region, comprising an amino acid sequence having at least 95%, 96%, 97%, 98%, or 99% sequence identity to the amino acid sequence set forth in any one of SEQ ID NOs: 54, 55, 56, 57, 62, 63, 64, 65, 70, 71, 76, 77, 78, 79, 84, 89, or 90; or
(III) a heavy chain variable region having the amino acid sequence set forth in SEQ ID NO: 46 and a light chain variable region having the amino acid sequence set forth in SEQ ID NO: 78; or a heavy chain variable region having the amino acid sequence set forth in SEQ ID NO: 47 and a light chain variable region having the amino acid sequence set forth in SEQ ID NO: 78; or a heavy chain variable region having the amino acid sequence set forth in SEQ ID NO: 48 and a light chain variable region having the amino acid sequence set forth in SEQ ID NO: 77; or a heavy chain variable region having the amino acid sequence set forth in SEQ ID NO: 8 and a light chain variable region having the amino acid sequence set forth in SEQ ID NO: 63; or a heavy chain variable region having the amino acid sequence set forth in SEQ ID NO: 24 and a light chain variable region having the amino acid sequence set forth in SEQ ID NO: 54; or a heavy chain variable region having the amino acid sequence set forth in SEQ ID NO: 8 and a light chain variable region having the amino acid sequence set forth in SEQ ID NO: 54;
preferably, the antibody comprises:
(I) a heavy chain, comprising the amino acid sequence set forth in any one of SEQ ID NOs: 92, 94, or 96, and a light chain, comprising the amino acid sequence set forth in any one of SEQ ID NOs: 91, 93, or 95; or
(II) a heavy chain, comprising an amino acid sequence having at least 95%, 96%, 97%, 98%, or 99% sequence identity to the amino acid sequence set forth in any one of SEQ ID NOs: 92, 94, or 96, and a light chain, comprising an amino acid sequence having at least 95%, 96%, 97%, 98%, or 99% sequence identity to the amino acid sequence set forth in any one of SEQ ID NOs: 91, 93, or 95; or
(III) a heavy chain having the amino acid sequence set forth in SEQ ID NO: 92 and a light chain having the amino acid sequence set forth in SEQ ID NO: 91, or a heavy chain having the amino acid sequence set forth in SEQ ID NO: 94 and a light chain having the amino acid sequence set forth in SEQ ID NO: 93, or a heavy chain having the amino acid sequence set forth in SEQ ID NO: 96 and a light chain having the amino acid sequence set forth in SEQ ID NO: 95.

4. The antibody or the antigen-binding fragment thereof according to any one of claims 1-3, wherein the antibody is selected from a murine antibody, a chimeric antibody, a humanized antibody, and a fully human antibody, and/or the antigen-binding fragment is selected from Fab, Fab', F(ab')₂, Fv, scFv, and sdAb.

5. The antibody or the antigen-binding fragment thereof according to any one of claims 1-4, wherein the antibody or the antigen-binding fragment thereof is of any IgG subtype, such as IgG1, IgG2, IgG3, or IgG4, preferably IgG1 subtype or IgG4 subtype.

6. An isolated anti-CGRP antibody or an antigen-binding fragment thereof, having at least one of the following properties:
(I) binding to an epitope on human CGRP protein that is identical to or completely or partially overlaps with that of the antibody or the antigen-binding fragment thereof according to any one of claims 1-5; and
(II) competing for binding to an epitope on human CGRP protein with the antibody or the antigen-binding fragment thereof according to any one of claims 1-5.

7. A polynucleotide molecule, encoding the anti-CGRP antibody or the antigen-binding fragment thereof according to any one of claims 1-5 or the isolated antibody or the antigen-binding fragment thereof according to claim 6.

8. An expression vector, comprising the polynucleotide molecule according to claim 7, wherein preferably, the vector is a eukaryotic expression vector.

9. A host cell, comprising the polynucleotide molecule according to claim 7 or the expression vector according to claim 8, wherein preferably, the host cell is a eukaryotic cell, more preferably, a mammalian cell.

10. A method for preparing the anti-CGRP antibody or the antigen-binding fragment thereof according to any one of claims 1-5 or the isolated antibody or the antigen-binding fragment thereof according to claim 6, comprising expressing the antibody or the antigen-binding fragment thereof in the host cell according to claim 9 in a condition suitable for the expression of the antibody or the antigen-binding fragment thereof, and isolating the expressed antibody or antigen-binding fragment thereof from the host cell.

11. A multispecific antibody, comprising the light chain variable region and the heavy chain variable region of the antibody or the antigen-binding fragment thereof according to any one of claims 1-5 or the isolated antibody or the antigen-binding fragment thereof according to claim 6.

12. A single-chain antibody, comprising the light chain variable region and the heavy chain variable region of the antibody or the antigen-binding fragment thereof according to any one of claims 1-5 or the isolated antibody or the antigen-binding fragment thereof according to claim 6.

13. A pharmaceutical composition, comprising the anti-CGRP antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the multispecific antibody according to claim 11, or the single-chain antibody according to claim 12, and a pharmaceutically acceptable carrier or excipient.

14. A pharmaceutical combination, comprising the anti-CGRP antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the multispecific antibody according to claim 11, the single-chain antibody according to claim 12, or the pharmaceutical composition according to claim 13, and one or more additional therapeutic agents.

15. Use of the antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the multispecific antibody according to claim 11, the single-chain antibody according to claim 12, the pharmaceutical composition according to claim 13, or the pharmaceutical combination according to claim 14 in preparing a medicament for treating and/or preventing a CGRP-mediated disease or disorder, wherein preferably, the disease or disorder is a symptom having CGRP-associated pain; more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica.

16. A method for treating and/or preventing a CGRP-mediated disease or disorder, comprising administering to a subject in need an effective amount of the antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the multispecific antibody according to claim 11, the single-chain antibody according to claim 12, the pharmaceutical composition according to claim 13, or the pharmaceutical combination according to claim 14, wherein the disease or disorder is a symptom having CGRP-associated pain; more preferably, the pain is selected from migraine with aura, migraine without aura, high-frequency episodic migraine, menstrual migraine, hemiplegic migraine, cluster headache, migrainous neuralgia, chronic headache, tension headache, general headache, hot flashes, chronic paroxysmal hemicrania, secondary headache due to potential structural problems in head, secondary headache due to potential structural problems in neck, cranial neuralgia, sinus headache, allergy-induced headache, trigeminal neuralgia, fibromyalgia, and sciatica; preferably, the subject is a human.

17. A kit, comprising the antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the multispecific antibody according to claim 11, the single-chain antibody according to claim 12, the pharmaceutical composition according to claim 13, or the pharmaceutical combination according to claim 14.

18. A method for detecting the presence of CGRP in a sample, comprising: contacting the antibody or the antigen-binding fragment thereof according to any one of claims 1-5 or the isolated antibody or the antigen-binding fragment thereof according to claim 6, or a detection composition comprising the antibody or the antigen-binding fragment thereof with the sample.
